# EUROPEAN PATENT SPECIFICATION

(11) **EP 3 209 692 B1**
(45) Date of publication and mention of the grant of the patent: **22.05.2024**
(21) Application number: 15794643.5
(22) Date of filing: 23.10.2015
(51) Int. Cl.: C07K 16/28, A61K 39/395, G01N 33/53, A61K 35/741

(54) **METHODS AND PRODUCTS FOR MODULATING MICROBIOTA COMPOSITION FOR IMPROVING THE EFFICACY OF A CANCER TREATMENT WITH AN IMMUNE CHECKPOINT BLOCKER**
VERFAHREN UND PRODUKTE ZUR BIOZÖNOSEZUSAMMENSETZUNGSMODULIERUNG ZUR VERBESSERUNG DER WIRKSAMKEIT EINER KREBSBEHANDLUNG MIT EINEM PRÜFPUNKTBLOCKER
PROCÉDÉS ET PRODUITS PERMETTANT DE MODULER LA COMPOSITION DU MICROBIOTE AFIN D'AMÉLIORER L'EFFICACITÉ D'UN TRAITEMENT ANTICANCÉREUX IMPLIQUANT UN INHIBITEUR DES POINTS DE CONTRÔLE DU SYSTÈME IMMUNITAIRE

(30) Priority: 23.10.2014 EP 14190167; 31.03.2015 EP 15162097
(43) Date of publication of application: 30.08.2017
(73) Proprietor: Institut Gustave Roussy, 94800 Villejuif (FR); Université Paris-Sud, 91400 Orsay (FR); Institut National de la Santé et de la Recherche Médicale (INSERM), 75013 Paris (FR); Institut national de recherche pour l'agriculture, l'alimentation et l'environnement, 75007 Paris (FR)
(72) Inventor: ZITVOGEL, Laurence, F-75006 Paris (FR); VETIZOU, Marie, Bethesda, Maryland 20814 (US); LEPAGE, Patricia, 75014 Paris (FR)
(74) Representative: Casalonga
(86) International application number: PCT/IB2015/058200
(87) International publication number: WO 2016/063263

(56) References cited:
- WO-A1-2009/055362
- WO-A1-2014/153150
- DATABASE MEDLINE [Online] US NATIONAL LIBRARY OF MEDICINE (NLM), BETHESDA, MD, US; May 2001 (2001-05), LÓPEZ FORNAS F ET AL: "Comparative study of treatment with penicillin, ceftriaxone, trovafloxacin, quinupristin-dalfopristin and vancomycin in experimental endocarditis due to penicillin- and ceftriaxone-resistant Streptococcus pneumoniae.", XP002739456, Database accession no. NLM11328774 & LÓPEZ FORNAS F ET AL: "Comparative study of treatment with penicillin, ceftriaxone, trovafloxacin, quinupristin-dalfopristin and vancomycin in experimental endocarditis due to penicillin- and ceftriaxone-resistant Streptococcus pneumoniae.", THE JOURNAL OF ANTIMICROBIAL CHEMOTHERAPY MAY 2001, vol. 47, no. 5, May 2001 (2001-05), pages 623-629, ISSN: 0305-7453
- BAKKEN JOHAN S: "Fecal bacteriotherapy for recurrent Clostridium difficile infection.", ANAEROBE DEC 2009, vol. 15, no. 6, December 2009 (2009-12), pages 285-289, XP002754036, ISSN: 1095-8274
- DATABASE MEDLINE [Online] US NATIONAL LIBRARY OF MEDICINE (NLM), BETHESDA, MD, US; September 2010 (2010-09), OCHOA-REPÁRAZ J ET AL: "A polysaccharide from the human commensal Bacteroides fragilis protects against CNS demyelinating disease.", XP002739453, Database accession no. NLM20531465 & OCHOA-REPÁRAZ J ET AL: "A polysaccharide from the human commensal Bacteroides fragilis protects against CNS demyelinating disease.", September 2010 (2010-09), MUCOSAL IMMUNOLOGY SEP 2010, VOL. 3, NR. 5, PAGE(S) 487 - 495 ISSN: 1935-3456
- DATABASE MEDLINE [Online] US NATIONAL LIBRARY OF MEDICINE (NLM), BETHESDA, MD, US; 1 October 2010 (2010-10-01), OCHOA-REPÁRAZ JAVIER ET AL: "Central nervous system demyelinating disease protection by the human commensal Bacteroides fragilis depends on polysaccharide A expression.", XP002739454, Database accession no. NLM20817872
- GROSSO JOSEPH F ET AL: "CTLA-4 blockade in tumor models: an overview of preclinical and translational research.", CANCER IMMUNITY, vol. 13, no. 1, PAPER 5, 22 January 2013 (2013-01-22), pages 1-14, XP002739455, ISSN: 1424-9634
- VIAUD S ET AL: "Gut microbiome and anticancer immune response: really hot Sh*t!", CELL DEATH AND DIFFERENTIATION FEB 2015, vol. 22, no. 2, 16 May 2014 (2014-05-16), pages 199-214, XP055227164, ISSN: 1476-5403
- M. VETIZOU ET AL: "Anticancer immunotherapy by CTLA-4 blockade relies on the gut microbiota", SCIENCE, vol. 350, no. 6264, 5 November 2015 (2015-11-05), pages 1079-1084, XP55691620, US ISSN: 0036-8075, DOI: 10.1126/science.aad1329

## Description

### FIELD OF THE INVENTION

The present invention relates to the field of anticancer treatment. In particular, the present invention concerns the role of the microbiota in the efficacy of cancer treatments with a drug blocking an immune checkpoint, and provides methods and probiotics to improve the efficacy of such a treatment in patients in need thereof.

Any references in the description to methods of treatment refer to the compounds, pharmaceutical compositions and medicaments of the present invention for use in a method for treatment of the human (or animal) body by therapy (or for diagnosis).

### BACKGROUND AND PRIOR ART

Oncogenesis and cancer progression result from a complex interplay between cell-autonomous (epi)genetic instability and the microenvironment. Microbial communities inhabiting our intestine and other portals of entry thus far are poorly appreciated environmental factors potentially impacting on carcinogenesis. Pioneering studies performed in germ-free mice, animals exposed to specific bacteria in specialized facilities (gnotobiotic mice) or in antibiotic-treated mice, revealed an unsuspected role of commensals and pathobionts in accelerating tumorigenesis. Contrasting with these findings, other observations support a beneficial role of some bacteria against cancer. Thus, prolonged treatment of mice expressing transgenic Her2/Neu with a combination of metronidazole and ciprofloxacine actually tripled breast cancer occurrence (Rossini et al, 2006). In humans, several epidemiologic studies revealed a dose-dependent association between antibiotic use and breast cancer risk (Blaser, 2011; Velicer et al, 2004).

Adding some complexity, the clinical management of cancer patients compromises the delicate symbiosis between the gut microbiota and the host. Mucositis, a major oncological problem caused by anticancer chemotherapeutic agents, is worsened by neutropenia and antibiotics (Stringer et al, 2009; van Vliet et al, 2010). The crucial role of gut microbiota in eliciting innate and adaptive immune responses beneficial for the host in the context of effective therapies against cancer was recently reported (Viaud et al, 2014a; Viaud et al, 2014b; Viaud et al, 2013). By compromising intestinal integrity, chemotherapeutic agents enhance gut permeability and favor the selective translocation of Gram⁺ bacteria (*L. johnsonii* + *E. hirae*) into secondary lymphoid organs. There, anti-commensal pathogenic TH17 (pTh17) T cell responses are primed, facilitating the intratumoral accumulation of tumor-specific TH1 T cells that is associated with tumor regression after chemotherapy with cyclophosphamide (CTX) (Viaud et al, 2014a; Viaud et al, 2014b; Viaud et al, 2013). To demonstrate a causal relationship between gut microbiota and systemic pTh17 responses induced by CTX, animals that had been previously sterilized by means of antibiotics were treated with a cocktail of Gram⁺ bacteria (*L. johnsonii* + *E. hirae*), and it was found that this cocktail (but not *L. reuteri* or *L. plantarum*) could induce pTh17 in the spleen of CTX (but not vehicle) -treated animals (Viaud et al, 2013) and restored the CTX- anti-cancer effects lost in antibiotic-treated mice (unpublished data). Importantly, the redox equilibrium of myeloid cells contained in the tumor microenvironment is also influenced by the intestinal microflora, contributing to tumor responses (Iida et al, 2013). Hence, the anticancer efficacy of alkylating agents and platinum salts is compromised in germ-free (GF) mice, as well as in mice treated with antibiotics. These findings represent a paradigm shift in the understanding of the mode of action of conventional chemotherapeutics.

The inventors have now demonstrated that the first-in-class monoclonal antibody targeting an immune checkpoint (anti-CTLA4 mAb, Ipilimumab/YERVOY^{®}) also mediates T cell-dependent antitumor effects *via* an effect on the intestinal microbiota.

GROSSO JOSEPH F ET AL: "CTLA-4 blockade in tumor models: an overview of preclinical and translational research.",CANCER IMMUNITY, vol. 13, no. 1, PAPER 5, 22 January 2013 (2013-01-22), pages 1-14, discloses the treatment of tumours via delivery of anti-CTLA4 antibody; combinations with other entities for therapy are also disclosed.

Ipilimumab is a fully human monoclonal antibody (mAb) directed against CTLA4, a key negative regulator of T cell activation (Peggs et al, 2006). CTLA4, which is present in intracytoplasmic vesicles of resting T cells, is upregulated in activated T cells and translocates to the plasma membrane to maintain self-tolerance and prevent autoimmunity (Tivol et al, 1995). Ipilimumab is the first FDA- and EMA- approved therapeutic (since 2011) that improves the overall survival of patients with metastatic melanoma (MM) in randomized Phase III trials (Hodi et al, 2010; Robert et al, 2011). After more than 7 year-follow up, it appears that Ipilimumab can achieve about 20% durable (often complete) disease control in MM and other malignancies (Page et al, 2013). However, blockade of CTLA4 by Ipilimumab often results in immune-related adverse events (irAEs) at sites that are exposed to commensal flora, namely the gut and the skin (Beck et al, 2006; Berman et al, 2010; Weber et al, 2009). These irAEs can be of grade III-IV in 20% cases, life threatening (in <5% cases), resistant to steroids and anti-TNFa Ab, thereby causing premature interruption of the therapy. The enterocolitis associated with Ipilimumab has features similar to both graft-versus-host disease and inflammatory bowel disease, the acute phase being characterized by neutrophilic infiltrates while the chronic phase involves granulomata and lymphocytes. Although incriminated in the first place, neither low regulatory T cell (Treg) numbers nor specific genetic traits constitute predictive biomarkers for Ipilimumab-induced enterocolitis. Patients develop antibodies to enteric flora (Berman et al, 2010), suggesting that intestinal commensals or bacterial antigens may contribute to colitis. Despite these deleterious colitogenic effects, investigators currently combine blockade of two immune checkpoints *i.e*., CTLA4 and PD1 receptors, which further increases the response rate, as well as the incidence and severity of irAEs (Hamid et al, 2013).

Ipilimumab generates micro-abscesses in colons of diseased patients. The formation of abscesses is a pathological response to distinct bacterial pathogens, in particular the anaerobic bacterium *Bacteroides fragilis* (Tzianabos et al, 1993). The ability of *B. fragilis* to cause these infections is tightly linked to the presence of a unique capsular polysaccharide (polysaccharide A, PSA) on this organism. *Bacteroides* zwitterionic polysaccharides (ZPS), which contain alternating positive and negative charges in each repeating unit, are taken up by antigen-presenting cells, processed by the major histocompatibility class II pathway, and presented to T cells in the context of MHC class II molecules (Stingele et al, 2004). After ZPS presentation, CD4⁺ T cells can recognize and respond specifically to these carbohydrates. T cells can be activated *in vitro* by abscess-inducing ZPS in the presence of CD28-B7 co-stimulation and then promote abscess formation when adoptively transferred to the peritoneal cavity of rodents. A blocking CTLA4 Ig prevented abscesses following challenge with *Bacteroides fragilis* or a combination of *Enterococcus faecium* and *Bacteroides distasonis* (Tzianabos et al, 2000a). However, in the context of gut homeostasis, *B. fragilis* induces regulatory T cells (Treg) to secrete the potent anti-inflammatory cytokine IL-10, limiting inflammation in the gut and at distant sites (Mazmanian et al, 2008; Surana & Kasper, 2012). In the absence of antigen presenting cells, PSA elicits IL-10 secretion by CD4⁺ T cells in a TLR2-dependent manner (Round et al, 2011). In a mouse model of colitis, Dasgupta et al. (2014) showed that PSA required both innate and adaptive immune mechanisms to restore gut tolerance. Plasmacytoid dendritic cells (pDC), but not other conventional DC, sense *B. fragilis* PSA through TLR2, upregulate ICOSL and CD86 and activate IL-10-producing Treg in a CD86- and ICOS-dependent manner, promoting anti-inflammatory effects and avoiding colitis (Dasgupta et al, 2014).

*B. fragilis* requires PSA to occupy a mucosal niche in the colon. Hence, *B. fragilis* deltaPSA failed to colonize the colonic crypts and induced significant Th17 responses in the lamina propria. Depletion of Treg and neutralization of IL-17A decreased and markedly increased niche-specific mucosal colonization of *B.fragilis* respectively (Round et al, 2011). There is a species-specific saturable colonization of the colonic crypts by *B. fragilis* via a unique class of polysaccharide utilization loci (PUL) that are highly conserved among intestinal *Bacteroides.* This genetic locus has been named *ccf* for « Commensal Colonization Factors », determines the composition of the bacterial glycocalix and hence *Bacteroides spp.* bio distribution and functions (Huang et al, 2011; Koropatkin et al, 2009; Lee et al, 2013; Martens et al, 2008).

There is therefore a compelling need for the development of improved treatments for cancer which favor a constructive interaction, if not a synergy, between treatments comprising at least a drug blocking an immune checkpoint and immunity. Uncoupling efficacy from gut toxicity also represents an unmet medical need challenging the future development of immune checkpoint blockers as antineoplastic agents.

### SUMMARY

The present invention relates to a combination of an antibody blocking the CTLA4 immune checkpoint and of an antibiotic killing Gram positive bacteria and sparing Bacteroidales and/or favoring the outgrowth of Gram negative bacteria, for use in the treatment of cancer. Non-limitating examples of such antibiotics are vancomycin and penicillin G (benzylpenicilline sodique).

The invention also pertains to the use of vancomycin or penicillin G, for modulating the gut microbiota of a patient to potentiate the anticancer effects of an antibody blocking CTLA4 administered to said patient.

Also described herein is an *in vitro* method of identifying a patient who cannot be a good responder to a drug blocking CTLA4, comprising determining the functionality of the toll-like receptor 4 (TLR 4) in said patient, wherein if said patient lacks a functional TLR 4, the patient is identified as not being a good responder to a drug blocking CTLA4. This part of the description is not covered by the claims.

The description also provides probiotic bacterial compositions comprising bacteria selected from the group consisting of *Bacteroides fragilis, Bacteroides thetaiotaomicron, Bacteroides salyersiae, Bacteroides acidifaciens, Bacteroides intestinalis, Bacteroides vulgatus, Burkholderia cepacia, Burkholderia cenocepacia, Bacteroides uniformis, Bacteroides massiliensis and Barnesiella intestinihominis* and mixtures thereof. According to the invention, compositions comprising at least *Bacteroides fragilis, Bacteroides thetaiotaomicron* and/or *Burkholderia cepacia* can be used in combination with an antibody blocking CTLA4 for inducing immunostimulation in a cancer patient.

The present description also relates to the use of a dendritic cell (DC) presenting antigens from one or several bacteria selected from the group consisting of *Bacteroides fragilis, Bacteroides thetaiotaomicron, Bacteroides salyersiae, Bacteroides acidifaciens, Bacteroides intestinalis, Bacteroides vulgatus, Burkholderia cepacia, Burkholderia cenocepacia, Bacteroides uniformis, Bacteroides massiliensis and Barnesiella intestinihominis,* and more specifically capsular polysaccharides A (PSA) or PSA and adjuvants or zwitterionic polysaccharide (ZPS) repeated motifs from *B. fragilis* or lysine-aspartic acid (KD) peptides with >15 repetitive units, for use in adoptive cell transfer (DC), in combination with an antineoplastic treatment comprising a drug blocking an immune checkpoint, for treating cancer. This part of the description is not covered by the claims.

Also herein described is the use of a polyclonal T cell line or bulk autologous T cells *ex vivo* expanded with dendritic cells (DC) presenting antigens from one or several bacteria selected from the group consisting of *Bacteroides fragilis, Bacteroides thetaiotaomicron, Bacteroides salyersiae, Bacteroides acidifaciens, Bacteroides intestinalis, Bacteroides vulgatus, Burkholderia cepacia, Burkholderia cenocepacia, Bacteroides uniformis, Bacteroides massiliensis and Barnesiella intestinihominis,* and more specifically capsular polysaccharides A (PSA) or PSA and adjuvants or zwitterionic polysaccharide (ZPS) repeated motifs from *B. fragilis* or lysine-aspartic acid (KD) peptides with >15 repetitive units, for use in adoptive cell transfer of T lymphocytes, in combination with an antineoplastic treatment comprising a drug blocking an immune checkpoint, for treating cancer. This part of the description is not covered by the claims.

An anticancer vaccine composition comprising PSA and/or KD and/or ZPS, as well as its use in combination with a drug blocking an immune checkpoint, for inducing immunostimulation in a cancer patient, is also described but is not covered by the claims.

Other aspects of the disclosure comprise methods for *ex vivo* determining whether a cancer patient is likely to benefit from a treatment with an immune checkpoint blocker, either by assessing the presence of memory Th1 cells towards *Barnesiella intestinihominis, Bacteroides fragilis, Bacteroides thetaiotaomicron, Burkholderia cenocepacia* and/or *Burkholderia cepacia* in a blood sample from said patient, or by analysing the gut microbiota in a feces sample from said patient. These aspects are not covered by the claims.

### LEGENDS TO THE FIGURES

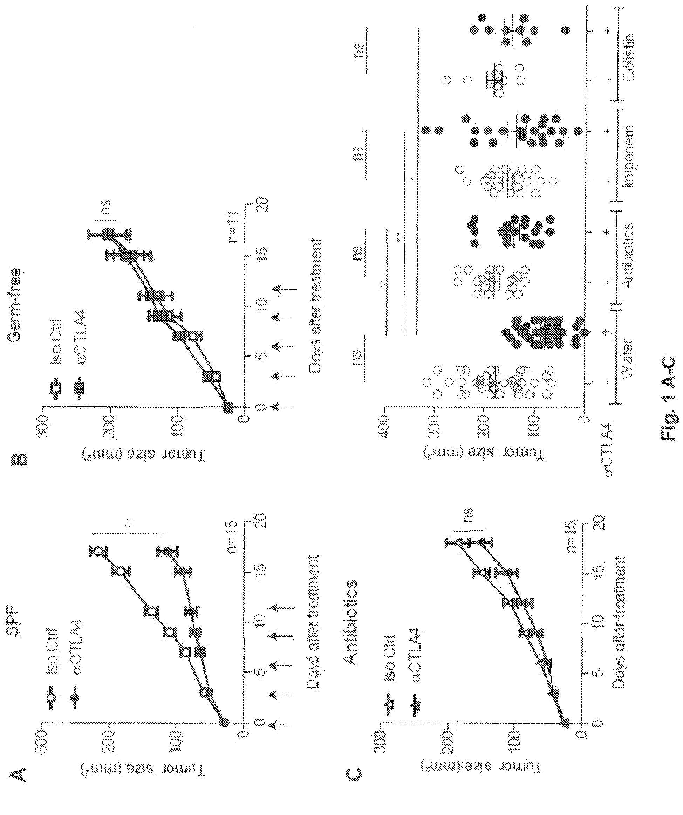

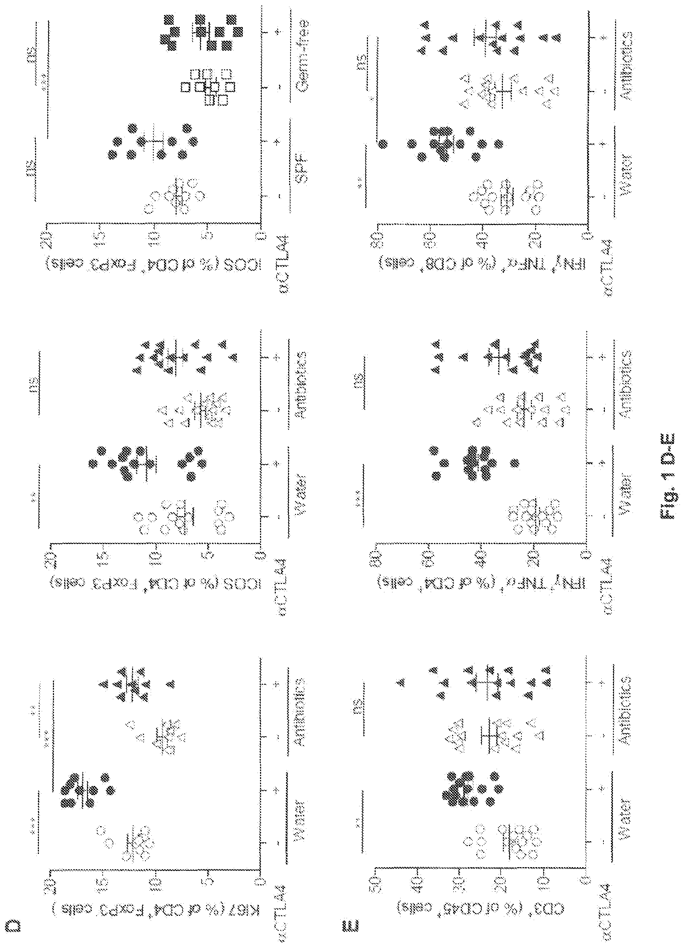

### Figure 1. Microbiota-dependent immunomodulatory effects of anti-CTLA4 Ab.

*A-B. Lack of antitumor activity of 9D9 Ab in germ-free (GF) mice.* Tumor growth kinetics of day 5 established -MCA205 sarcoma treated with 5 injections (indicated by arrows) of 9D9 anti-CTLA4 (αCTLA4) or isotype control (Iso Ctrl) mAb, are shown in animals reared in normal, specific pathogen free (SPF) (A) or germ-free (GF) (B) conditions in one representative experiment out of three. C. *Effects of oral antibiotics on the antitumor effects of anti-CTLA4 Ab againstMCA205.* Id. as in A-B in the presence (C, left panel) of a combination of broad-spectrum antibiotics (ampicillin, colistin, streptomycin, ACS) or the indicated single antibiotic regimen (C, right panel) in the drinking water. *D-E. Effects of oral antibiotics on the immunostimulatory activity of anti-CTLA4 Ab.* Flow cytometry analyses of Ki67 and ICOS expression on effector CD4⁺Foxp3⁻T cells (D) harvested from the spleen two days after the third administration of 9D9 or Iso Ctrl Ab (in the same setting as in A, or at day 20 for the right panel in GF) as well as of CD3⁺ T cells among CD45⁺ tumor infiltrating cells (E, left panel) or intracellular staining for Th1 cytokines in CD4⁺ or CD8⁺ TILs after PMA+ionomycin stimulation (E, middle and right panel). Each dot represents one mouse and graphs depict two to three independent experiments of 5 mice/group. ANOVA statistical analyses: *p<0.05, **p<0.01, ***p<0.001, ns: not significant.

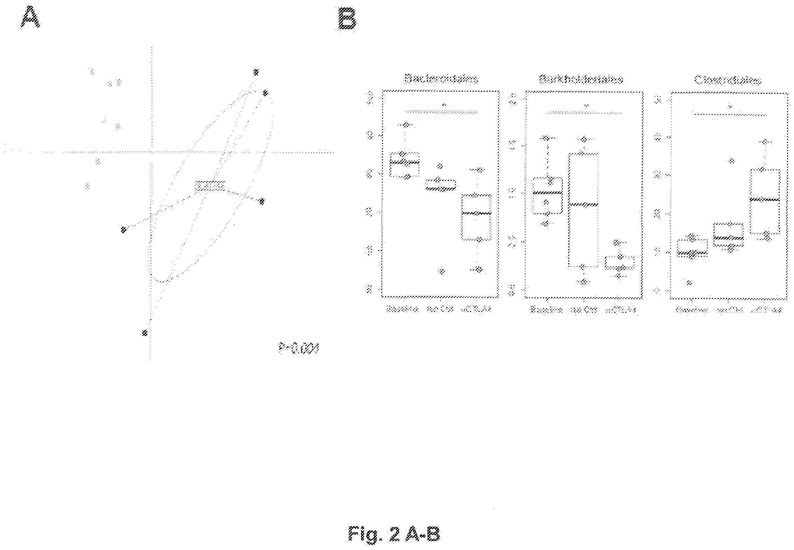

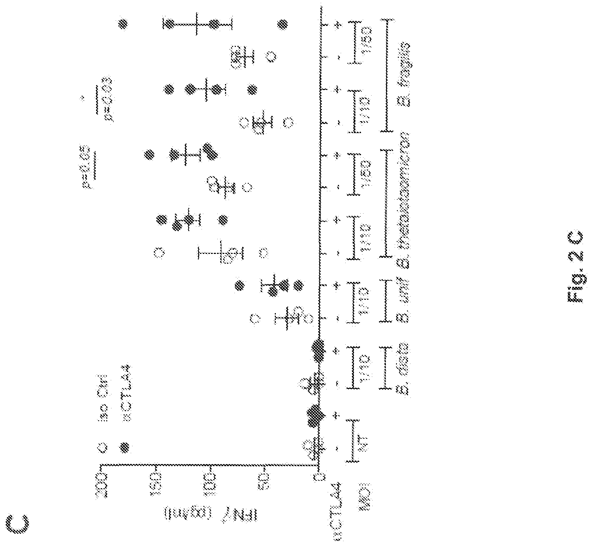

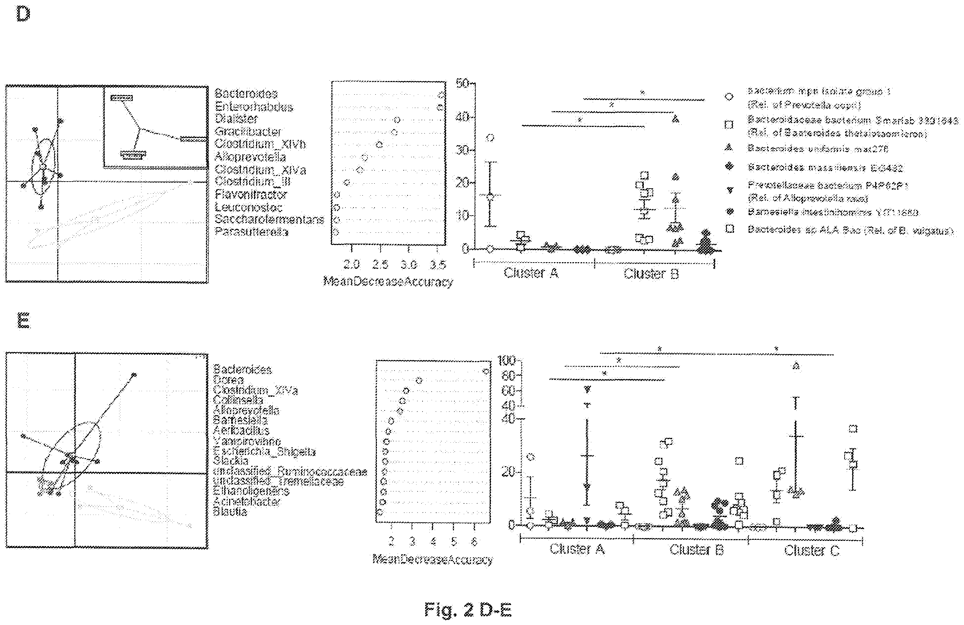

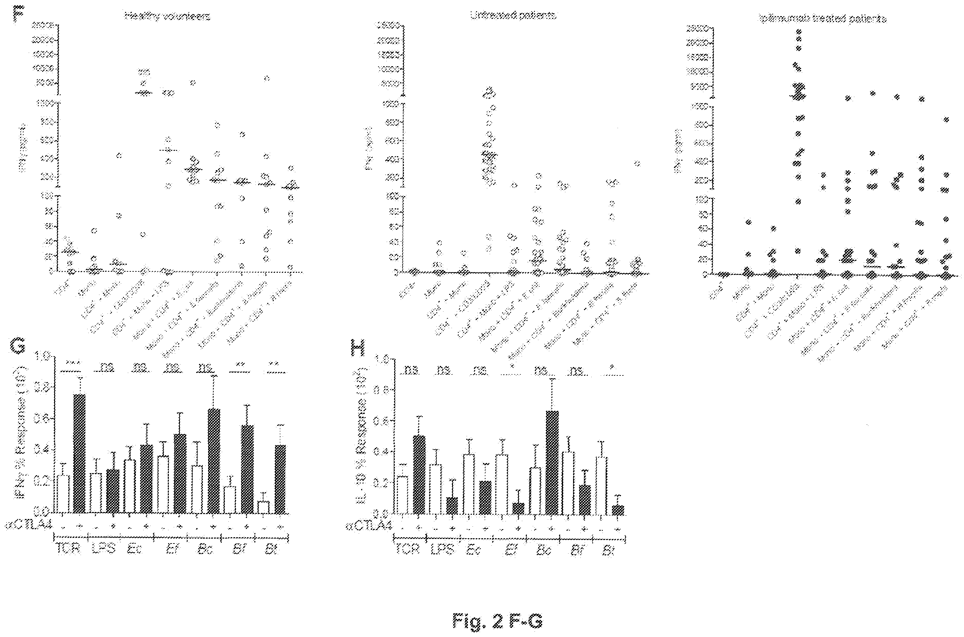

### Figure 2. Memory T cell responses against Bacteroidaceae in mice and patients.

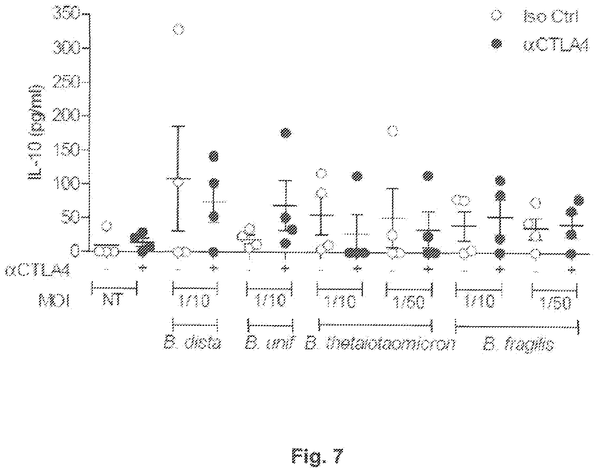

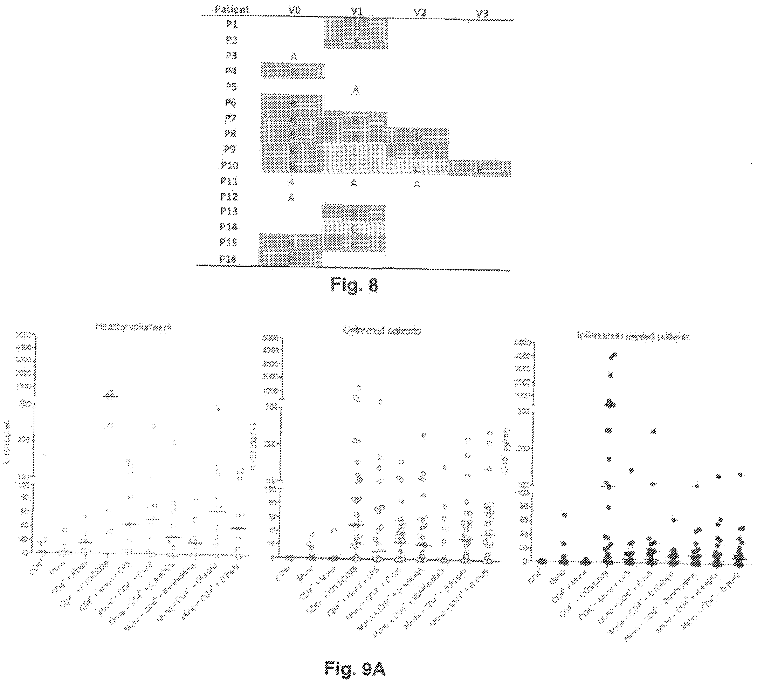

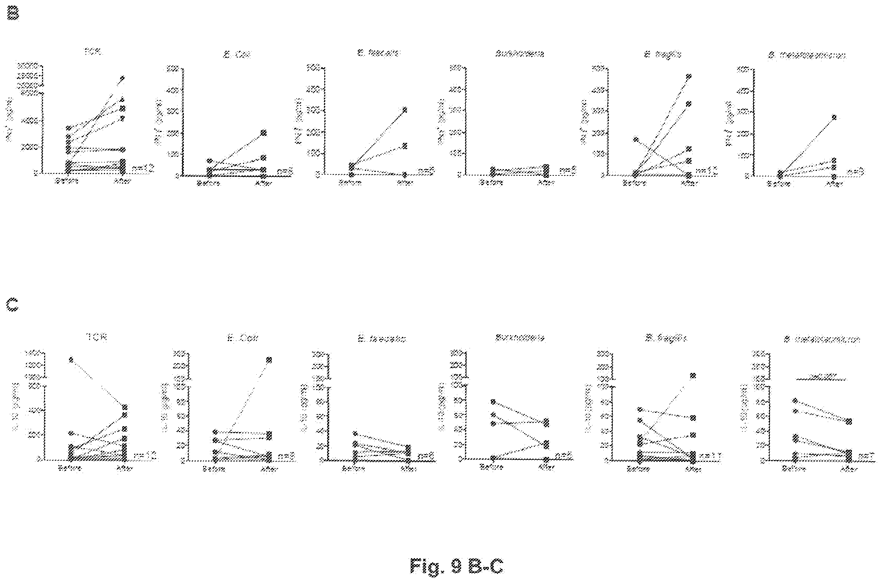

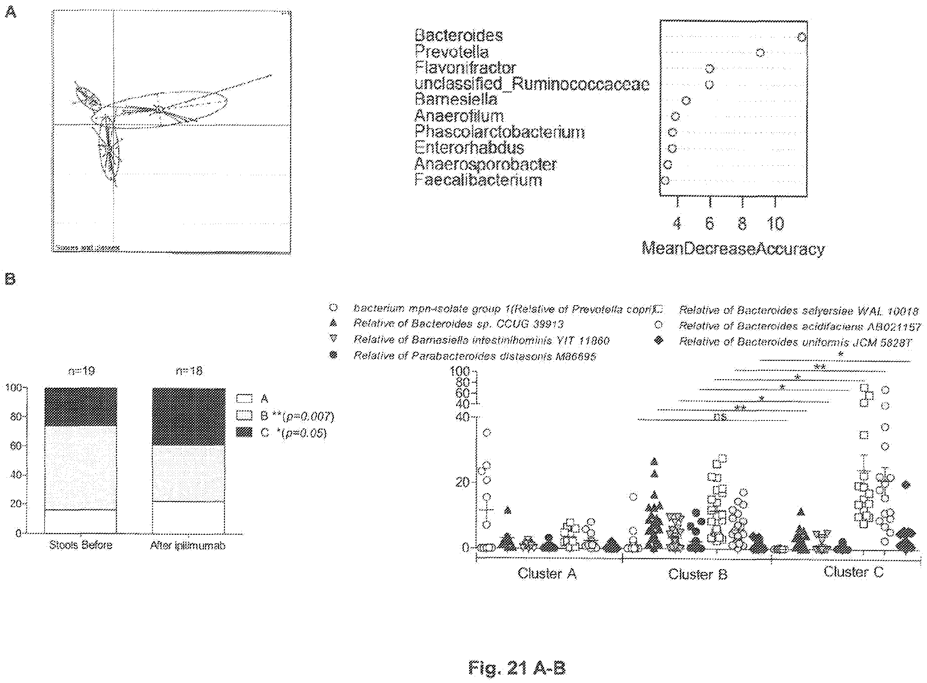

*A. Principle component analyses (PCA) of the effects mediated by CTLA4 blockade in tumor bearers.* PCA on a relative abundance matrix of genus repartition highlights the clustering between controls (baseline; n=6), isotype controls (Iso ctrl; n=5) and anti-CTLA4 treated animals after one ip injection (αCTLA4, n=5). Ellipses are presented around the centroids of the resulting 3 clusters. The first two components explain 34.41 % of total variance (Component 1: 20.04%; Component2: 14.35%). Based on a Monte-Carlo test with 1000 replicates, a significant difference was found between the three clusters (*p* = 0.0049). *B. Relative loss of Bacteroidales and Burkholderiales orders induced by anti-CTLA4 Ab.* Pyrosequencing of 16S rRNA gene amplicons of feces from tumor bearers before and 48 hours after one ip administration of 9D9 or iso. Ctrl Ab. Means ±SEM of relative abundance for each 3 orders for 5 mice/group are shown. *C. Recall responses of CD4⁺ T splenocytes to various bacterial strains in 9D9 Ab* (*or Iso. Ctrl Ab) treated-mice.* BM-DC loaded with 30 × 10⁶ (1:10 DC:bacterium ratio) or 150 × 10⁶ (1:50) bacteria of the indicated strain were incubated with CD4⁺ T cells harvested from the spleen at day 8 (two days after 3 ip inoculations of 9D9 or Iso Ctrl Ab). The graph represents IFNγ concentrations of the 24 hours-coculture media (the IL-10 levels shown in Fig. 7) for each spleen (one dot). *D-E. Melanoma patients clustering based on genus composition revealing the importance of Bacteroidaceae during therapy with ipilimumab.* The k-means clustering algorithm was applied based on genus composition before (D) and after (E) ipilimumab. The number and quality of clusters were validated using the Calinski-Harabasz Index (12), which showed good performance in recovering two clusters before therapy. PCA analysis of patients distribution based on bacterial genus composition depending on cluster A or B classification before therapy (D, left panel)) and on an additional cluster C after therapy (E, left panel) highlighted a significant clustering (Monte-Carlo test, *p* = 0.046 (D), Monte-Carlo test, *p* = 0.07 (E)). Random Forest analysis was applied to decipher main genera responsible for this significant clustering (D & E, middle panels). The relative abundance of *Bacteroides* and *Alloprevotella* species significantly represented between clusters are indicated in the graph (D & E, right panels). Figure 21A shows a refined version of this clustering, obtained with an increased number of MM patients, both before and during treatment with Ipilimumab. *F-G. Selective restoration of T cell unresponsiveness to B. fragilis* and *B. thetaiotaomicron in advanced cancer patients with ipilimumab.* CD4⁺CD45RO⁺ T cells from PBMC of 11 healthy volunteers or 29 advanced patients before and after at least 2 injections of ipilimumab were restimulated with autologous CD14⁺ cells (sorted prior to ipilimumab) loaded with various bacterial strains (as indicated, bacteria neutralized with antibiotics before coculture with T cells) in duplicate wells. IPNγ (F) and IL-10 (Fig. 9) were monitored in the supernatants by commercial ELISA or by luminex Magpix technology at 48 hours. No cytokine release was observed in the absence of bacteria or with loaded monocytes in the absence of T cells. Each dot represents IFNγ secretion of an individual patient. Longitudinal analyses could be performed on 5 to 12 patients out of the 29 presented (Fig. 9b-c). The percentages of melanoma patients presenting with IFNγ or IL-10 values above the mean (calculated on the absolute values of the melanoma patient cohort before ipilimumab) are indicated, before and after ipilimumab (G). Student t-test statistical analyses: *p<0.05, **p<0.01, ns: not significant.

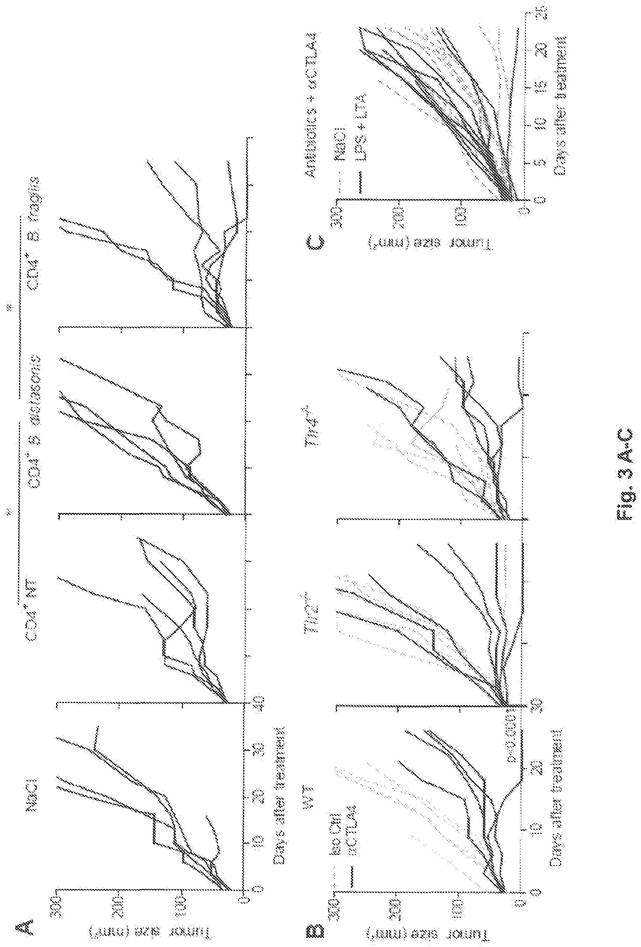

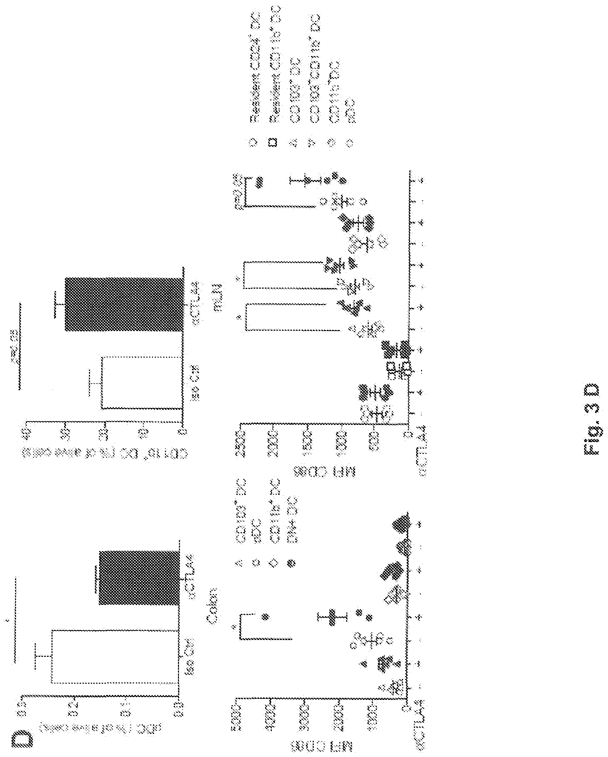

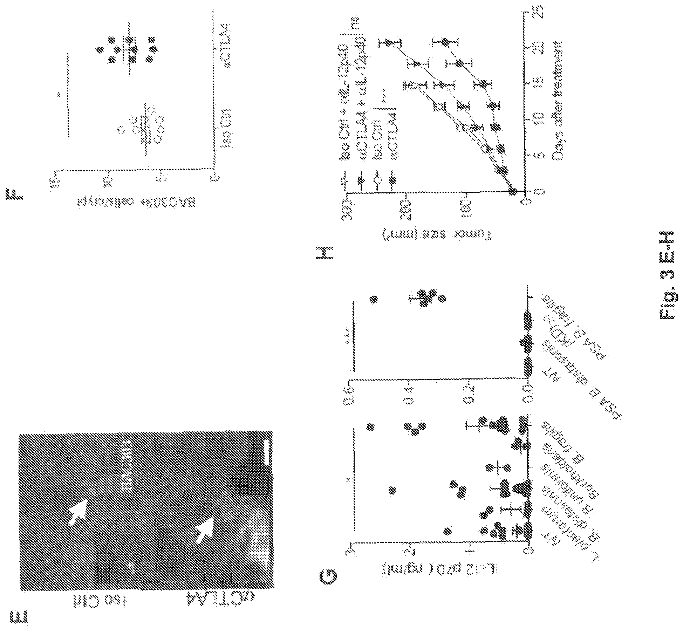

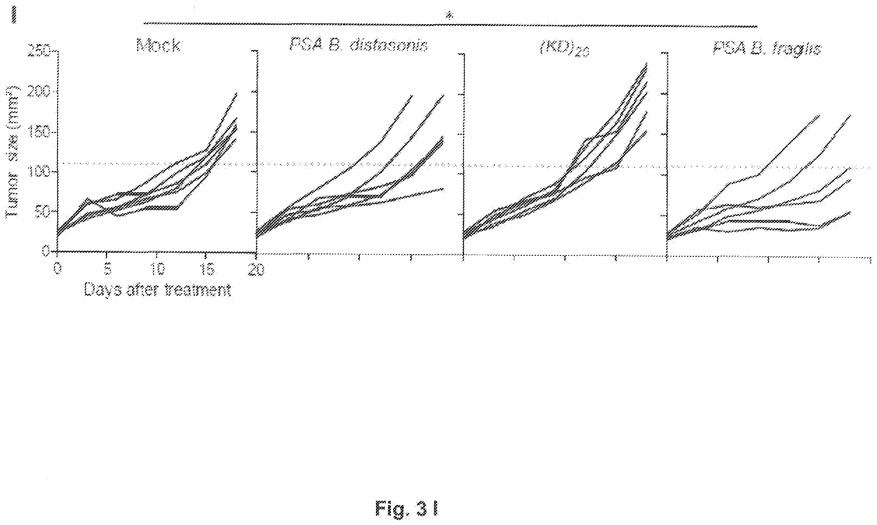

### Figure 3. IL-12-dependent Th1 cognate immune responses against B. fragilis in the antitumor effects of CTLA4 blockade.

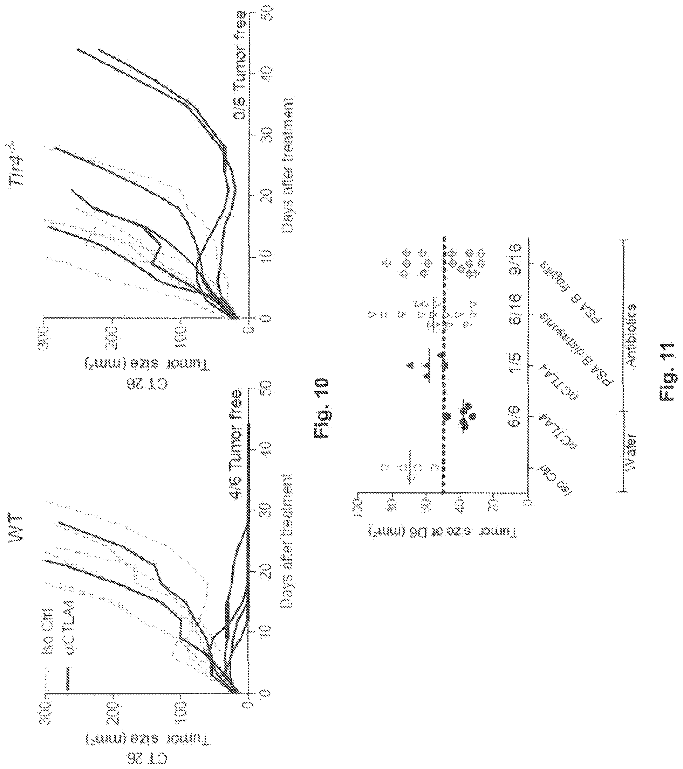

*A. Adoptive T cell transfer of T cells restimulated with Bacteroidales species into GF tumor bearers treated by CTLA4 blockade.* One million T cells harvested from spleens of mice exposed to anti-CTLA4 Ab and restimulated with *B. fragilis* versus *B. distasonis* (as stated above in Fig. 2C. at 1:10 ratio) or BM-DC alone (CD4⁺ NT) were infused iv in day 6 MCA205 -tumor bearing GF mice. A representative experiment containing 5-6 mice/group is shown. Similar conclusions were drawn from adoptive T cell transfer in antibiotics-treated mice (not shown). *B-C.Partial role of TLR2 and TLR4 in the tumoricidal activity of anti-CTLA4 Ab.* Same setting as in Fig. 1A. in WT versus *Tlr2* or *Tlr4* gene deficient-mice. Tumor growth kinetics of MCA205 sarcoma in a representative experiment of 5-7 mice/group, out of two yielding similar conclusions in C57BL/6 mice (or BALB/c in Fig. 10) (B). 500 µg of *E. Coli* LPS or lipoteichoic acid were orally fed to tumor bearers pretreated with antibiotics as described in Fig. 1A. Tumor growth kinetics of 5 animals/group are depicted (C). *D. Changes in DC subsets in the colonic lamina propria (LP) during CTLA4 blockade.* Enumeration of percentages of pDC and CD11b⁺ DC gating in CD45⁺CD11c⁺I-A^{b+}cells by flow cytometry of colonic LP harvested after 2 injections of 9D9 (versus Iso Ctrl) Ab in 6 naive animals in representative dot plot analysis indicating the mean percentages ±SEM. Mean expressions ±SEM of CD86 in DC subsets of mesenteric LN or colon LP are depicted in the graph for 6 animals per group. *E-F. FISH using BAC303 probe identifying Bacteroides at the bottom of the crypts in ilea and colons.* A representative micrograph of Iso Ctrl and αCTLA4 Ab-treated mice and concatenated data of two experiments, are shown, each dot representing one mouse. Scale in pictures represents 100µm.. *G-I. Role of IL-12 in the antitumor effects of CTLA4 blockade.* IL-12p70 production by CD11b⁺ DC loaded with *B. fragilis* or PSA enriched fractions of *B. fragilis* and other isolates (as listed) as determined in commercial ELISA at 24 hrs (G) in triplicate wells in 1-4 experiments (each dot representing one well). Neutralizing anti-IL-12p40 Ab (or irrelevant Iso Ctrl Ab) administered in experimental settings described in Fig. 1A. One representative experiment out of two performed in C57BL/6 is depicted in H. (identical results in CT26 treated with anti-CTLA4 and anti-IL-12p40 Ab in BALB/c mice, not shown). I. Intravenous adoptive transfer of 1 × 10⁶ BM-DC pulsed with PSA enriched fractions of *B. fragilis* (or polysaccharide -enriched capsids of *B. distasonis*), synthetic KD peptides, or unpulsed (mock) controls, in tumor bearers treated with antibiotics and then anti-CTLA4 Ab. A representative experiment out of two yielding similar results of tumor growth kinetics for 5 mice/group is depicted. Anova statistical analyses: *p<0.05, **p<0.01, ***p<0.001, ns: not significant.

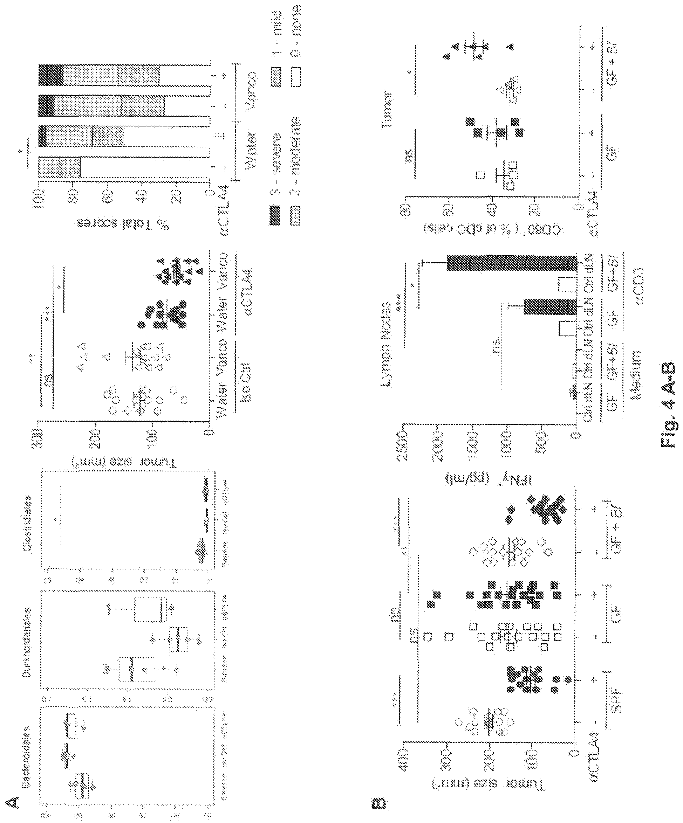

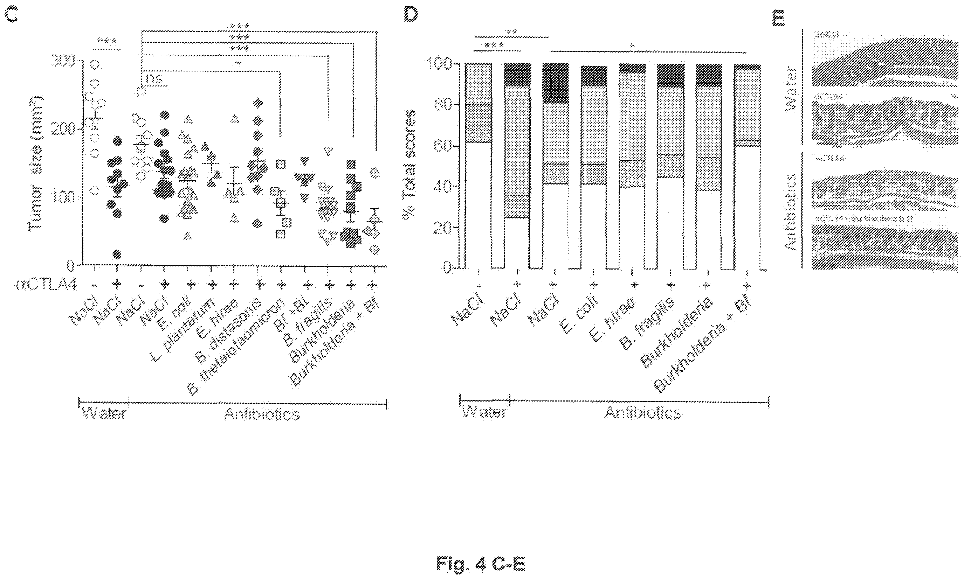

### Figure 4. B. fragilis (alone or combined with B. cepacia) accounts for the efficacy of anti-CTLA4 Ab.

*A. Vancomycin augments the tumoricidal effects of CTLA4 blockade.* Vancomycin prevented the loss of *Bacteroidales* and *Burkholderiales* orders induced by anti-CTLA4 Ab (left panel). Pyrosequencing of 16S rRNA gene amplicons of feces of tumor bearers before and 48 hours after one ip administration of 9D9 or Iso Ctrl Ab in the setting of a vancomycin (or water) conditioning, as presented in Fig. 2A. Means +SEM of relative values for each order for 5 mice/group are shown (A, left panel). The effects of Vancomycin (vanco) -conditioning on mice inoculated at day 14 with MCA205 and treated at day 2 post-tumor inoculation with 9D9 (or iso Ctrl) Ab are shown as in Fig. 1C. Tumor sizes at day 14 post-tumor inoculation are depicted for 2 experiments (A, middle panel). Histopathological score of colonic mucosae in vancomycin-treated tumor bearers receiving anti-CTLA4, established on H&E stained colons as described in M&M at day 20 in 5 animals/group for at least 6 independent areas (A, right panel). Anova statistical analyses: *p<0.05, **p<0.01, ***p<0.001, ns: not significant. *B. B. fragilis restores the efficacy of CTLA4 blockade in GF mice and modulates the microenvironmental tone of the tumor.* Oral feeding of tumor bearing-mice reared in SPF and GF conditions (as in Fig. 1) with *B. fragilis.* Tumor sizes at day 15 post-tumor inoculation are depicted (B, left panel). Each dot represents one tumor size and graphs depict two to three independent experiments of 5 mice/group. Tumor draining-and contralateral lymph nodes were harvested at day 20 post-tumor inoculation and stimulated with anti-CD3 Ab for 48 hrs. IFNγ concentrations were monitored in culture supernatants using commercial ELISA. Means±SEM of IFNγ release for 5mice/group are shown (B, middle panel). Flow cytometry determination of CD80 expression on CD11c⁺I-A^{b+}CD45⁺ tumor infiltrating cells in one representative experiment out of two yielding similar results (B, right panel). C. *Tumoricidal effects of mono-or bi-association of mice with B. fragilis and*/*or various bacterial strains by oral feeding in broad spectrum antibiotics -treated mice.* Tumor sizes at day 15 post-tumor inoculation are depicted (B, left panel). Each dot represents one tumor size and graphs depict two to three independent experiments of 5 mice/group. *D-E. Histopathological score of colonic mucosae in antibiotics-treated tumor bearers receiving anti-CTLA4 after oral gavage with various bacterial strains.* Same setting as in C. with histological assessment of H&E stained colons monitoring microscopic lesions as described in M&M at day 20 in 5 animals/group on at least 6 independent areas. Representative micrographs are shown (E) for NaCl/water and ACS/*Burkholdheria*+*Bf* groups receiving αCTLA4 Ab. ANOVA statistical analyses: *p<0.05, **p<0.01, ***p<0.001, ns: not significant. Scale in pictures represents 100µm.

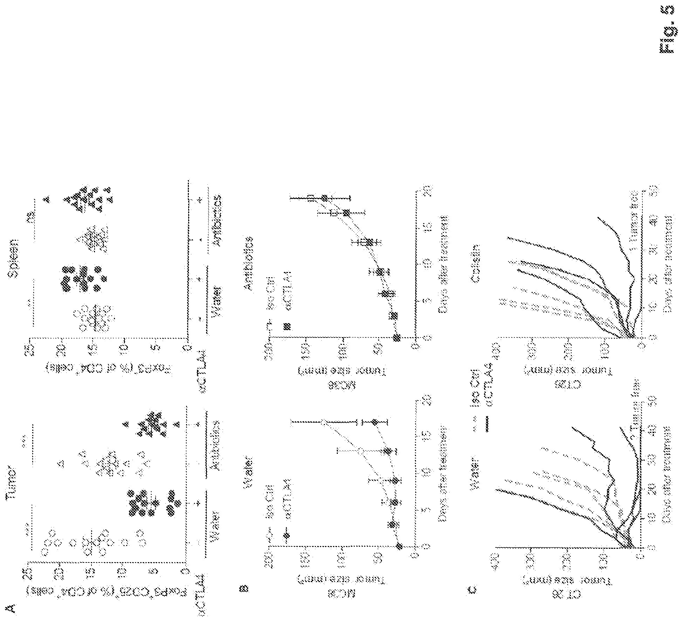

**Figure 5****. The gut microbiota is implicated in the immunostimulatory properties of anti-CTLA4 mAb in the MC38 and CT26 colon carcinoma models.** *A. Modulation of Tregs with antibiotics.* The percentages of CD4⁺Foxp3⁺ Tregs present in tumors and spleens of MCA205 -bearing C57BL/6 mice treated or not with ACS and anti-CTLA4 or isotype control mAbs were determined by flow cytometry. Three experiments comprising 5 mice/group are pooled, each dot representing one mouse. Data were analysed by one-way ANOVA test: *** *p* < 0.001, ** *p <* 0.01, ns = not significant. *B-C. Effects of ACS or colistin in the efficacy of CTLA4 blockade against transplantable colon cancers.* One representative graph out of two independently performed experiments (n = 5 per group) is depicted. C57BL/6 or BALB/c mice previously fed antibiotics (broad spectrum or colistin) or water as control, in the drinking water to deplete gut microbiota were implanted s.c. with MC38 (B) or CT26 (C) colon carcinoma cells respectively. Mice were subsequently treated with anti-CTLA4 or isotype control mAbs, when implanted tumors were palpable, and tumor growth was followed over 20 days. Data were analysed by one-way ANOVA with Bonferroni's multiple comparison test. * *p <* 0.05, ** *p <* 0.01, ns = not significant.

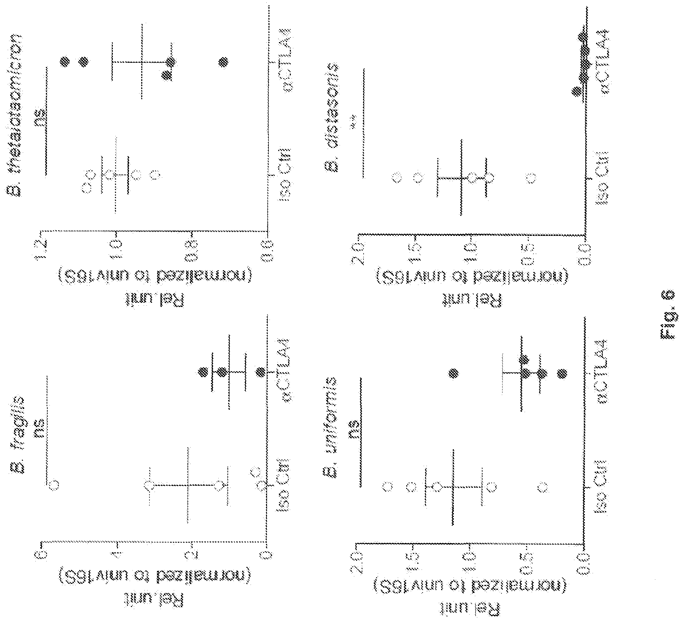

**Figure 6****. *Bacteroides* spp. are decreased in fecal samples following anti-CTLA4 therapy.** The levels of *B. fragilis, B. thetaiotaomicron, B. uniformis,* and *B. distasonis* present in fecal samples were determined by qRT-PCR, relative to universal 16S rRNA as detailed in Materials & Methods. Data shown are from one experiment out of two yielding similar results and depict the means ± SEM (*n* = 5 per group). ***p <* 0.01, ns = not significant, by unpaired two-tailed t-test.

**Figure 7****. IL-10 release by memory T cell splenocytes exposed to *Bacteroides spp.* after CTLA4 blockade.** Same experimental setting as that described in Fig. 2c monitoring IL-10 in ELISA from supernatants of mixed cocultures of BM-DC and CD4⁺ T cells with *Bacteroides spp.*

**Figure 8****. Clustering of patients before and during ipilimumab therapy.** V0 represents the clustering before ipilimumab, V1-V2-V3 at 3 weeks post-1^{st}, 2^{nd} and 3^{rd} administration respectively.

**Figure 9****. Paired analyses of Th1 and Tr1 memory T cell responses towards *Bacteroides spp.* and other commensals before and after ipilimumab.** Same experimental setting as described in Fig. 2F-G, showing the IL-10 concentrations in the supernatants of HV and patients in the coculture assays (A) and using paired specimen for each patient (B-C). Results were analyzed in a paired Student t-test (B-C).

**Figure 10****. *Tlr4*^{*-*/*-*} mice partially lose anti-CTLA4-mediated tumor control.** BALB/c WT and *Tlr4-*/*-* mice bearing CT26 tumors were administered anti-CTLA4 or isotype control as detailed before. Tumor growth and tumor-free animals were recorded as shown. Data shown are from one of two independently performed experiments (n = 6).

**Figure 11****. Antitumor effects mediated by oral feeding with capsular polysaccharide enriched fractions of *Bacteroides* spp.** C57BL/6 ACS-treated mice were orally administered the enriched capsular polysaccharide fractions of *B. distasonis,* or *B. fragilis,* at doses of 0.5, 5, or 50 µg per mouse, one day after treatment with anti-CTLA4 mAb. Control mice received anti-CTLA4 mAb alone, with or without prior ACS administration, or isotype control mAb without ACS. Tumor sizes from mice 6 days following mAb with PSA doses for each *Bacteroides* spp. combined. The number of protected mice, as determined for the anti-CTLA4 Ab-treated control group, are displayed.

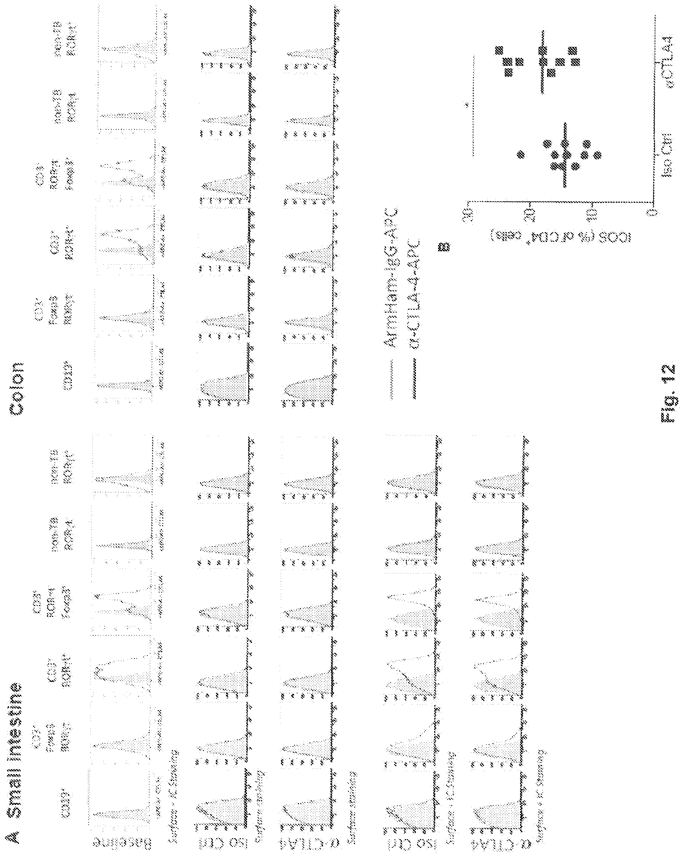

**Figure 12****. CTLA-4 expression on lymphocyte subsets of the intestines at baseline and post-CTLA4 blockade.** Intestinal lymphocytes were isolated from the small intestines and colons. CTLA-4 expression was determined (surface and intracellular staining combined) (A, baseline) as well as surface expression without permeabilization (B, after aCTLA4 Ab) in the indicated lymphocytic populations. Representative overlay are shown for one experiment. C. ICOS expression determined by flow cytometry analyses of LP CD4⁺ lymphocytes before and after aCTLA4 Ab is shown in one representative experiment.

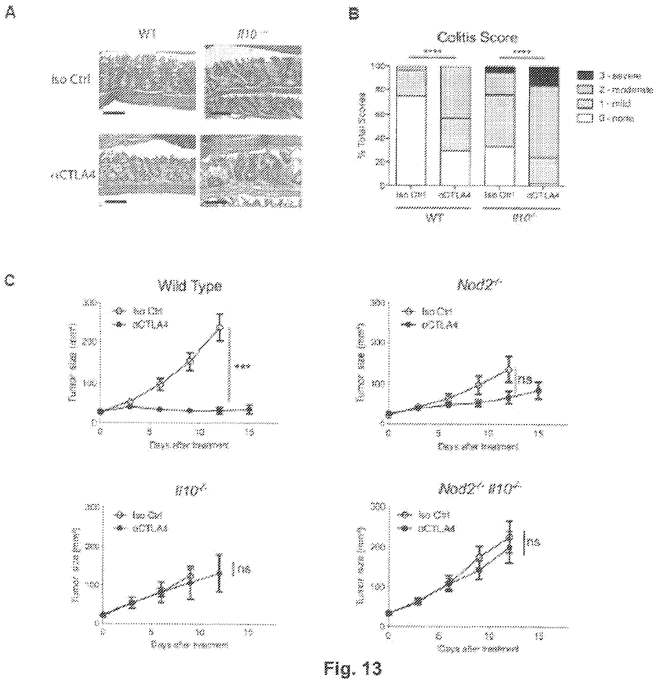

**Figure 13****. Co-blockade of IL-10 and CTLA4 provokes colitis and tumor escape.** Small intestines and colons from *Il10-*/*-* or WT C57BL/6 mice were taken and processed for histopathological scoring (as detailed in the Materials & Methods) from MCA205 tumor-bearing mice 8 days after beginning αCTLA4 (or isotype control) mAb therapy as detailed before. **A** shows a representative picture of colon sections from each group. The level of colitis was scored from at least 8 distinct regions from each mouse **(B). C.** Tumor growth kinetics with or without aCTLA4 Ab in various genetic backgrounds in BALB/c mice. BALB/c WT, *Il10*^{*-*/*-*} and *Nod2*^{*-*/*-*} and *Il10*^{*-*/-} x *Nod2*^{*-*/*-*} mice bearing CT26 tumors were administered anti-CTLA4 or isotype control as before, and tumor growth kinetics were recorded and depicted in **C.** Scale bar in **A** is 100 µm. Data shown in **C** depict the means ± SEM (*n* = 5-8). **** *p <* 0.0001 by Kruskal-Wallis non-parametric test with Dunn's multiple comparison test **(B).** *** *p* < 0.001 , ns: non significant, by unpaired two-tailed t-test. Scale in pictures represents 100µm.

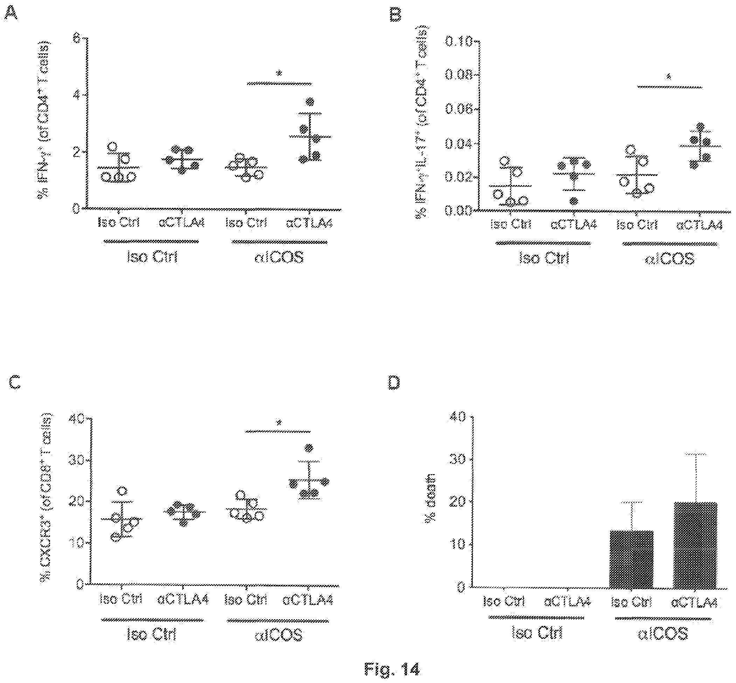

**Figure 14****. Co-blockade of ICOS and CTLA4 induced overt Th1 responses and animal death. A-C.** *Th1, pTH17 and Tc1 systemic immunity during the ICOS*/*CTLA4 co-blockade.* MCA205 tumor-bearing C57BL/6 mice were administered anti-ICOS or isotype control mAbs on commencement of αCTLA4 (or isotype control) mAb therapy. At day 10 post mAb treatments, splenocytes were isolated and IFN-γ⁺ **(A)** and IFN-γ⁺IL-17⁺ **(B)** CD4⁺ T cells and CXCR3⁺ CD8⁺ T cells **(C)** were determined by flow cytometry following 4h stimulation with PMA and ionomycin. **D.** *Lethal effects of the ICOS*/*CTLA4 coblockade.* Shows the percentage death per group of mice from three independent experiments. Data shown depict the mean ± SEM (*n* = 5). * *p* < 0.05 by one-way ANOVA with Bonferroni's multiple comparison test.

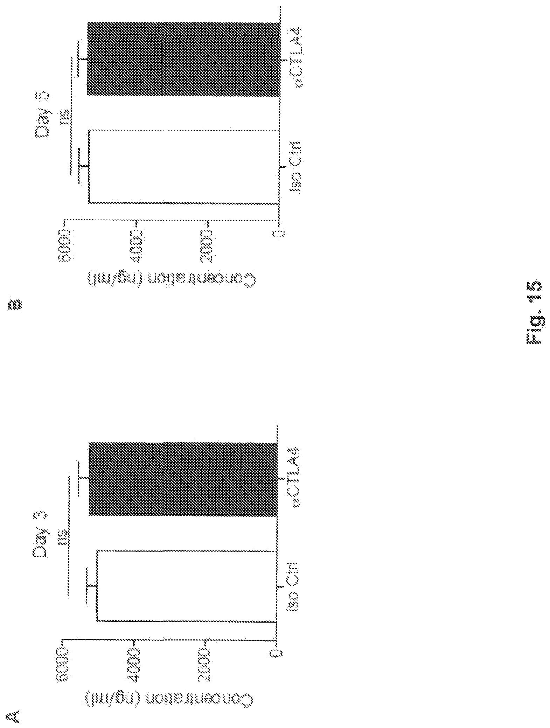

**Figure 15****. Intestinal permeability is not disrupted following CTLA4 blockade in vivo.** C57BL/6 mice were administered either αCTLA4 or isotype control mAb from when implanted tumors reached 25mm² in size. Two days later, mice were water-starved overnight, and the following day (day 3 post mAb dosing), mice were orally administered FITC-dextran. After 4 hours, blood was collected from each mouse by cardiac puncture and the blood concentration of FITC-dextran was determined **(A).** The same procedure was carried out in a separate group of mice that had received two anti-CTLA4 (or isotype control) mAb doses **(B).** Data depict the mean ± SEM. ns = not significant by unpaired two-tailed t-test.

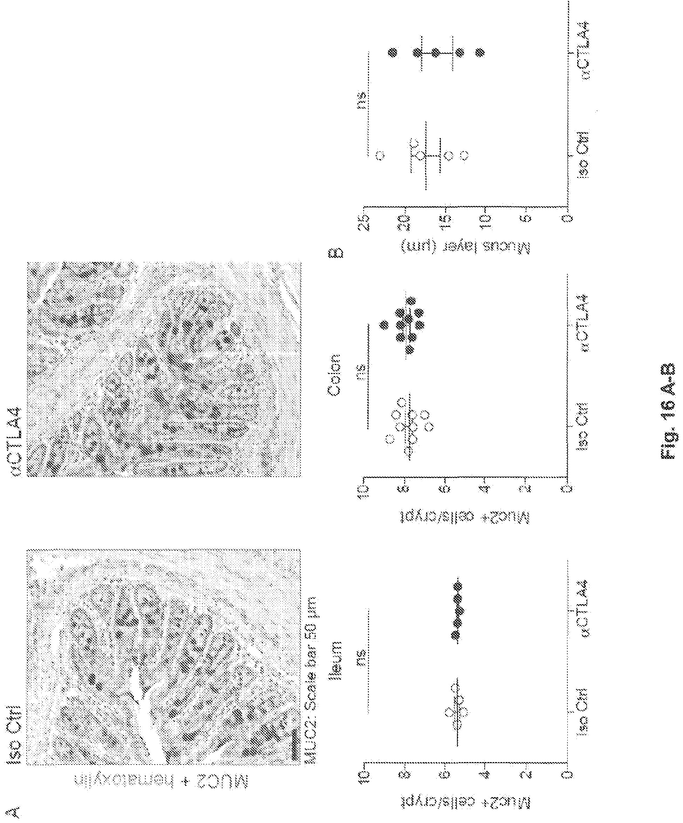

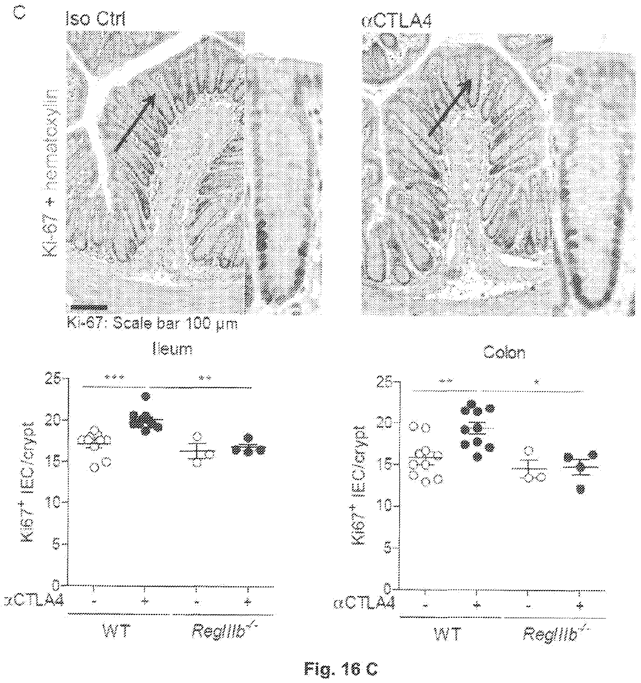

**Figure 16****. Assessment of goblet cells, mucus layers and proliferation of epithelial cells in intestines during CTLA4 blockade.** Small intestines and colons were taken and processed for immunohistochemistry and immunofluorescence staining in naïve WT versus Reg3b deficient C57BL/6 mice before and 48 hours after the first αCTLA4 (or isotype control) mAb inoculation. The number of Muc2⁺ goblet cells (upper panels) and Ki67⁺ IEC (lower panels) were counted for each villus of the distal ileum from **(A)** and in colonic LP **(B)** at least 8 distinct regions from each mouse. A representative micrograph picture is shown for IEC proliferation at 48 hrs post-1^{st} injection of mAb (C, D). Data depict the means ± SEM of values. *** *p* < 0.001 by Mann-Whitney non-parametric test. ns = not significant by unpaired two-tailed t-test.

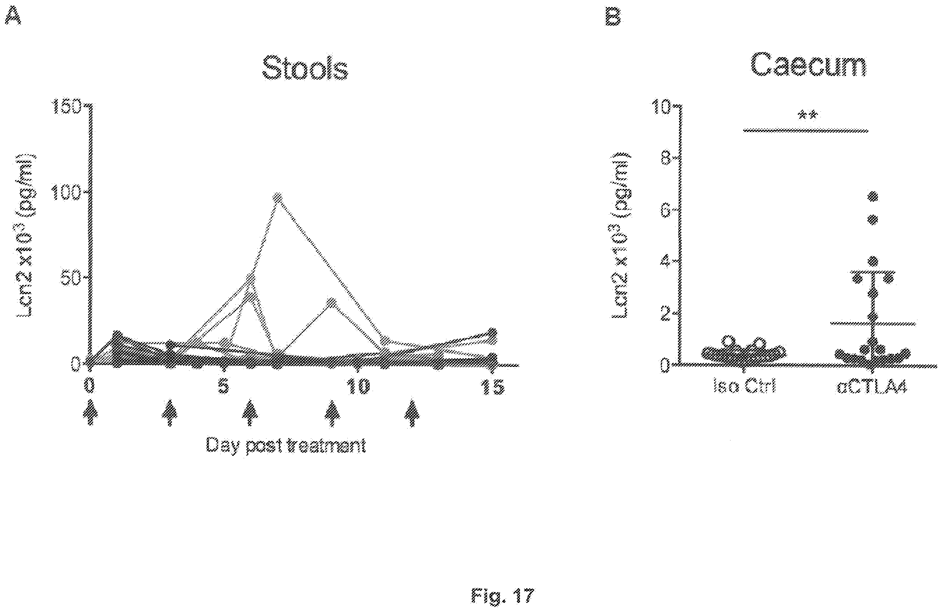

**Figure 17****. Augmented concentrations of the inflammatory mediator lipocalin-2 are observed in the caeca and stools of αCTLA4-treated mice.** MCA205 tumor-bearing mice were administered either αCTLA4 or the appropriate isotype control mAb, every 3 days, from when the implanted tumors reached 25mm² in size. Eight days after the beginning of mAb administration (i.e. after 3 mAb injections), the contents of caeca were collected for measurement of lipocalin-2 (Lcn2) by ELISA **(A).** In separate groups of mice, stools were collected throughout the time course of mAb treatment for subsequent Lcn2 ELISA **(B).** Arrows in **B** indicate the times of mAb administration. Data in **A** and **B** are pooled from 4 independent experiments (*n* = 5 per group) and depict the mean ± SEM. ***p* < 0.01 by unpaired two-tailed t-test.

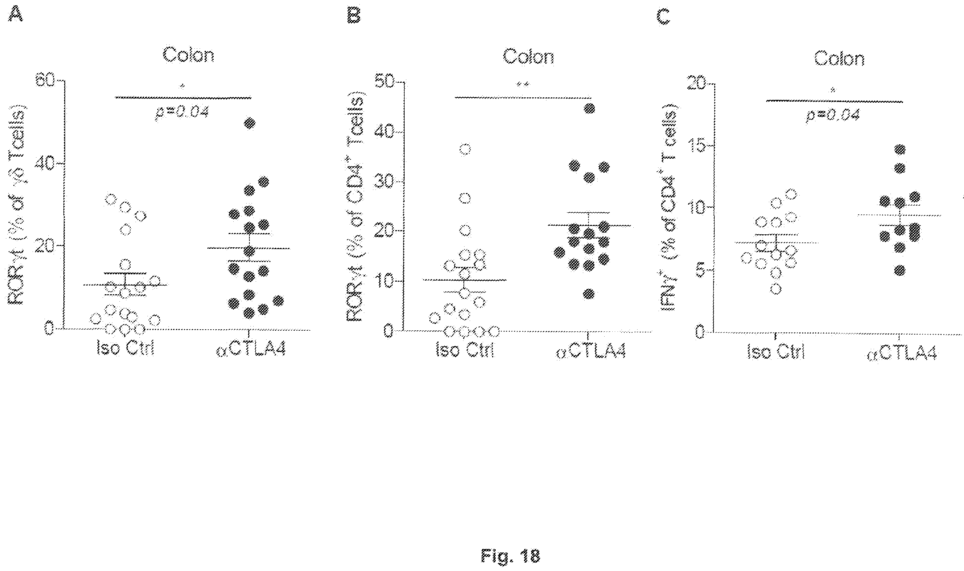

**Figure 18****. Dynamic changes of lymphocyte subsets in the colonic lamina propria following anti-CTLA4 mAb therapy.** C57BL/6 mice were administered either anti-CTLA4 or the appropriate isotype control mAb every 3 days as detailed before. After 3 mAb injections (day 8 post start of treatment), colonic lymphocytes were isolated and the percentages of RORγt⁺ γδ T cells **(A),** RORγt⁺ CD4⁺ T cells **(B)** and IFN-γ⁺ CD4⁺ T cells **(C)** were determined by flow cytometry. Data depict the mean ± SEM at least three independently performed experiments that have been pooled. ** *p <* 0.01, * *p <* 0.05, ns = not significant, by unpaired two-tailed t-test.

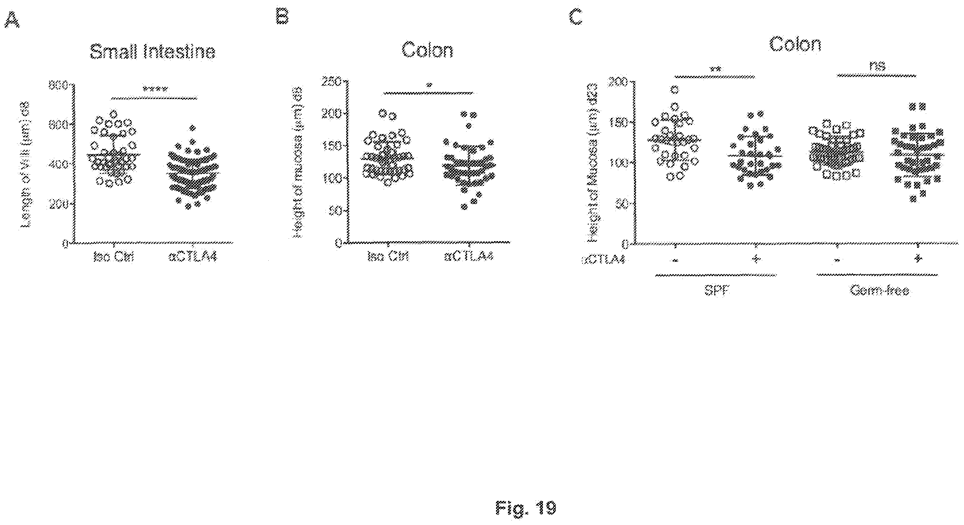

### Figure 19. Hallmarks of intestinal pathology observed in αCTLA4-treated mice are dependent upon intestinal microbiota.

Small intestines and colons were taken and processed for histopathological scoring (as detailed in the Materials & Methods) from MCA205 tumor-bearing specific pathogen free (SPF) mice 8 days after beginning αCTLA4 (or isotype control) mAb therapy as detailed before (i.e. after 3 mAb injections). The length of small intestine villi **(A)** and the height of the mucosa of colons **(B)** were measured from at least 8 distinct regions from each mouse. In an additional experiment, the colon mucosal height was measured in the same way in SPF and germ-free mice **(C),** 23 days following the beginning of mAb treatment. Data shown are from one of 2 independent experiments (n = 5 per group) and depict the mean ± SEM. **** *p <* 0.0001, * *p <* 0.05 by unpaired two-tailed *t*-test **(A** & **B);** ** *p* < 0.01, ns = not significant by one-way ANOVA with Bonferroni's multiple comparison test **(C).**

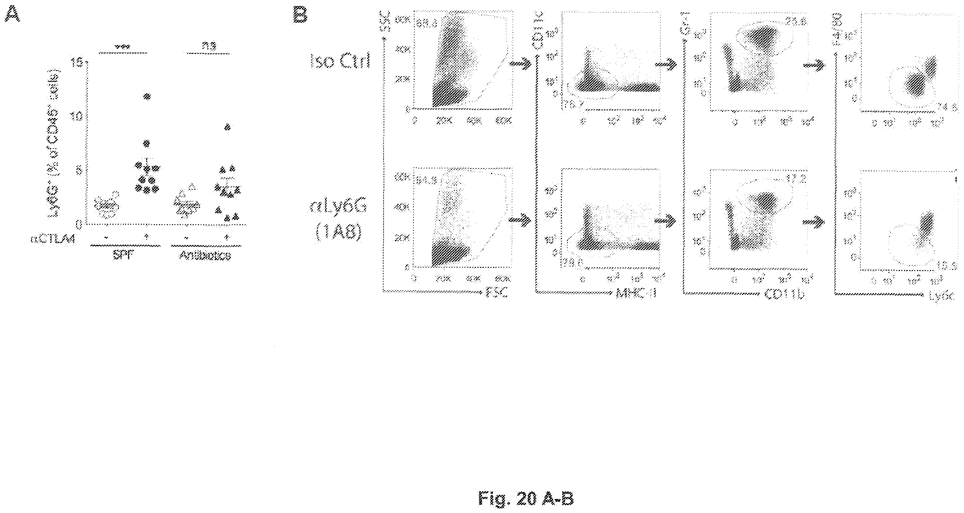

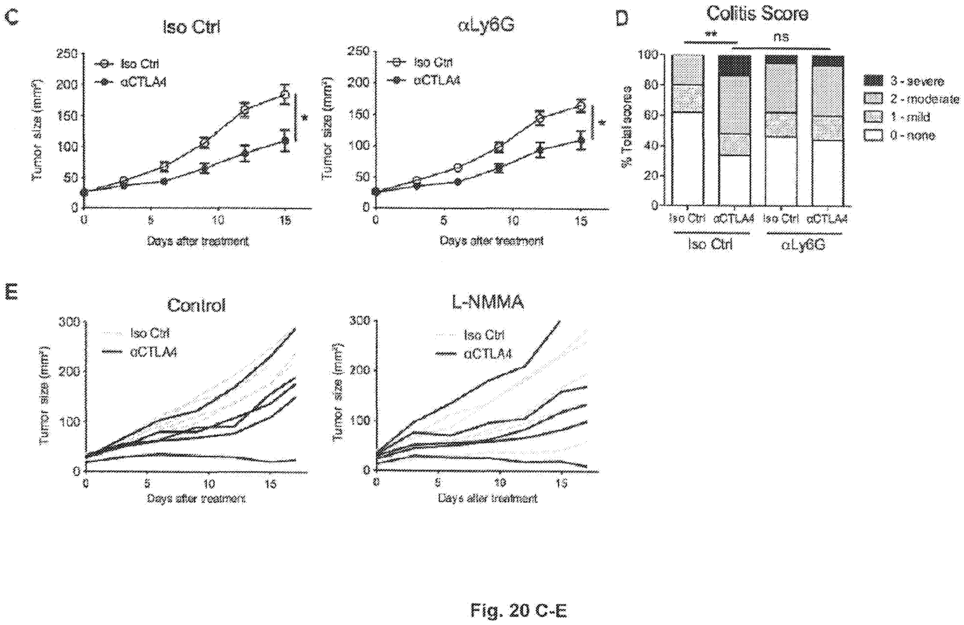

**Figure 20****. No role for neutrophils in efficacy or toxicity of anti-CTLA4 Ab.** A. *Microbiota-dependent neutrophil accumulation in tumors.* Tumoral neutrophils (Ly6G⁺CD45⁺) were determined by flow cytometry in SPF and ACS-treated MCA205-bearing C57BL/6 mice at 8 days post anti-CTLA4 (or isotype control) mAb therapy **(A).** Each dot represents one mouse tumor. **B-D.** *Neutrophil depletion does not prevent antitumor efficacy of CTLA4 blockade.* In an additional experiment, mice were administered anti-Ly6G 1A8 (or isotype control) mAb every other day on commencement of αCTLA4 (or isotype control) mAb therapy, and neutrophil depletion was confirmed for each mouse from blood sampling 7 days later (post- 4 anti-Ly6G injections) by flow cytometry as depicted by the representative dot plots shown in **B.** Tumor growth kinetics for control or neutrophil-depleted mice are shown in **C** (left and right panel) for a representative experiment out of two yielding similar results. **D.** *Neutrophil depletion does not prevent gut toxicity.* Colons harvested from these mice, 15 days post the start of mAb therapy were sectioned for colitis scoring as detailed in Materials & Methods. Neutralization of nitric oxide using the iNOS inhibitor L-NMMA was performed at the onset of CTLA4 blockade and did not alter the antitumor effects **(E).** Data shown depict the mean ± SEM (*n* = 5). *** *p* < 0.001, ns = not significant, by one-way ANOVA with Bonferroni's multiple comparison test in **A.** ** *p* < 0.01, ns = not significant, by Kruskal-Wallis non-parametric test with Dunn's multiple comparison test in **B**. * *p* < 0.05, ns = not significant by unpaired two-tailed *t*-test **(C).**

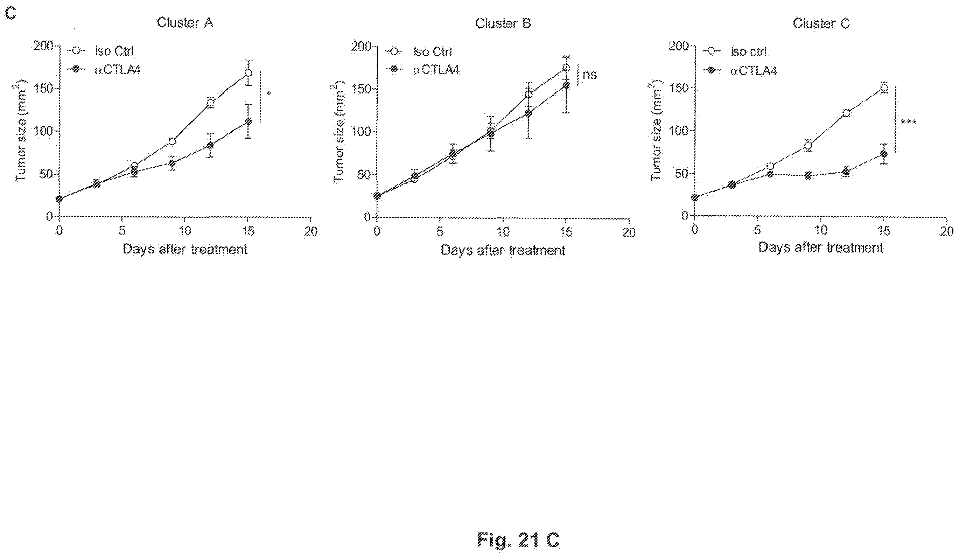

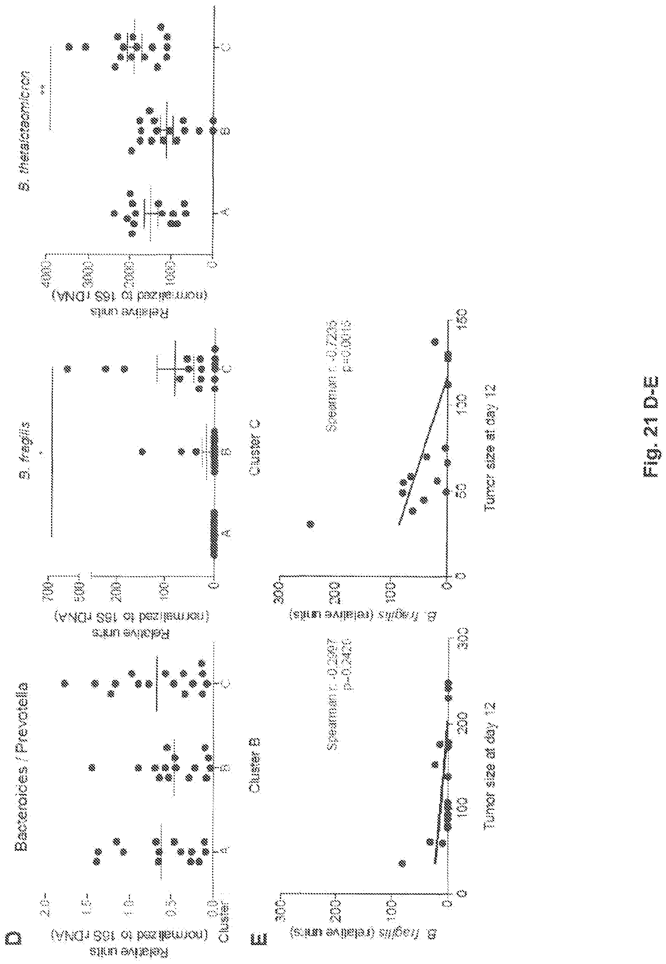

**Figure 21****. Biological significance of ipilimumab-induced dysbiosis in patients.** The k-means clustering algorithm was applied based on genus composition before and during ipilimumab (ipi) treatment in 25 MM patients, validated using the Calinski-Harabasz Index, and showed good performance in recovering three clusters before and after therapy (inter-class PCA; A, left panel, Monte-Carlo test, p=0.000199). Random Forest analysis was applied to decipher main genera responsible for this significant clustering (A, right panel). The relative abundance of main *Bacteroides* spp significantly differed between cluster B and C (B, right panel). The proportions of patients falling into each cluster were analyzed in a non-paired manner before versus after ipi injections regardless of the time point (B, left panel). C. FMT of feces post-ipi from 8 patients falling into each of the three clusters into GF animals. One representative experiment out of three is shown with means + SEM of tumor sizes depicted for each cluster over time.

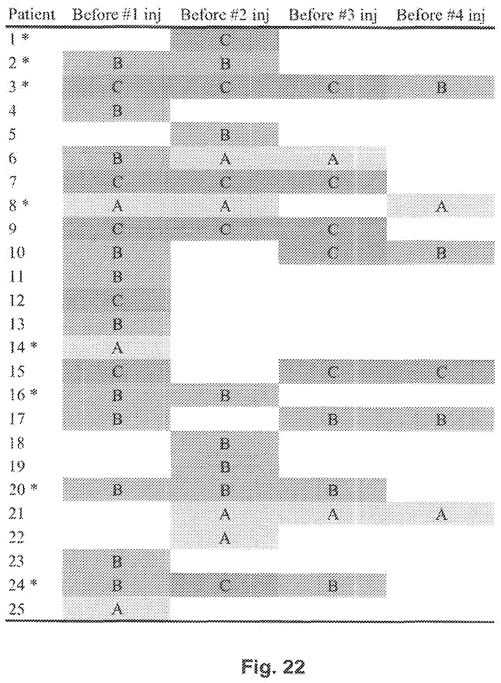

**Figure 22****. Clustering of patients before and during ipilimumab therapy.** Composition of the gut microbiome before and after ipilimumab treatment. A clustering algorithm based on genus composition was applied to cluster patients into one of three enterotypes as described in Fig. 21A-C. Follow up detailed chart of the clustering for each patient over time. * indicates the feces samples utilized for the FMT described in Fig. 21A-C.

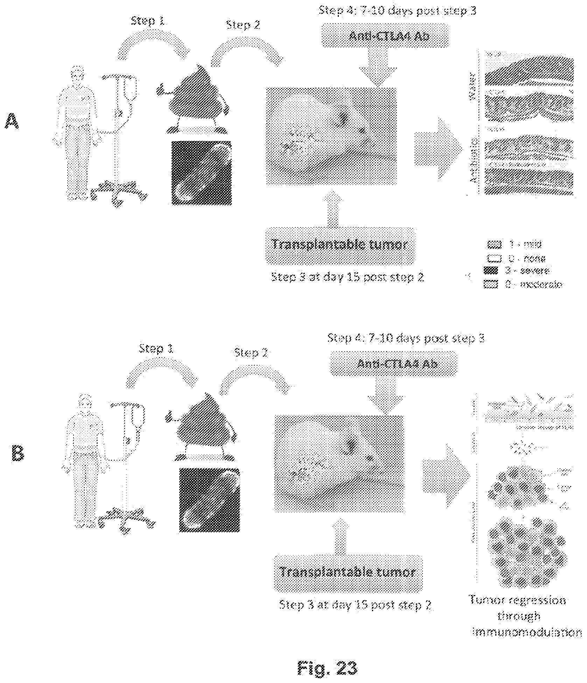

**Figure 23****. FMT experiments.** Fecal samples from a metastatic melanoma cohort were used for FMT experiments according to the method described in Example 2 below. Two weeks after microbiota transfer, MCA205-OVA tumor was injected subcutaneously and mice were treated with anti-CTLA4 mAb or isotype control as follows: mice were subcutaneously injected into the right flank with 1 × 10⁶ MCA205-OVA. When tumors reached a size of 20 to 40 mm² (day 0), mice were injected intraperitoneally (i.p) with 100µg of anti-CTLA-4 mAb (clone 9D9) or isotype control (clone MPC11) (right panel). Mice were injected 5 times at 3-day intervals with 9D9, and tumor size was routinely monitored by means of a caliper. The left panel describes the histopathological score of small and large intestines based on several parameters.

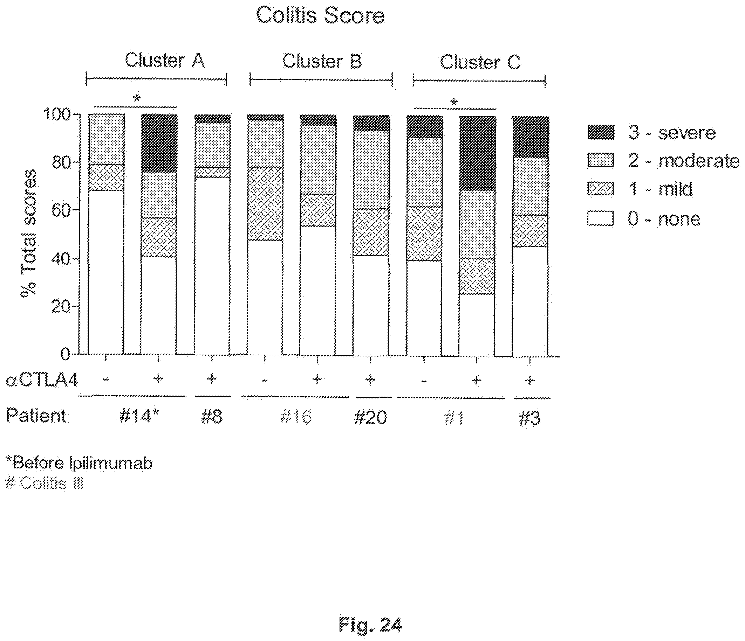

**Figure 24****. Histopathological score of colonic mucosae post FMT, established on H&E stained colons as described in M&M.** Overall epithelium irregularity scored from at least 6 sections per mouse (0=normal, 1=minor, 2=moderate, 3=severe), and all of these scores plotted. Anova statistical analyses, histopathological scoring by proportional odds logistic regression, *p<0.05, **p<0.01, ***p<0.001, ns: not significant.

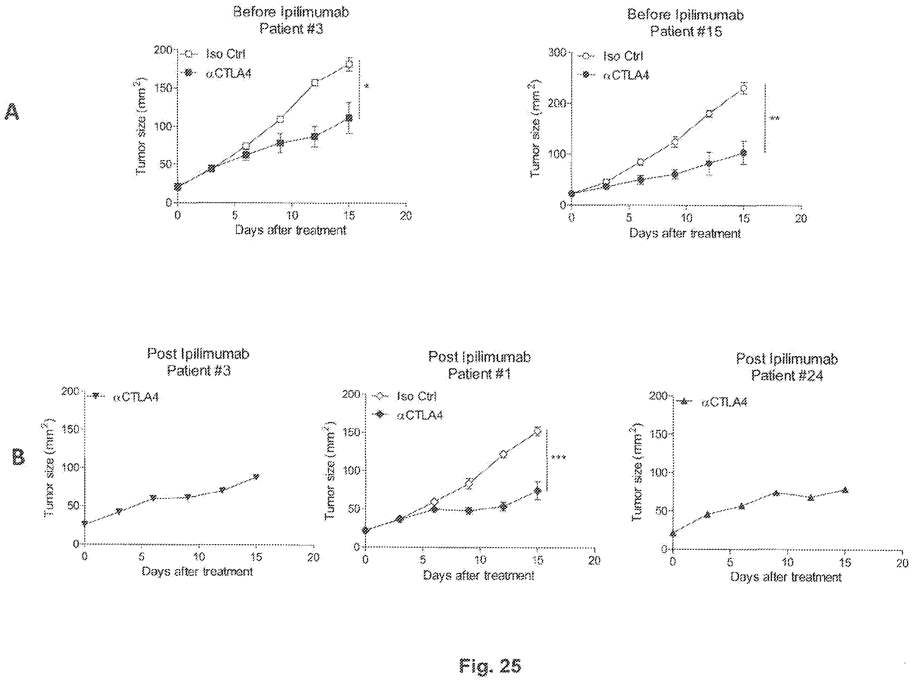

**Figure 25****. Cluster C feces predicts favorable clinical outcome in GF tumor bearers treated with anti-CTLA4 Ab.** FMT using feces thawed from either prior to therapy (A) or after 1 or 2 injections of 9D9 Ab (anti-CTLA4) was performed from one individual (metastatic melanoma) into 5 GF mice (according to the methodology described in Fig. 23) Tumor growth kinetics is depicted. *p<0.05.

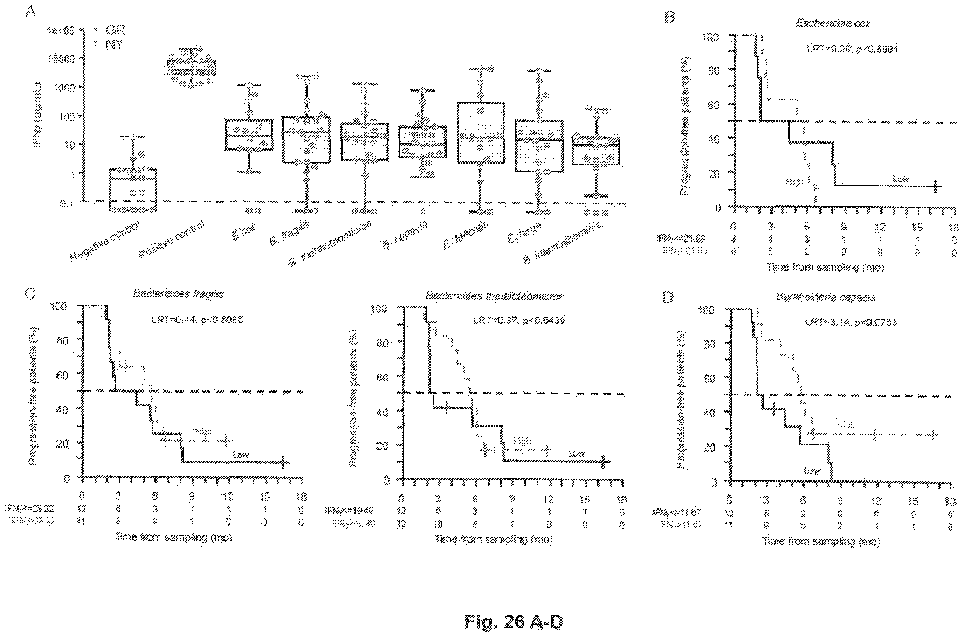

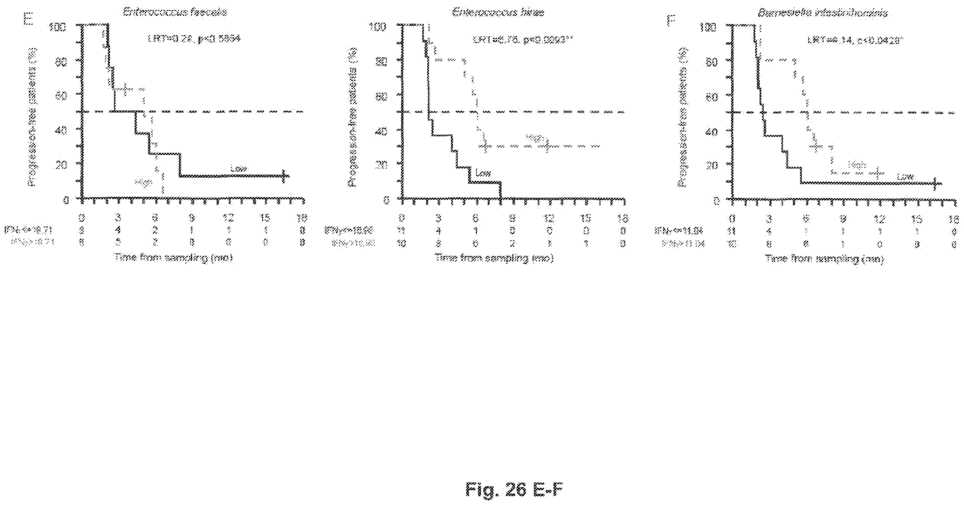

**Figure 26****. Memory Th1 immune responses to *B. intestinihominis* associated with prolonged PFS in end stage NSCLC patients previously treated with platinum-based chemotherapy and enrolled in CTX-based exosomal vaccines or immune checkpoint trial.** After completion of platinum-based chemotherapy, PBMCs from 26 NSCLC patients were collected at baseline before starting either Dex/CTX or Ipi/Radiotherapy trial and were segregated into two subgroups based on the median IFNγ production released by CD4⁺CD45RO⁺ T cells incubated with autologous monocytes exposed to distinct bacterial spp, *i.e* for *E.coli* (A) for *Bacteroides* spp, (B) for *Burkholderia cepacia,* (C) for *enterococci* species (D) and for *Barnesiella intestinihominis*) (E). The association between the ratio between Th1 cytokines and IL-10 and PFS is shown for *B.cepacia* (C, right panel) and *B*.*intestinihominis* (E, right panel) but is not significant for *E.hirae* (not shown). The PFS for each subgroup of patients above/below this mean is represented for each bacterial spp. Univariate analyses and Kaplan Meier curves are shown. *p<0.05.

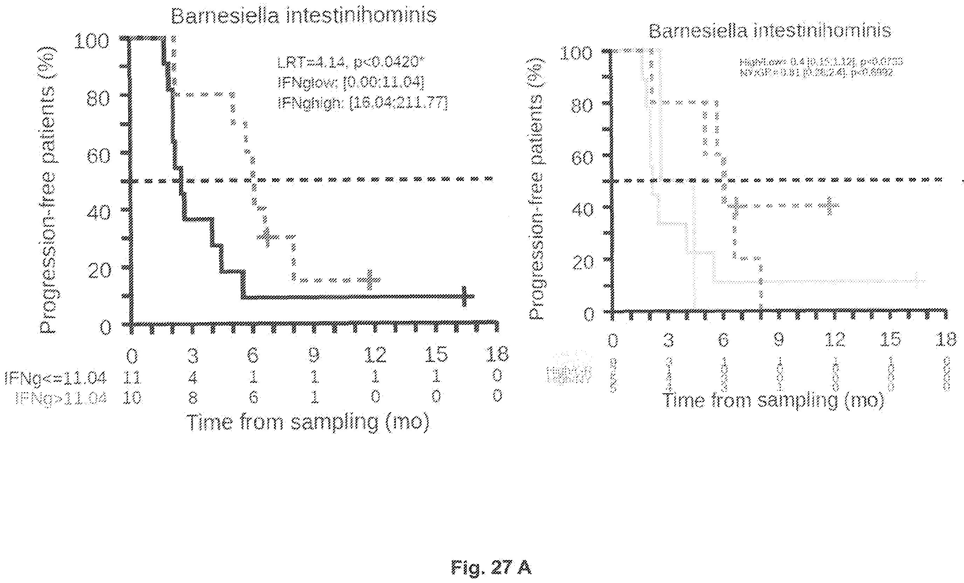

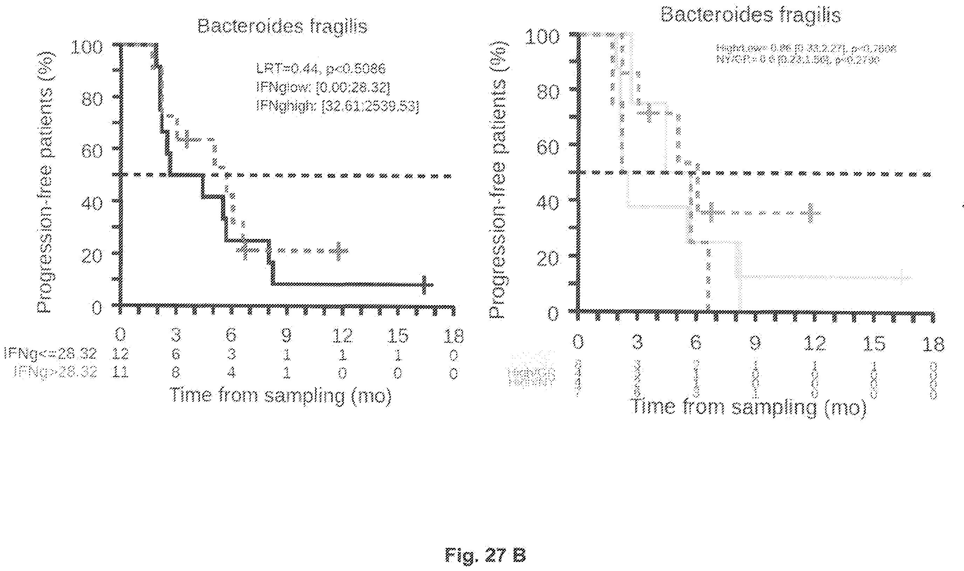

**Figure 27****. Idem as** **Fig. 26** **but splitting the two cohorts and performing a separate statistical analysis.** The GR (Gustave Roussy, CTX+Dex treated NSCLC) and the NY (New York, Ipilimumab+radiotherapy) cohorts were segregating and indicating similar conclusions for *Barnesiella intestinihominis* in NSCLC. In contrast to melanoma patients who responded to *B. fragilis,* NSCLC patients failed to respond to *B. fragilis* post-ipilimumab. They rather responded to *Barnesiella intestinihominis* and to a lesser extent to *Burkholderia cepacia.*

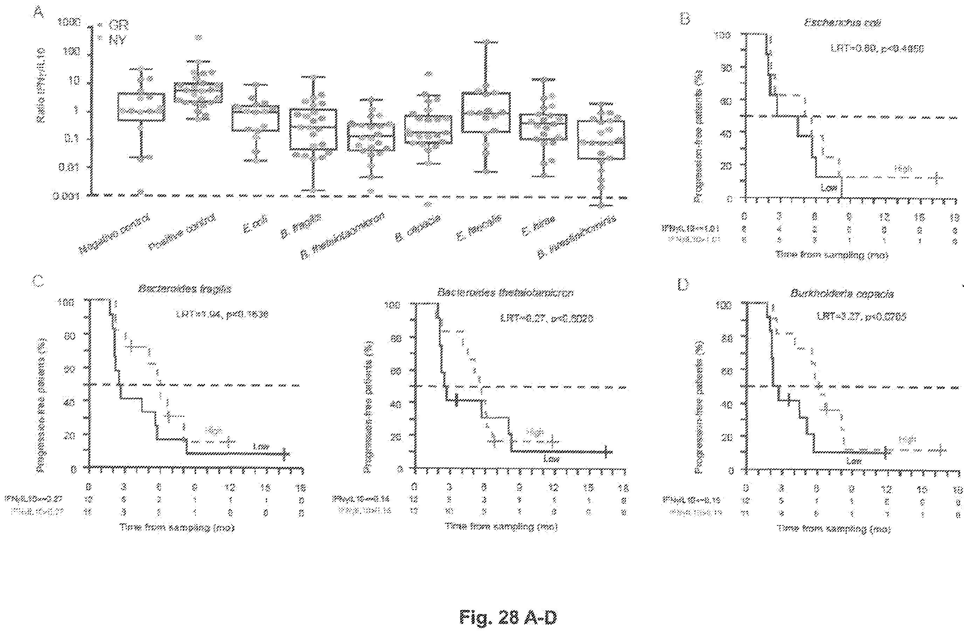

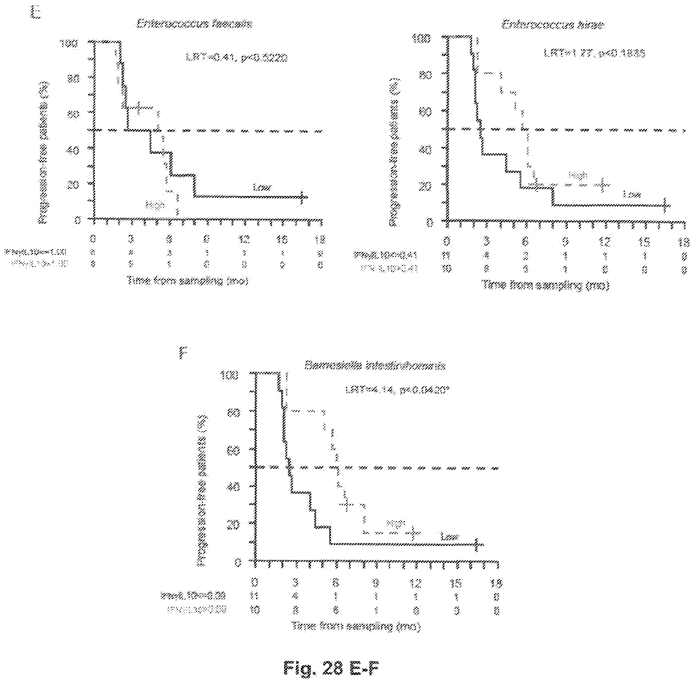

**Figure 28****. IPNγ and IL-10 release from memory circulating CD4+ T cells after *ex vivo* restimulation with distinct bacterial species in end stage NSCLC patients after chemotherapy.** Determination by ELISA of cytokine release IFNγ, IL-10 and the IFNγ/IL-10 ratio after 48 hrs of restimulation of CD4⁺CD45RO⁺ T cells with monocytes loaded with bacteria or PBS. The positive control was the stimulation with anti-CD3/CD28 coated beads. The cytokine levels in the supernatants of monocytes loaded with bacteria in the absence of T cells are shown. Each dot represents one patient at the time preceeding CTX/Dex therapy, after stabilisation pots-chemotherapy. Survival curves were estimated by the Kaplan-Meier product-limit method and survival distribution compared by Cox regression.

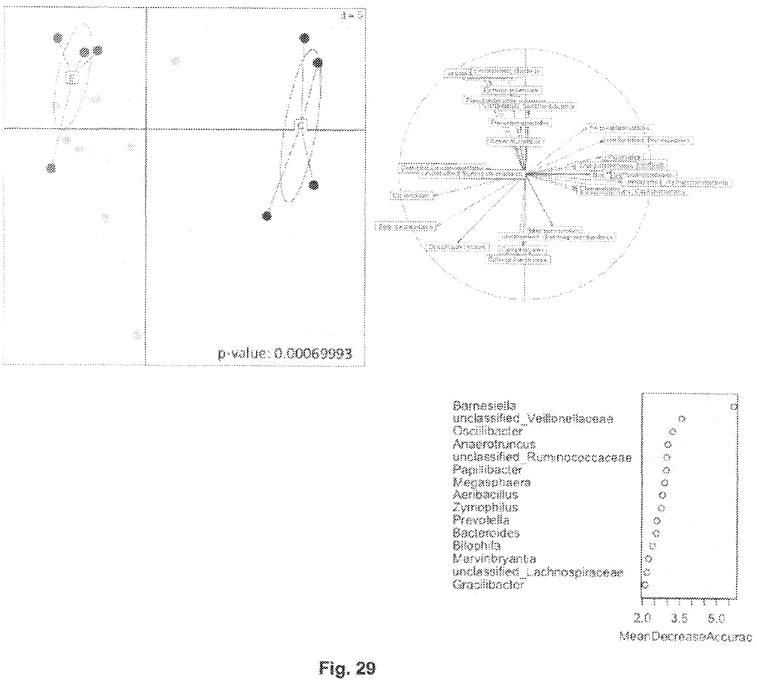

**Figure 29****. Enterotyping of tumor bearing C57BL/6 mice treated with 9D9 Ab.** The k-means clustering algorithm was applied based on genus composition before and during 9D9 anti CTLA4 Ab treatment in 6 mice /group, validated using the Calinski-Harabasz Index, and showed good performance in recovering three clusters after therapy. The relative abundance of main *Barnesiella intestinihominis* significantly differed between cluster B and C.

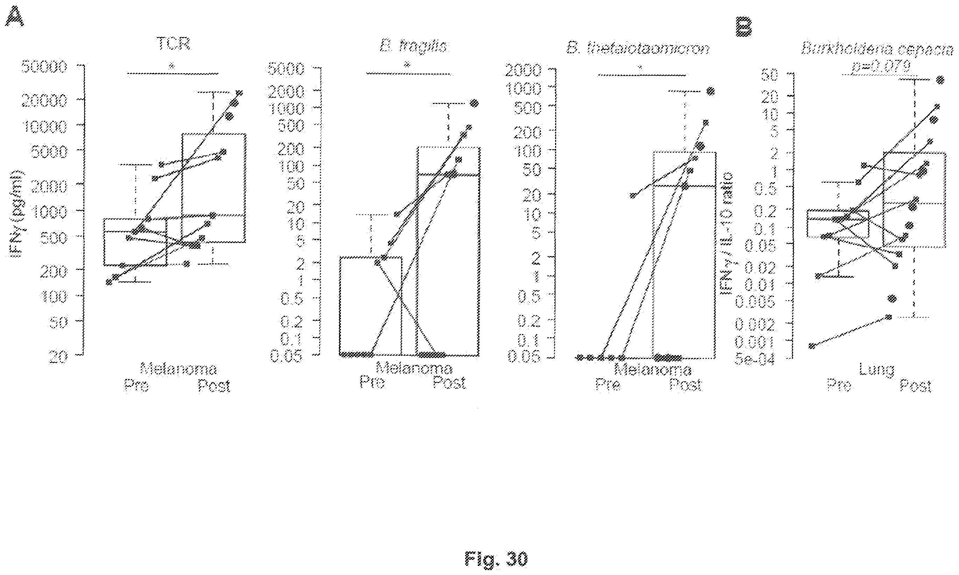

**Figure 30****. Th1 memory T cell responses against *Bacteroides* spp (*B. Fragilis* and *B. thetaiotaomicron*) are induced by CTLA4 blockade.** A. Selective restoration of T cell unresponsiveness to Bf and Bt in melanoma and to Burkholderia in NSCLC treated with ipilimumab. CD4⁺CD45RO⁺ T cells from PBMC of advanced melanoma patients before and after at least 2 injections of ipilimumab were restimulated with autologous CD14⁺ cells loaded with various bacterial strains (then, neutralized with antibiotics before coculture with T cells) in duplicate wells. IFNγ (A) and IFNγ/IL-10 ratios (B) were monitored in NSCLC patients the supernatants by luminex Magpix technology at 48 hrs. No cytokine release was observed in the absence of bacteria or with loaded monocytes in the absence of T cells. Each dot represents IPNγ secretion (A) or ratios (B) for one individual patient. Paired analyses are represented by linking dots pre-and post-ipilimumab. Student t-test statistical analyses: *p<0.05, **p<0.01, ns: not significant.

### DETAILED DESCRIPTION OF THE PREFERRED EMBODIMENTS

In the present text, the following general definitions are used:

### Gut microbiota

The "gut microbiota" (formerly called gut flora or microflora) designates the population of microorganisms living in the intestine of any organism belonging to the animal kingdom (human, animal, insect, *etc.*)*.* While each individual has a unique microbiota composition (60 to 80 bacterial species are shared by more than 50% of a sampled population on a total of 400-500 different bacterial species/individual), it always fulfils similar main physiological functions and has a direct impact on the individual's health:
- it contributes to the digestion of certain foods that the stomach and small intestine are not able to digest (mainly non-digestible fibers);
- it contributes to the production of some vitamins (B and K);
- it protects against aggressions from other microorganisms, maintaining the integrity of the intestinal mucosa;
- it plays an important role in the development of a proper immune system;
- a healthy, diverse and balanced gut microbiota is key to ensuring proper intestinal functioning.

Taking into account the major role gut microbiota plays in the normal functioning of the body and the different functions it accomplishes, it is nowadays considered as an "organ". However, it is an "acquired" organ, as babies are born sterile; that is, intestine colonisation starts right after birth and evolves afterwards.

The development of gut microbiota starts at birth. Sterile inside the uterus, the newborn's digestive tract is quickly colonized by microorganisms from the mother (vaginal, skin, breast, *etc.*)*,* the environment in which the delivery takes place, the air, *etc.* From the third day, the composition of the intestinal microbiota is directly dependent on how the infant is fed: breastfed babies' gut microbiota, for example, is mainly dominated by *Bifidobacteria,* compared to babies nourished with infant formulas.

The composition of the gut microbiota evolves throughout the entire life, from birth to old age, and is the result of different environmental influences. Gut microbiota's balance can be affected during the ageing process and, consequently, the elderly have substantially different microbiota than younger adults.

While the general composition of the dominant intestinal microbiota is similar in most healthy people (4 main phyla, *i.e., Firmicutes, Bacteroidetes, Actinobacteria* and *Proteobacteria*)*,* composition at a species level is highly personalised and largely determined by the individuals' genetic, environment and diet. The composition of gut microbiota may become accustomed to dietary components, either temporarily or permanently. Japanese people, for example, can digest seaweeds (part of their daily diet) thanks to specific enzymes that their microbiota has acquired from marine bacteria.

### Dysbiosis

Although it can adapt to change and has a high resilience capacity, a loss of balance in gut microbiota composition may arise in some specific situations. This is called "dysbiosis", a disequilibrium between potentially "detrimental" and known "beneficial" bacteria in the gut or any deviation to what is considered a "healthy" microbiota in terms of main bacterial groups composition and diversity. Dysbiosis may be linked to health problems such as functional bowel disorders, inflammatory bowel diseases, allergies, obesity and diabetes. It can also be the consequence of a treatment, such as a cytotoxic treatment or an antibiotic treatment.

A specific dysbiosis can be highlighted depending on the pathogenic condition. For instance, patients with Crohn's disease, a chronic inflammatory bowel disease, present a microbiota with reduced percentages and diversity of bacteria belonging to the Firmicutes phylum, and mostly from the *Clostridium leptum* (cluster IV) group (Manichanh et al., 2006; Sokol et al., 2006).Generally, decreased percentages of bacteria from the Lachnospiraceae family can be observed. Moreover mucosa-associated microbiota of these patients is depleted in bacteria from the *Bifidobacterium* and *Lactobacillus* genera toward increased levels of potentially pathogenic bacteria such as specific strains of *Escherichia coli* with adherent and invasive phenotypes (AIEC) (Darfeuille-Michaud et al., 2004; Joossens et al.).

To the contrary, patients with obesity and metabolic disorders have higher proportions of bacteria belonging to the Firmicutes phylum and lower levels of *Escherichia coli* in their feces (Ley et al., 2005; Tumbaugh et al., 2009). An increased in proportions of *E. coli* in these patients has been associated with weight loss following bariatric surgery and lower levels of serum leptin (Furet et al.).

In patients with colorectal cancer (CRC), however, gut microbial dysbiosis relates to enrichment in bacterial species from the *Bacteroides* genus and decrease of *Faecalibacterium* and *Roseburia* genera belonging species (Sobhani et al., ; Wu et al.). Specifically, *Fusobacterium* and *Campylobacter genera* were found to be consistently increased in both feces and mucosa of CRC patients.

In the context of cancer, "beneficial or "favorable" bacteria are essentially *Lactobacillus* and *Bifidobacterium,* and "detrimental" or "unfavorable" bacteria are essentially the species *Parabacteroides distasonis* and *Faecalibacterium prausnitzii,* the genera *Gemmiger, Alistipes* and *Clostridium Cluster IV. (Clostridium leptum* group).

### Antineoplastic treatments

"Antineoplastic treatments" herein designate any treatment for cancer except surgery. They include chemotherapy, hormonal and biological therapies, and radiotherapy.

### Biological therapies

Anti cancer "biological therapies" involve the use of living organisms, substances derived from living organisms, or laboratory-produced versions of such substances to treat cancer, by targeting either the cancer cells directly, or by stimulating the body's immune system to act against cancer cells ("immunotherapy"). Biological therapies include monoclonal antibodies (including those targeting cancer cell surface, e.g. rituximab and alemtuzumab; anti-CTLA4 Mabs, such as ipilimumab; targeting growth factors, *e.g*.: bevacizumab, cetuximab, panitumumab and trastuzumab; anti-PD-1 Mabs; anti-Tim3 Mabs; anti-ICOS Mabs), immunoconjugates (*e*.*g*.: ⁹⁰Y-ibritumomab tiuxetan, ¹³¹I-tositumomab, and ado-trastuzumab emtansine), cytokines (including interferons such as IFNα; interleukins such as IL-2, IL-11, G-CSM, GM-CSF), therapeutic vaccines (*e*.*g*.: Sipuleucel-T (Provenge^{®})), the bacterium bacillus Calmette-Guérin, cancer-killing viruses, gene therapy, and adoptive T-cell transfer.

### Probiotics

"Probiotics" are micro-organisms that have claimed health benefits when consumed. Probiotics are commonly consumed as part of fermented foods with specially added active live cultures, such as in yogurt, soy yogurt, or as dietary supplements. Generally, probiotics help gut microbiota keep (or re-find) its balance, integrity and diversity. The effects of probiotics are usually strain-dependent.

### Immune checkpoint blockers

In the present text, a "drug blocking an immune checkpoint", or "immune checkpoint blocker" or "immune checkpoint blockade drug" designates any drug, molecule or composition which blocks an immune checkpoint. In particular, it encompasses anti-CTLA-4 antibodies, anti-PD-1 antibodies and anti-PD-L1 antibodies. More particularly, it can be an anti-CTLA-4 monoclonal antibody, especially an anti-CTLA-4 monoclonal IgG1 such as Ipilimumab or an anti-CTLA-4 monoclonal IgG2a such as Tremelimumab.

### Cancer, treatment, etc.

As used herein, "cancer" means all types of cancers. In particular, the cancers can be solid or non solid cancers. Non limitative examples of cancers are carcinomas or adenocarcinomas such as breast, prostate, ovary, lung, pancreas or colon cancer, sarcomas, lymphomas, melanomas, leukemias, germ cell cancers and blastomas.

The immune system plays a dual role against cancer: it prevents tumor cell outgrowth and also sculpts the immunogenicity of the tumor cells. Drugs blocking an immune checkpoint can hence be used to treat virtually any type of cancer. Thus, the methods according to the invention are potentially useful for patients having a cancer selected amongst adrenal cortical cancer, anal cancer, bile duct cancer (e.g. periphilar cancer, distal bile duct cancer, intrahepatic bile duct cancer), bladder cancer, bone cancers (e.g. osteoblastoma, osteochrondroma, hemangioma, chondromyxoid fibroma, osteosarcoma, chondrosarcoma, fibrosarcoma, malignant fibrous histiocytoma, giant cell tumor of the bone, chordoma, lymphoma, multiple myeloma), brain and central nervous system cancers (e.g. meningioma, astocytoma, oligodendrogliomas, ependymoma, gliomas, medulloblastoma, ganglioglioma, Schwannoma, germinoma, craniopharyngioma), breast cancer (e.g. ductal carcinoma *in situ,* infiltrating ductal carcinoma, infiltrating lobular carcinoma, lobular carcinoma *in situ,* gynecomastia), Castleman disease (e.g. giant lymph node hyperplasia, angiofollicular lymph node hyperplasia), cervical cancer, colorectal cancer, endometrial cancers (e.g. endometrial adenocarcinoma, adenocanthoma, papillary serous adnocarcinoma, clear cell), esophagus cancer, gallbladder cancer (mucinous adenocarcinoma, small cell carcinoma), gastrointestinal carcinoid tumors (e.g. choriocarcinoma, chorioadenoma destruens), Hodgkin's disease, non-Hodgkin's lymphoma, Kaposi's sarcoma, kidney cancer (e.g. renal cell cancer), laryngeal and hypopharyngeal cancer, liver cancers (e.g. hemangioma, hepatic-adenoma, focal nodular hyperplasia, hepatocellular carcinoma), lung cancers (e.g. small cell lung cancer, non-small cell lung cancer), mesothelioma, plasmacytoma, nasal cavity and paranasal sinus cancer (e.g. esthesioneuroblastoma, midline granuloma), nasopharyngeal cancer, neuroblastoma, oral cavity and oropharyngeal cancer, ovarian cancer, pancreatic cancer, penile cancer, pituitary cancer, prostate cancer, retinoblastoma, rhabdomyosarcoma (e.g. embryonal rhabdomyosarcoma, alveolar rhabdomyosarcoma, pleomorphic rhabdomyosarcoma), salivary gland cancer, skin cancer (e.g. melanoma, nonmelanoma skin cancer), stomach cancer, testicular cancers (e.g. seminoma, nonseminoma germ cell cancer), thymus cancer, thyroid cancers (e.g. follicular carcinoma, anaplastic carcinoma, poorly differentiated carcinoma, medullary thyroid carcinoma, thyroid lymphoma), vaginal cancer, vulvar cancer, and uterine cancer (e.g. uterine leiomyosarcoma). More particularly, the method according to the disclosure can be used for predicting and optimizing a patient's response to a medicament targeting an immune checkpoint, wherein the patient has a cancer selected from the group consisting of metastatic melanoma, non-small cells lung carcinoma (NSCLC), small cell lung cancer (SCLC), prostate cancer and prostatic neoplasms (especially metastatic hormone-refractory prostate cancer), sarcoma, Wilm's tumor, lymphoma, neuroblastoma, and bladder cancer.

As used herein, the terms "treat", "treatment" and "treating" refer to any reduction or amelioration of the progression, severity, and/or duration of cancer, particularly a solid tumor; for example in a breast cancer, reduction of one or more symptoms thereof that results from the administration of one or more therapies.

Other definitions will be specified below, when necessary.

A first aspect of the present disclosure is the use of a combination of an immune checkpoint blocker and of an antibiotic selected from the group consisting of vancomycin, penicillin G (benzylpenicillin) and any other antibiotic killing Gram positive bacteria and sparing *Bacteroidales* and/or favoring the outgrowth of Gram negative bacteria, especially any antibiotic to which *B.fragilis* strains are resistant, for treating a cancer. In the frame of the present invention, the immune checkpoint blocker is an antibody blocking CTLA4.

According to a particular embodiment, the antibiotic is vancomycin.

According to another particular embodiment, the immune checkpoint blocker is an anti-CTLA-4 antibody. In particular, an anti-CTLA-4 monoclonal antibody can advantageously be used, such as, for example, an anti-CTLA-4 monoclonal IgG1 (e.g., Ipilimumab) or an anti-CTLA-4 monoclonal IgG2a (e.g., Tremelimumab).

Of course, since the immune system plays an important part against all the cancers, the present invention can benefit to patients who suffer from virtually any cancer. According to a particular aspect, the patient suffers from metastatic melanoma or from advanced non-small cell lung cancer (NSCLC).

As used herein, the term "combination" refers to the use of more than one agent (e.g., vancomycin and Ipilimumab, possibly associated to radiotherapy). The use of the term "combination" does not restrict the order in which therapies are administered to the patient, although it is preferable to administer the antibiotic prior to or simultaneously with the immune checkpoint blocker. For example, vancomycin can be administered prior to Ipilimumab (e. g., 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), either punctually or several times (for example, each day) before the antineoplastic treatment is administered. Advantageously, the antibiotic is administered before administration of the drug blocking an immune checkpoint, in order to modulate the patient's gut microbiota to optimize the effect of the immune checkpoint blocker (such as those defined above).

The present invention also pertains to the use of an antibiotic to which *B. fragilis* strains are resistant, such as, but not limited to, vancomycin and penicillin G, as an adjuvant therapy to potentiate the anticancer effects of a drug blocking CTLA4. Indeed, as illustrated in the experimental part below, it can be useful to modulate the gut microbiota of a patient, through the use of antibiotics and/or probiotics, for increasing the anticancer effects of an immune checkpoint blocker. In particular, an antibiotic can be used for increasing the relative amount of Gram negative bacteria, especially the relative amount of *Bacterioides* such as *B. fragilis, B. thetaiotaomicron, B. vulgatus, B. uniformis* and *B. massiliensis,* as well as the relative amount of other bacteria such as *Burkholderia cepacia, Burkholderia cenocepacia* and *Barnesiella intestinihominis* in the gut microbiota of a patient before administering an immune checkpoint blocker to said patient (or at the same time). Vancomycin is particularly useful to that aim.

In what precedes, the "relative amount", which can also be designated as the "relative abundance", is defined as the number of bacteria of a particular taxonomic level (from phylum to species) as a percentage of the total number of bacteria in a biological sample. This relative abundance can be assessed, for example, by measuring the percentage of 16S rRNA gene sequences present in the sample which are assigned to these bacteria. It can be measured by any appropriate technique known by the skilled artisan, such as 454 pyrosequencing and quantitative PCR of these specific bacterial 16S rRNA gene markers or quantitative PCR of any gene specific for a bacterial group.

The present invention also relates to the use of a probiotic bacterial composition comprising bacteria selected amongst *Bacteroides fragilis, Bacteroides thetaiotaomicron,* and *Burkholderia cenocepacia,* for treating a cancer in combination with CTLA4 blockade. According to a particular embodiment of the invention, said composition further comprises bacteria selected amongst *Bacteroides vulgatus, Burkholderia cenocepacia, Bacteroides uniformis, Bacteroides massiliensis and Barnesiella intestinihominis.* By "selected amongst" is meant that bacteria from one or several of the recited species can be comprised in said composition. Of course, other agents, such as bacteria of additional species, can also be included in the probiotic compositions according to the present invention. According to a preferred embodiment, the probiotic composition comprises bacteria from at least two or at least three or four different species selected from the species of any of the lists recited above.

The probiotic compositions according to the present invention are used in combination with an immune checkpoint blocker as defined above, for inducing immunostimulation in a cancer patient. A probiotic bacterial composition comprising bacteria selected from the group consisting of *Bacteroides fragilis, Bacteroides thetaiotaomicron, Burkholderia cenocepacia* and mixtures thereof, and optionally bacteria selected from the group consisting of *Bacteroides salyersiae, Bacteroides acidifaciens, Bacteroides intestinalis, Bacteroides vulgatus, Burkholderia cepacia, Burkholderia cenocepacia, Bacteroides uniformis, Bacteroides massiliensis* and *Barnesiella intestinihominis,* can be used prior to administering an anti-CTLA4 blocking antibody to said patient. According to a particular embodiment of this method, the bacterial composition comprises bacteria selected from the group consisting of *Bacteroides fragilis, Bacteroides thetaiotaomicron, Bacteroides vulgatus, Burkholderia cepacia, Burkholderia cenocepacia, Bacteroides uniformis, Bacteroides massiliensis and Barnesiella intestinihominis* and mixtures thereof. The probiotic compositions can be optimized depending on the patient's condition. For example, a probiotic composition for treating a patient with a lung cancer will preferably comprise *Barnesiella intestinihominis,* and a probiotic composition for treating a patient with a melanoma will preferably comprise *Bacteroides* spp., such as *Bacteroides fragilis.*

The probiotic bacterial composition can be formulated for oral administration. The skilled artisan knows a variety of formulas which can encompass living or killed microorganisms and which can present as food supplements (e.g., pills, tablets and the like) or as functional food such as drinks, fermented yoghurts, *etc.*

This aspect of the present invention is particularly useful for a cancer patient who has a dysbiosis with an under-representation of Gram-negative bacteria and/or who has previously received a broad-spectrum antibiotic treatment.

Another aspect of the present disclosure is a method for *in vitro* determining whether a cancer patient can benefit from an antineoplastic treatment comprising a drug blocking an immune checkpoint. This method is not covered by the claims. It comprises the following steps:
(i) from an appropriate biological sample from said patient, determining the relative abundance of *Bacteroides fragilis, Bacteroides thetaiotaomicron, Bacteroides vulgatus, Burkholderia cepacia, Burkholderia cenocepacia, Bacteroides uniformis, Bacteroides massiliensis and Barnesiella intestinihominis* in said patient's gut microbiota;
(ii) determining the presence or absence of an intestinal dysbiosis;
wherein an intestinal dysbiosis with an under-representation of bacteria recited in step (i) indicates that the patient will not be a good responder to the antineoplastic treatment.

In the present text, a "good responder to a treatment", also called a "responder" or "responsive" patient or in other words a patient who "benefits from" this treatment, refers to a patient who is affected with a cancer and who shows or will show a clinically significant relief in the cancer after receiving this treatment. Conversely, a "bad responder" or "non responder" is one who does not or will not show a clinically significant relief in the cancer after receiving this treatment. The disease clinical data may be assessed according to the standards recognized in the art, such as immune-related response criteria (irRC), WHO or RECIST criteria.

The biological sample can be a biofilm of a biopsy (preferably of a large biopsy) of caecum or colon mucosae obtained from the patient. For example, this biopsy can have been obtained during a specific surgery in pancreatic, stomach, biliary tract or colon cancers.

For any type of cancer, the biological sample can be a sample of feces obtained from the patient. This sample can have been collected at diagnosis, for example, or at any moment before deciding the beginning of the treatment.

As illustrated in the examples below, the inventors identified several enterotypes in the cancer patients they studied. Indeed, using a clustering algorithm based on genus composition, they first identified two and three clusters before and after the first cycle of ipilimumab respectively. *Bacteroides, Enterorhabdus* and *Alloprevotella* were the main determinants of the clustering in ipilimumab-naïve patients, with *Alloprevotella* driving cluster A and *Bacteroides species* driving cluster B (mostly *B. thetaiotaomicron, B. uniformis* and *B. massiliensis*)*.* Post-ipilimumab, a third cluster (cluster C, comprising *B. vulgatus* and *B. thetaiotaomicron*) was observed. Pursuing their experiments, the inventors then observed that some patients could qualify as cluster C patients even before any treatment with an ICB, which shows that increasing the number of enterotyped patients can lead to a better definition of said enterotypes. It appears that cluster C is the most favorable cluster, and patients who fall into this cluster, either initially (before treatment with an ICB such as ipilimumab) or after one or two injection(s) of an ICB are most likely to respond to said treatment.

Cluster C patients can be identified through several ways. The first one is through analysis of a nucleic acid extracted from a feces sample, for example by 16S rRNA pyrosequencing. The main bacteria which are over-represented and under-represented in this cluster are indicated in Example 3 below. In particular, *Bacteroides* spp such as *Bacteroides salyersiae, Bacteroides acidifaciens, Bacteroides fragilis, Bacteroides thetaiotaomicron, Bacteroides uniformis, and Bacteroides intestinalis* are over-represented in cluster C, while butyrate-producing bacteria are under-represented. Of course, as already mentioned, the skilled artisan can refine this profile. A second method for determining if a patient falls into cluster C is by FMT into a germ-free animal. Stools capable of allowing the niching/colonization of *B. thetaiotaomicron* and/or *B. fragilis* by 14 days upon transfer in germ free animals will be considered as belonging to cluster C. A third way of defining cluster C is by FMT into a tumor bearing-germ free animal then treated with an anti-CTLA4. Stools capable of allowing the expansion or overrepresentation of *B. fragilis* by 14 days upon transfer into germ free tumor bearing-animals after a therapy with 9D9 anti-CTLA4 Ab. An aspect of the present disclosure is a method for determining whether an individual is likely to respond to a treatment by an ICB, especially an anti-CTLA4 treatment, comprising determining if said individual falls into cluster C by any of the above-described protocols. This method is not covered by the claims.

The present disclosure also pertains to a method for predicting the response of an individual to any treatment with an immune checkpoint blocker, from a feces sample from said individual obtained before the beginning of said treatment, comprising (i) performing a fecal microbial transplantation (FMT) of said feces into germ free (GF) model animals such as GF mice; (ii) at least 7 to 14 days after step (i), inoculating said mice with a transplantable tumor model; (iii) treating the inoculated mice with an immune checkpoint blocker (equivalent of the ICB for the animal model); and (iv) measuring the tumor size and/or the colitis in the treated animals, wherein the results of step (iv), regarding colitis and/or anti-tumor effect of the treatment, are illustrative of the response that can be expected for said patient to said treatment. When performing this method, step (iii) is preferably performed once the tumor is sufficiently established which, in a murine model, corresponds to a size of 20 to 40 mm². This method is not covered by the claims.

The present disclosure also pertains to a method of assessing if a cancer patient is likely to be a good responder to a treatment with an immune checkpoint blocker, comprising the steps of (i) subjecting nucleic acid extracted from a feces sample of said patient to a genotyping assay that detects at least three of *Bacteroides salyersiae, Bacteroides acidifaciens, Bacteroides intestinalis, Bacteroides thetaiotaomicron, Bacteroides vulgatus, Prevotella, Bacteroides uniformis, Bacteroides massiliensis, Barnesiella intestinihominis, Prevotellaceae* and *Alloprevotella* bacteria, for example a genotyping assay that detects at least three of *Bacteroides thetaiotaomicron, Bacteroides vulgatus, Prevotella, Bacteroides uniformis, Bacteroides massiliensis, Barnesiella intestinihominis, Prevotellaceae* and *Alloprevotella* bacteria; (ii) determining a relative abundance of the at least three of the bacteria recited in (i), thereby generating a microbiome profile for said patient; (iii) comparing the obtained profile to at least two reference profiles corresponding to (a) patients statistically having more *Provetella* and/or *Alloprevotella* in their gut microbiota than a general population and (b) patients having more *Bacteroides thetaiotaomicron* in their gut microbiota than a general population; and (iv) if the obtained profile is closer to the profile recited in (a), the patient is likely to exhibit severe colitogenic effects. When this method is performed with a feces sample that has been obtained after at least one administration of said immune checkpoint blocker, step (iii) preferably also comprises the comparison of the obtained profile to a third reference profile corresponding to (c) patients statistically having more *Bacteroides vulgatus* in their gut microbiota than a general population, wherein if the obtained profile is closer to the profile recited in (c), the patient is likely to be a good responder to the treatment. This method is not covered by the claims..

Another method of assessing if a cancer patient is likely to be a good responder to a treatment with an immune checkpoint blocker based on a genotyping assay performed with nucleic acid extracted from a feces sample of said patient includes (i) determining a relative abundance, in said sample, of at least two bacteria selected amongst *Bacteroides salyersiae, Bacteroides acidifaciens, Bacteroides uniformis, Bacteroides fragilis, Bacteroides thetaiotaomicron, Bacteroides intestinalis* and (ii) determining a relative abundance, in said sample, of at least two bacteria selected amongst *Faecalibacterium prausnitzii, Ruminococcaceae, Parabacteroides distasonis, Candidatus Alistipes marseilloanorexicus, Coprobacter fastidiosus, Roseburia faecis, Oscillibacter valericigenes, Dorea longicatena, Blautia obeum, Oscillospiraceae bacterium, Alistipes shahii, Collinsella aerofaciens, Parabacteroides distasonis, Clostridium* sp., *Clostridiales* bacterium and *Alistipes finegoldii;* wherein if the patient statistically has more bacteria from the list recited in (i) and less bacteria from the list recited in (ii) in their gut microbiota than a general population, the patient is likely to be a good responder to the treatment. This method is not covered by the claims either.

When performing the above methods necessitating genotyping, any genotyping assay can be used. For example, this can be done by sequencing the 16S or the 23 S ribosomal subunit or by the more novel metagenomics shot gun DNA sequencing associated with metatranscriptomics.

Referring to the clusters described in the experimental part, the present disclosure relates to a method for determining, from a feces sample from a cancer patient obtained before administration of a treatment with an immune checkpoint blocker, whether said patient is likely to be a good responder to such a treatment, comprising analyzing the microbiota present in said sample, wherein if the profile of said microbiota corresponds to that of cluster B, the patient is likely to be a good responder to said treatment (provided the treatment induces a change in the enterotype pattern so that the patient falls into the favorable cluster C, which happens in about 30% of the cases, so that a cluster B patient has about 30% of chances to respond to the treatment, and 70% to resist thereto), and if the profile of said microbiota corresponds to that of cluster C, the patient is most likely to be a good responder to said treatment. If the profile of said microbiota corresponds to that of cluster A, the patient is likely to exhibit severe colitogenic effects (but, especially in the case of metastatic melanoma, the patient is likely to respond to the treatment). This method is not covered by the claims either.

Still referring to the clusters described in the experimental part, the present disclosure relates to a method for determining, from a feces sample from a cancer patient obtained after administration of a treatment with an immune checkpoint blocker, whether said patient is likely to be a good responder to such a treatment, comprising analyzing the microbiota present in said sample, wherein if the profile of said microbiota corresponds to that of cluster B, especially after two administrations of said ICB, the patient is likely to be a poor responder to said treatment, and if the profile of said microbiota corresponds to that of cluster C, the patient is likely to be a good responder to said treatment. If the profile of said microbiota corresponds to that of cluster A, the patient may exhibit severe colitogenic effects (but, especially in the case of metastatic melanoma, the patient is likely to respond to the treatment). This method is not covered by the claims either.

The present disclosure also relates to a method of assessing if a cancer patient who received a treatment with an immune checkpoint blocker is likely to be a good responder to such a treatment, comprising the steps of (i) analyzing gut microflora from a feces sample from said patient in order to determine a gut microbiome signature for said patient; (ii) comparing said gut microbiome signature of said patient to two or more gut microbiome reference signatures, wherein said two or more gut microbiome reference signatures include at least one of a positive gut microbiome reference signature based on results from patients who proved good responders to the same treatment and at least one of a negative gut microbiome reference signature based on results from patients who encountered severe colitogenic effects and/or failed to respond to said treatment; and (iii) if said gut microbiome signature for said patient statistically significantly matches said positive gut microbiome reference signature, then concluding that said patient is likely to be a good responder to said treatment; and/or if said gut microbiome signature for said patient statistically significantly matches said negative gut microbiome reference signature, then concluding that said patient is likely to be a poor responder to said treatment and/or to have severe side effects. This method is not covered by the claims either.

When an intestinal dysbiosis with an under-representation of the "favorable" bacteria listed above is observed, this shows that the patient requires a treatment to balance the gut microbiota prior to starting the antineoplastic treatment with an immune checkpoint blocker, or as an adjuvant of said treatment (e.g.: prebiotics or probiotics administration before/when starting the therapy). This is especially the case when the patient exhibits a cluster B profile. Hence, decision can be made to adapt the patient's regimen (providing pre- or probiotics) during a period of time (for example, a few weeks) before beginning the antineoplastic treatment. In particular, if the patient has been classified as likely to be a poor responder to said treatment and/or to have severe side effects by the above method, a further a step is preferably added, which comprises administering to said patient a probiotic composition and/or a fecal microbiota composition as above-described to ameliorate his/her response to the treatment. This aspect is not covered by the claims.

According to another aspect of the present disclosure, an individual in need of a treatment with a drug blocking CTLA4 such as an anti-CTLA4 antibody is treated by fecal microbiota transplant (FMT), using to this aim fecal microbiota from one or several patient(s) falling into cluster C, and/or fecal microbiota from healthy individual(s). This can be done for any patient, but of course such an FMT is particularly useful for an individual who has been identified as likely to be a poor responder to a treatment with said ICB, for example an individual fallin into cluster B. The present invention hence pertains to the use of a fecal microbiota composition as described above, for potentiating the anticancer effects of a treatment comprising immunotherapy with an anti-CTLA4 antibody blocking CTLA4 such as an anti-CTLA4 monoclonal antibody. The fecal microbiota composition used for FMT can be tested through any of the tests described above for identifying cluster C stools. The composition can allow the niching of *Bacteroides fragilis* and/or *Bacteroides thetaiotaomicron* upon transplant into a germ-free animal. It can also allow the expansion of *B. fragilis* upon transplant into a germ-free tumor-bearing animal after treatment of said animal with an anti-CTLA4 antibody. This FMT is preferably performed before the beginning of the treatment, for example a few days before, but it can also be performed in the course of said treatment.

As described in the experimental part below, the inventors also showed that a memory Th1 response towards certain bacteria is indicative that a patient treated with an anti-CTLA4 antibody is a good responder to the treatment. Accordingly, the present disclosure also pertains to a method for *ex vivo* determining whether a cancer patient is likely to benefit from a treatment with an immune checkpoint blocker, comprising assessing the presence of memory Th1 cells towards *Barnesiella intestinihominis, Bacteroides fragilis, Bacteroides thetaiotaomicron, Burkholderia cenocepacia* and/or *Burkholderia cepacia* in a blood sample from said patient, wherein the presence of such memory Th1 cells indicates that the patient is likely to be a good responder to said treatment, and the absence of such cells indicates that the patient is likely to be a poor responder and/or to encounter severe colitogenic effects. This method is not covered by the claims. As shown in Example 6 below, in lung cancer patients, the presence of memory T cells recognizing *Barnesiella intestinihominis* (and, to a lesser extent, *Burkholderia cepacia*) indicates that the patient is likely to be a good responder to a treatment with an immune checkpoint blocker, especially to a treatment combining irradiation and anti-CTLA4 antibodies. As shown in Example 6 below, the presence of memory Th1 cells towards *Bacteroides fragilis* and/or *Bacteroides thetaiotaomicron* in a patient having a metastatic melanoma indicates that the patient is likely to be a good responder to the treatment.

This method can be performed with a blood sample obtained before the beginning of any treatment with an immune checkpoint blocker. However, the sensitivity of the test may be insufficient to provide any clear result. This is not a problem anymore after one or two administrations of said immune checkpoint blocker, which is another moment when this method can be performed.

The T cell response towards *Barnesiella intestinihominis, Bacteroides fragilis, Bacteroides thetaiotaomicron, Burkholderia cenocepacia* and/or *Burkholderia cepacia* can also be measured in blood samples obtained from said patient prior to the therapy by an immune checkpoint blocker and after one or two administrations of said immune checkpoint blocker. In such a case, a decrease of IL-10 release and/or an increase of IFNγ release (upon *ex vivo* restimulation by CD14+ cells loaded with said bacteria) in said T cell response indicates that the patient is a good responder to said treatment. According to another aspect, the present disclosure pertains to a method for *in vitro* determining whether an antineoplastic treatment is to be continued or stopped for a cancer patient. This method is not covered by the claims. It comprises the following steps:
(i) from a biological sample from said patient, obtained 3 to 9 weeks after the beginning of said antineoplastic treatment, analyzing memory CD4⁺ T cell response directed against at least one commensal species of bacteria, for example against *Bacteroides fragilis* and *Bacteroides thetaiotaomicron;*
(ii) for each commensal species against which the CD4⁺ T cell response is analyzed, classifying the response in one of the following categories:
   - no memory CD4⁺ T cell response;
   - memory response of a Th10 phenotype;
   - memory response of a Th1 phenotype,
wherein if a memory response of a Th1 phenotype is observed for at least one commensal species, the antineoplastic treatment is continued, and in absence of such a response, the antineoplastic treatment is stopped.

In order to classify the responses, the secretions of IL-2, TNFα, IFNγ and IL-10 are measured in *ex vivo* restimulation assays. A first assay can be done before the beginning of the treatment, in order to compare the cytokine secretion profile after a few weeks of treatment to that observed pre-treatment. These assays can be performed, for example, using patients' autologous monocytes loaded with defined bacteria and incubated with CD4⁺CD45RO⁺ T cells purified from autologous blood. The response will be classified in the third (favourable) category if it is of a Th1 phenotype, *i.e.,* if restimulation triggers a significant secretion of IL-2, TNFα and IFNγ, and a low secretion of IL-10, especially when comparing the results obtained post- to pre-treatment. Typically, for a patient having a response of the Th1 phenotype, at least a 2-fold increase of IPNγ secretion is observed post-treatment (compared to pre-treatment). The first category (no memory CD4⁺ T cell response) corresponds to the absence of significant cytokine secretion in restimulation assays post-treatment, whereas the second category corresponds to a response in which the IL-10 secretion in a restimulation assay post-treatment is superior to that observed pre-treatment.

One particularly advantageous aspect of this pharmacodynamic method is that it can be performed using a blood sample. Of course, it can be done for patients having any kind of cancers.

To further investigate the role of Gram-negative bacteria in the immune response triggered by anti-CTLA4 molecules, the inventors have tested the response to Ipilumumab in mice lacking a functional TLR4 gene. The toll-like receptor 4, encoded by the TLR4 gene, detects lipopolysaccharide from Gram-negative bacteria and is thus important in the activation of the innate immune system. As shown below, the inventors demonstrated that the antitumor efficacy of CTLA4 blockade is partially dependent on TLR4 and TLR2. Hence, the present disclosure also pertains to an *in vitro* method of identifying a patient who is unable to fully respond to a drug blocking CTLA4, comprising determining the functionality of the toll-like receptor 4 (TLR4) and/or determining the functionality of the toll-like receptor 2 (TLR2) in said patient; wherein a patient lacking a functional TLR4 and/or a functional TLR2 is identified as not being a good responder to a drug blocking CTLA4. Two cosegregating single nucleotide polymorphisms (SNPs) - Asp299Gly and Thr399Ile - have been identified within the gene encoding TLR4. These SNPs are present in approximately 10% of white individuals, and have been found to be positively correlated with several infectious diseases. This method is not covered by the claims. In a particular embodiment of this method, the presence or absence of one or both of these SNPs is determined, for example by PCR or by any other method known by the skilled artisan.

Another aspect of the present disclosure not covered by the claims is the use, in adoptive T cell transfer, of a dendritic cell (DC) presenting antigens from one or several bacteria selected from the group consisting of *Bacteroides fragilis, Bacteroides thetaiotaomicron, Bacteroides salyersiae, Bacteroides acidifaciens, Bacteroides intestinalis, Bacteroides vulgatus, Bacteroides uniformis, Bacteroides massiliensis, Barnesiella intestinihominis, Burkholderia cepacia* and *Burkholderia cenocepacia,* in combination with an antineoplastic treatment comprising a drug blocking an immune checkpoint, especially in combination with an anti-CTLA4 molecule, for treating cancer. According to a particular embodiment, the DC presents antigens from one or several bacteria selected from the group consisting of *Bacteroides fragilis, Bacteroides thetaiotaomicron, Bacteroides vulgatus, Bacteroides uniformis, Bacteroides massiliensis, Barnesiella intestinihominis, Burkholderia cepacia* and *Burkholderia cenocepacia.* According to another particular embodiment, the DC presents capsular polysaccharides A (PSA) or PSA and adjuvants (such as TLR3L and anti-CD40 agonistic Ab) or zwitterionic polysaccharide repeated motifs from *B. fragilis* or lysine-aspartic acid (KD) peptides with >15 repetitive units or a combination of TLR2 and TLR4 agonists. Adoptive T cell transfer specific for *Bacteroides* spp., for example adoptive transfer of T cells amplified or primed *ex vivo* with antigen presenting cells exposed to ZPS or PSA or KD peptides, in a dysbiotic patient bearing a cancer and receiving anti-CTLA4 Ab, is hence an important part of the present disclosure.

The present disclosure also relates to a method for *ex vivo* obtaining T cells able to improve the anticancer activity of a drug blocking an immune checkpoint, comprising *ex vivo* expanding a polyclonal T cell line or bulk autologous T cells with dendritic cells (DC) presenting antigens from one or several bacteria selected from the group consisting of *Bacteroides fragilis, Bacteroides thetaiotaomicron, Bacteroides vulgatus, Bacteroides uniformis, Bacteroides massiliensis, Barnesiella intestinihominis, Burkholderia cepacia* and *Burkholderia cenocepacia,* and/or from the group consisting of *Bacteroides salyersiae, Bacteroides acidifaciens* and *Bacteroides intestinalis,* for example with DC presenting capsular polysaccharides A (PSA) or PSA and adjuvants or zwitterionic polysaccharide (ZPS) repeated motifs from *B. fragilis* or lysine-aspartic acid (KD) peptides with >15 repetitive units. The cells obtained through this method are advantageously used in adoptive cell transfer of T lymphocytes, in combination with an antineoplastic treatment comprising a drug blocking an immune checkpoint, for treating cancer. Such an adoptive cell transfer of T lymphocytes is particularly useful for improving the likelihood of a cluster B patient to respond to an antineoplastic treatment comprising a drug blocking an immune checkpoint such as an anti-CTLA4 antibody. This method is not covered by the claims.

Finally, the present disclosure also provides anticancer vaccines, comprising PSA and/or ZPS and/or KD. These vaccines are advantageously used in combination with an immune checkpoint blocker. These vaccines are not covered by the claims.

Other characteristics of the invention will also become apparent in the course of the description which follows of the biological assays which have been performed in the framework of the invention and which provide it with the required experimental support, without limiting its scope.

### EXPERIMENTAL RESULTS

### EXAMPLE 1 : ANTICANCER IMMUNOTHERAPY BY CTLA4 BLOCKADE RELIES ON THE GUT MICROBIOTA

**Abbreviations list:** ACS: antibiotic treatment with ampicillin, colistin and streptomycin, Bc: *Bukholderia cepacia* , Bf: *Bacteroides fragilis,* BM-DC: Bone marrow-derived dendritic cells, CTLA-4 : Cytotoxic T-Lymphocyte Antigen-4, DC: Dendritic cells, EMA: European Medicine Agency, FDA: Food and drug administration, FITC: fluorescein isothiocyanate, FMT: fecal microbiota transplant, GF: Germ-free, GM-CSF: Granulocyte-macrophage colony-stimulating factor, HV: Healthy volunteers, IBD: Inflammatory bowel diseases, ICB: Immune checkpoint blocker, ICOS: Inducible T-cell costimulatory, IL-12: Interleukin-12, LP: Lamina propria, mAb: Monoclonal antibody, MHC II: class II molecules , mLN: Mesenteric lymph node, MM: Metastatic melanoma, MOI: Multiplicity of infection, NOD2: Nucleotide-binding oligomerization domain-containing protein 2, PCA: Principle component analysis, PD1: Programmed cell death protein 1, PSA: Polysaccharide A, PBMC: peripheral blood mononuclear cells, SPF: Specific pathogen free, Tc1: Type 1 cytotoxic T-cells, Th1: T helper type 1 , TLR: Toll like receptor, Tr1: Type 1 regulatory T-cells, Tregs: Regulatory T cells.

### Materials & Methods

**Patients and cohorts characteristics.** All clinical studies were conducted after informed consent of the patients, following the guidelines of the Declaration of Helsinki. Peripheral blood mononuclear cells (PBMC) were provided by Gustave Roussy (Villejuif, France) and by the Department of Radiation Oncology (New York University, New York, NY, USA). Patients were included in the following protocols: LUDWIG: MAGE3 protein-based vaccines (25), Meladex vaccine Phase I trial (26), Mel-Ipi-Rx, NCT01557114 (European Union Drug Regulating Authorities clinical trial EudraCT 2010-020317-93) and a pilot study approved by the Kremlin Bicêtre Hospital Ethics Committee (n° SC12-018; ID RCB: 2012-A01496-37). Ipilimumab/radiotherapy abscopal effect trials in MM and NSCLC at NYU (Study code: NCT01689974, Study code: NCT0222173). MM patients at baseline were investigated prior to vaccine injections in the Ludwig and Meladex protocols. For memory T cell responses, blood samples were drawn from patients before and after 3 or 4 cycles of ipilimumab. Pyrosequencing analyses of 16S rRNA of gene amplicons in patients feces pre-and post-injections of ipilimumab (V0, V1, V2) were performed according to n° SC12-018; ID RCB: 2012-A01496-37 pilot study endpoints by GATC Biotech AG (Konstanz, Germany).

### Clinical studies.

GOLD: Prospective immunomonitoring study of patients with metastatic melanoma receiving four injections of Ipilimumab every three weeks. In addition to peripheral blood sampling for memory T cell responses assessment, feces were collected before each Ipilimumab injection. Feces samples were frozen at -80°C and sent for 16RNA pyrosequencing analysis at GATC Biotech AG (Konstanz, Germany).
MEL/IPI: Phase 1 trial that combines ipilimumab and radiation therapy to assess the synergy between the two modalities. Dose escalation radiotherapy was administered with ipilimumab on week 1, 4, 7 and 10 weeks at 10mg/kg. Subsequently, a maintenance dose of ipilimumab was given every 12 weeks as long as the patient had positive clinical response. Study code: NCT01557114
MAGE3.A1: Phase1b-II study to determine the toxicity and effect of subcutaneous injection of recombinant protein MAGE-3 in patients with metastatic melanoma not receiving any other immunologic agent. Study reference: (27)
MELADEX: Phase 1 clinical trial to assess safety and immunization of metastatic melanoma with exosomes purified from autologous monocyte derived dendritic cells pulsed with MAGE 3.A2 peptides. Study reference: (26)
MELANOMA ABSCOPAL TRIAL: Phase 2 randomized trial of ipilimumab versus ipilimumab plus radiotherapy in metastatic melanoma. Eligible patients have metastatic melanoma with at least 2 measurable sites of disease. All patients are randomly assigned to receive ipilimumab 3mg/kg i.v. versus ipilimumab 3 mg/kg i.v. plus fractionated radiotherapy to one of their measurable lesions. For patients assigned to the ipilimumab plus radiotherapy arm, ipilimumab treatment starts after radiotherapy, with a dose given on day 4 from the first radiotherapy fraction and repeated on days 25, 46 and 67. Response to treatment is evaluated at week 12 to assess clinical and radiographic responses in the non-irradiated measurable metastatic sites. Study code: NCT01689974.
NON-SMALL CELL LUNG CANCER ABSCOPAL TRIAL: Phase 2 study of combined ipilimumab and radiotherapy in metastatic non-small cell lung cancer (NSCLC). Eligible patients have chemo-refractory metastatic NSCLC with at least 2 measurable sites of disease. Patients receive ipilimumab 3mg/kg i.v., within 24 hours of starting fractionated radiotherapy. Ipilimumab is repeated on days 22, 43, and 64. Patients are re-imaged between days 81-88 and evaluated for responses in the non-irradiated measurable metastatic sites. Study code: NCT02221739

**Mice.** All animal experiments were carried out in compliance with French and European laws and regulations. Mice were used between 7 and 14 weeks of age. WT specific pathogen-free (SPF) C57BL/6J and BALB/c mice were obtained from Harlan (France) and Janvier (France), respectively, and were kept in SPF conditions at Gustave Roussy. C57BL/6 GF mice were obtained from Institut Pasteur and maintained in sterile isolators. *Il-10*^{*-*/*-*} C57BL/6 mice and WT C57BL/6 control animals were kindly provided by Anne O'Garra (National Institute for Medical Research, UK). *NOD2*^{*-*/*-*}, *Il10*^{*-* /-}, and *NOD2*^{*-*/}*⁻Il10*^{*-*/*-*} mice (BALB/c background) were obtained from Institut Pasteur Lille. C57BL/6 *Tlr2*^{*-*/*-*} mice were provided by Ivo Gompers Boneca (Institut Pasteur, Paris, France), *Tlr4*^{*-*/*-*} mice were bred and maintained in the animal facility of Gustave Roussy, Villejuif, France.

**Cell culture and reagents.** OVA-expressing mouse fibrosarcoma MCA205 cells, murine colon carcinoma MC38 cells (class I MHC H-2^{b}, syngeneic for C57BL/6 mice) and mouse colon carcinoma CT26 cells (class I MHC H-2^{d}, syngeneic for BALB/c mice) were cultured at 37°C under 5% CO₂ in RPMI-1640 medium supplemented with 10% heat-inactivated fetal bovine serum (FBS), 100 units/ml penicillin G sodium, 100 µg/ml streptomycin sulfate, 2mM L-glutamine, 1mM sodium pyruvate and non-essential amino acids (from this point on referred to as complete RPMI-1640; all reagents from Gibco-Invitrogen, Carlsbad, CA, USA). OVA-expressing MCA205 cells were selected in complete RPMI-1640 medium (as above) though supplemented with 50 µg/ml hygromycin B (Invitrogen, Life Technologies^{™}).

**Tumor challenge and treatment.** Mice were subcutaneously injected into the right flank with 1 × 10⁶ MCA205-OVA or MC38, or with 0.2 × 10⁶ CT26 tumor cell lines. When tumors reached a size of 25 to 40 mm² (day 0), mice were injected intraperitoneally (i.p) with 100µg of anti-CTLA-4 mAb (clone 9D9) or isotype control (clone MPC11). Mice were injected 5 times at 3-day intervals with 9D9, and tumor size was routinely monitored by means of a caliper. In order to evaluate the synergistic effect of vancomycin and anti-CTLA4, a prophylactic setting was established. In figure 1D, anti-CTLA4 treatment started two days after tumor inoculation. In experiments using anti-IL-12p40 mAb (clone C17.8, 500µg per mouse) or anti-Ly6G mAb (clone 1A8, 200µg per mouse), mAbs (or their isotype controls, clone 2A3 in both cases) were injected i.p. every 2 days starting from day 0 until the final anti-CTLA-4 injection. Anti-ICOS mAb (clone 17G9) or isotype control mAb (clone LTF-2) were injected at 250µg per mouse, at the same times as for anti-CTLA4 mAb. All mAbs for in vivo use were obtained from BioXcell (West Lebanon, NH, USA), using the recommended isotype control mAbs. The inhibitor of iNOS, NG-monomethyl-L-arginine (L-NMMA; Sigma), was administered in PBS daily to mice i.p. at a dose of 2mg per mouse from the start of anti-CTLA4 treatment.

**Antibiotic treatments.** Mice were treated with antibiotics 2-3 weeks before tumor implantation and continued on antibiotics until the end of the experiment. A mix of ampicillin (1 mg/ml), streptomycin (5 mg/ml), and colistin (1 mg/ml) (Sigma-Aldrich), or vancomycin alone (0.25 mg/ml), or imipenem alone (0.25 mg/ml), or colistin alone (2.103U/ml) were added in sterile drinking water. Solutions and bottles were changed 2-3 times a week, or daily for experiments with imipenem. Antibiotic activity was confirmed by macroscopic changes observed at the level of caecum (dilatation) and by cultivating the fecal pellets resuspended in BHI+15% glycerol at 0.1g/ml on blood agar plates for 48h at 37°C with 5% CO₂ in aerobic or anaerobic conditions.

**Flow cytometry.** Tumors and spleen were harvested two days after the third injection of anti-CTLA-4 for antibiotics experiments or at the end of tumor growth (around day 20) for germ-free experiments. Excised tumors were cut into small pieces and digested in RPMI medium containing Liberase^{™} at 25µg/ml (Roche) and DNase1 at 150UI/ml (Roche) for 30 minutes at 37°C. The mixture was subsequently passaged through a 100µm cell strainer. Two million splenocytes (after red blood cells lysis) or tumor cells were preincubated with purified anti-mouse CD16/CD32 (clone 93; eBioscience) for 15 minutes at 4°C, before membrane staining. For intracellular staining, the FoxP3 staining kit (eBioscience) was used. Dead cells were excluded using the Live/Dead Fixable Yellow dead cell stain kit (Life Technologies^{™}). Stained samples were run on a Canto II (BD Bioscience, San Jose, CA, USA) cytometer, and analyses were performed with FlowJo software (Tree Star, Ashland, OR, USA). For cytokine staining, cells were stimulated for 4 hours at 37°C with 50ng/ml of phorbol 12-myristate 13-acetate (PMA; Calbiochem), 1µg/ml of ionomycin (Sigma), and BD Golgi STOP^{™} (BD Biosciences). Anti-CD45.2 (104), anti-FoxP3 (FJK-16s), anti-ICOS (7E17G9), anti-IFN-γ (XMG1.2), anti-TNF-α (MP6-XT22), anti-CXCR3 (CXCR3-173) and isotype controls rat IgG1 (eBRG1), IgG2a (eBRG2a), IgG2b (eBRG2b) were purchased from eBioscience. Anti-CD3 (145-2C11), anti-CD25 (PC61.5.3), KI67 (FITC mouse antihuman KI67 set), rat IgG1κ were obtained from BD Bioscience. Anti-CD4 (GK1.5), anti-CD8β (YTS1567.7), Rat IgG2a (RTK2758) were purchased from Biolegend (San Diego, CA, USA). Anti-CCR6 (140706) was obtained from R&D Systems, Minneapolis, MN. Eight-color flow cytometry analysis was performed with antibodies conjugated to fluorescein isothiocyanate, phycoerythrin, phycoerythrin cyanin 7, peridinin chlorophyll protein cyanin 5.5, allophycocyanin cyanin 7, pacific blue, or allophycocyanin. All cells were analyzed on a FACS CANTO II (BD) flow cytometer with FlowJo (Tree Star) software.

**Microbial DNA extraction, 454 pyrosequencing and bacteria identification.** Fecal samples used in this study were collected before or after one injection of anti-CTLA4 (or isotype control) from mice under vancomycin regimen or water, and were kept at -80°C until further analysis. Library preparation and sequencing were conducted at GATC Biotech AG (Konstanz, Germany). *Bacterial isolation, culture, and identification.* Fecal pellet contents were harvested and resuspended in BHI+15% glycerol at 0.1g/ml. Serial dilutions of feces were plated onto sheep's blood agar plates and incubated for 48h at 37°C with 5% CO₂ in aerobic or anaerobic conditions. After 48h, single colonies were isolated and Gram staining was performed. The identification of specific bacteria was accomplished through the combination of morphological tests and analysis by means of an Andromas mass spectrometer (BioMérieux, France).

**Gut colonization with dedicated bacterial species.** For inoculation of GF mice or mice treated with broad-spectrum antibiotics, colonization was performed the day following the first anti-CTLA4 injection by oral gavage with 100µl of suspension containing 1 × 10⁹ bacteria. Efficient colonization was checked by culture of feces 48h post oral gavage. *Bacteroides fragilis, Bacteroides thetaiotaomicron, Bacteroides distasonis, Bacteroides uniformis, Lactobacillus plantarum and Enterococcus hirae* were grown on COS agar plates (Biomerieux) for 48h at 37°C with 5% CO₂ in anaerobic conditions. *E. coli* and *Burkholderia cepacia* were grown on COS agar plates for 24h at 37°C with 5% CO₂ in aerobic conditions. Bacteria were harvested from the agar plates, suspended in sterile PBS, centrifuged and washed once with PBS, then resuspended in sterile PBS at an optical density (600nm) of 1, which corresponds approximately to 1 × 10⁹ colony-forming units (CFU)/ml. In cases where more than one bacteria was administered, an equal volume of each bacteria suspension was mixed to give a suspension of equal proportion of each type of bacteria, to a total 1 × 10⁹ bacteria/ml. For bacteria reconstitution experiments using mice previously treated with antibiotics, antibiotics treatment was stopped after 2-3 weeks at the first anti-CTLA4 injection, and mice were orally gavaged with 1 × 10⁹ CFU the following day. *B. distasonis, B. uniformis, E. hirae,* and *E. coli* isolates used in the experiments were originally isolated from feces or mesenteric lymph nodes of SPF mice treated with anti-CTLA4 and identified as described above. *L. plantarum, B. fragilis and B. thetaiotaomicron* were provided by the Biobank of the Pasteur Institute, Paris, France. *Burkholderia cepacia* was kindly provided by the IUH Méditerranée Infection, Marseille, France (Table 2). LPS-EK and LTA-SA (Invivogen) were administrated by oral gavage, at a dose of 500µg per mouse.

**TCR cross-linking assays.** For cross-linking experiments, total cells isolated from draining or contralateral lymph nodes (after red blood cell lysis) were incubated in MaxiSorp plates (Nunc; 2 × 10⁵ cells per well), precoated with anti-CD3 mAb (145-2C11) (0.5 g per well; eBioscience). The supernatants were assayed at 48h by ELISA for mouse IFN-γ (BD).

**Cytokine and antimicrobial peptide quantification.** IL-12p70, IFN-γ (BD Biosciences) and IL-10 (R&D Systems, Minneapolis, MN), were measured by ELISA following the manufacturer's instructions. For quantification of lipocalin-2 from feces and caecum contents, individual (not pooled) samples were reconstituted in PBS containing 0.1% Tween 20 (at 100 mg/ml) and vortexed for 10-20 min to get a homogenous fecal suspension. These samples were then centrifuged for 10 min at 10,000 rpm. Clear supernatants were collected and stored at -20°C until analysis. Lipocalin-2 levels were determined in the supernatants using the Duoset murine Lcn-2 ELISA kit (R&D Systems, Minneapolis, MN) following the manufacturer's instructions.

**FITC Dextran Assay to assess intestinal permeability.** Mice were injected with either one dose or two doses of anti-CTLA4 or isotype control mAbs (administration as detailed above), and were water-starved overnight, 2 days following their last i.p. mAb administration. The following day, mice were orally administered with 0.44 mg/g body weight of a 100 mg/ml solution of FITC-dextran (FD4, Sigma) in PBS (pH 7.4). Four hours later, blood was collected from each mouse by cardiac puncture. Blood was allowed to clot overnight at 4°C, then subsequently centrifuged at 3,000 rpm for 20 minutes to collect the serum. Dilutions of FITC-dextran in PBS, and separately in pooled mouse serum were used as a standard curve, with serum from mice not administered FITC-dextran used to determine the background. Absorbance of 100µl serum (diluted in PBS) was measured by microplate reader with excitation and emission filters set at 485 (20 nm band width) and 528 nm (20 nm band width), respectively (28). Experiments were performed at least twice, independently, with each read performed in duplicate.

**Histology of gut tissue.** The whole small intestine (duodenum, jejunum and ileum) and the colon were removed, cleaned from feces and fixed in 4% PFA for 1h. Rehydratation of the tissue was performed in 15% sucrose for 1h and in 30% sucrose overnight. Small intestines or colons were cut longitudinally, with the resulting ribbons rolled, then embedded in optimum cutting temperature (OCT) compound (Sakura), snap frozen, and longitudinal 6 µm sections were prepared. For histological analysis, longitudinal sections were counterstained with hematoxilin and eosin. For histological quantitative analysis, inflammatory foci, appearance of the submucosa, length of villi, and the thickness of lamina propria were scored for each section by a pathologist.

**Intestinal MUC2 and Ki67 staining and evaluation.** Intestinal tissue was fixed in freshly prepared Methacarn solution for 4 hours, and subsequently incubated in methanol and toluene and embedded in paraffin. For immunohistochemistry, 5 µm-thick tissue sections were placed on Superfrost Plus slides (Thermo Scientific), incubated for 10 min at 60° C and rehydrated through a series of graded alcohol and distilled water. Endogenous peroxidases were blocked by 3% hydrogen peroxide for 10 min. Antigen retrieval was performed in citrate buffer (10 mM, pH 6) by steaming sections in a microwave oven for 20 min. Tissue sections were blocked with 5% BSA/PBS for 30 min at RT and primary antibodies against MUC 2 (1:200, sc 15334, Santa Cruz Biotechnology) and Ki-67 (1:100, ab15580, Abcam) were directly applied and incubated for 1 h at RT. Slides were washed 3x in PBS and secondary antibody (1:200, UP511380, Uptima) was applied for 1 hour at RT. Targeted antigens were visualized by using 3.3'-diaminobenzidine solution (BD Pharmingen) followed by nuclear counterstain with hematoxylin. For immunofluorescence staining, staining procedure was done according to the protocol provided by Cell Signaling Technology. Primary antibody against MUC2 was used as indicated. Secondary antibody (1:200, A11008, Life Technologies) was applied for 1 hour at RT followed by nuclear counterstain with DAPI. Microscopic analyses were performed by using the Zeiss Axioplan 2 imaging microscope, Axio Imager Z1 microscope, Axiovision software (all from Zeiss, Oberkochen, Germany), and ImageJ software (29). Evaluation of MUC2- and Ki-67-positive signals was performed by counting MUC2- and Ki-67-positive epithelial cells in all intact villi and/or crypts per tissue sections. Thickness of pre-epithelial mucus layer in distal colon was obtained by calculating mean values of 10 distinct measurement points per tissue section.

**Fluorescent in situ hybridization.** Methacarn-fixed, paraffin-embedded tissue sections (7 µm) of distal ileum and distal colon were incubated for 10 min at 60° C and deparaffinized in toluene for 30 min. After fixation in 95 % ethanol for 30 min, tissue sections were air-dried and incubated overnight (45° C) in hybridization buffer (20 mM Tris-HCl, 0.9 M NaCl, 0.1 % SDS, pH 7.4) containing Cy3-labelled EUB338 or Alexa488-labelled BAC303 bacterial probe in a concentration of 5 ng/µl. Non-specific binding of probes was removed by subsequent incubation of slides in prewarmed hybridization buffer and washing buffer (20 mM Tris-HCl, 0.9 M NaCl, pH 7.4), both for 15 min at 37° C. DAPI was used for nuclear counterstain and air-dried tissue sections were covered by using ProLong^{®} Gold Antifade reagent (Life Technologies, Saint Aubin, France). For combined mucus staining, anti-MUC2 antibody (1:200, sc 15334, Santa Cruz Biotechnology, Heidelberg, Germany) was applied for 1 hour at RT after first washing step in hybridization buffer, followed by secondary antibody incubation and DAPI staining. Tissue sections were washed in washing buffer for 15 min at RT and embedded in antifade mounting medium. Microscopic analysis was performed as described above. BAC303-positive signals were assessed by counting bacteria in a minimum of 10 intact crypts per tissue section.

**Quantification of bacteria by qRT-PCR.** Genomic DNA was isolated from fecal samples using the QIAamp DNA Stool Mini Kit (Qiagen) following the manufacturer's instructions. qRT-PCR was performed using the following specific 16S rRNA primers with SYBR green, with each primer mix including 10pM forward/reverse primers: *B. fragilis*16S forward : TGATTCCGCATGGTTTCATT (SEQ ID No: 1) and *B. fragilis*16S reverse: CGACCCATAGAGCCTTCATC (SEQ ID No: 2), *B.thetaiotaomicron\6S* forward : GGTA GTCCACACAGTAAACGATGAA (SEQ ID No: 3) and *B.thetaiotaomicron*16S reverse : CCCGTCAATTCCTTT GAGTTTC (SEQ ID No: 4), *B.uniformis* forward : TCTTCCGCATGGTAGAACTATTA (SEQ ID No: 5) and *B. uniformis* reverse : ACCGTGTCTCAGTTCCAATGTG (SEQ ID No: 6), *Parabacteroides distasonis* forward : TGCCTATCAGAGG GGGATAAC (SEQ ID No: 7) and *Parabacteroides distasonis* reverse : GCAAATATTCCCATGCGGGAT (SEQ ID No: 8), Universal 16S forward : ACTCCTACGGGAGGCAGCAGT (SEQ ID No: 9) and Universal 16S reverse : ATTACCGCG GCTGCTGGC (SEQ ID No: 10). qRT-PCR was carried out using a ABI PRISM 7300 quantitave PCR System (Applied Biosystem). Relative quantity was calculated by the ΔCt method and normalized to the amount of total bacteria

**Flow cytometry analyses of LP cell subsets.** *Isolation of lamina propria cells from colon.* The whole colon was harvested and Peyer's patches were removed, as well as all fat residues and feces. Colons were cut longitudinally and then transversally into pieces of 1-2 cm length. After removing the intra-epithelial lymphocytes (IELs), the colon pieces were further cut into approximately 1mm squares, and incubated with 0.25 mg/ml collagenase VIII and 10 U/ml DNase I for 40 min at 37 °C with shaking, in order to isolate lamina propria cells (LPCs). After digestion, intestinal pieces were passaged through a 100µm cell strainer. For flow cytometry analysis, cell suspensions were subjected to a percoll gradient for 20 min, centrifuged at 2100 RPMI. Anti-mouse antibodies for CD45.2 (104), CD3 (145-2C11), CD4 (GK1.5), IFN-γ (XMG1.2), RORyt (AFKJS-9), anti-ICOS (7E17G9) were obtained from BioLegend, eBioscience and R&D. *CTLA4 staining on lamina propria cell subsets.* Large intestines were opened longitudinally, washed from feces and incubated on ice in PBS/EDTA (25 mM, without Ca²⁺/Mg²⁺, Gibco). Epithelial cells were removed by repeated rounds of shaking in PBS. Subsequently, the intestine was cut into small pieces and digested with Liberase TL (Roche) / DNaseI (Sigma) mix in DMEM (Gibco) at 37°C. Tissue was disrupted by pipetting and passing over a 100 µm mesh (BD). Homogenates were subjected to a 40/80 % Percoll (GE Healthcare) gradient. Lymphocytes were harvested from the interphase and stained with fixable Live/Dead stain Blue (Invitrogen) according to the manufacturer's instruction. Surface staining was performed with anti-CD3-PE-CF594 (145-2C11, BD), anti-CD4-HorizonV500 (RM4-5, BD), anti-CD19-BrilliantViolet650 (6D5, BioLegend), anti-Thy1.2-PerCp-eFluor710 (30-H12, eBioscience), anti-NKp46-biotin (polyclonal, R&D), Streptavidin-BrilliantViolet421 (BioLegend) and anti-CTLA-4-APC (UC10-4B9, eBioscience) or Armenian Hamster IgG Isotype Control APC (eBio299Arm, eBioscience). Cells were fixed with 4% PFA (Sigma) and permeabilized and stained with 1x Perm buffer (eBioscience) and anti-Foxp3-PE (eBioscience), anti-GFP-Alexa488 (polyclonal, Molecular Probes) and CTLA-4-APC (UC10-4B9, eBioscience). Nonspecific binding was blocked with purified CD16/32 (93, eBioscience) and rat IgG (Dianova). Flow cytometry was performed on a Fortessa (BD) and data was analysed with FlowJo (TreeStar). *Analyses of DC subsets in anti-CTLA4 antibody-treated intestines.* Mesenteric lymph nodes were prepared by digestion with collagenase and DNase for 60 min and subsequently strained through a 70 m mesh. Colonic lymphocytes were isolated as previously described (30). In brief, colons were digested in PBS containing 5 mM EDTA and 2 mM DTT, with shaking at 37°C. After initial digestion, colonic tissue pieces were digested in collagenase/DNAse containing RPMI medium for 30 min. Tissue pieces were further strained through a 70 µm mesh. For flow cytometry analyses, cell suspensions were stained with antibodies against the following surface markers: CD11c (N418), CD11b (M1/70), MHC class II (M5/114.15.2), CD24 (M1/69), CD317 (ebio927), CD45 (30-F11), CD86 (GL1), CD40 (1C10). DAPI was used for dead cell exclusion. Antibodies were purchased from eBiosciences, BD Biosciences or BioLegend respectively. Cell populations were gated as follows: CD103⁺ DC (CD45⁺ CD11c⁺ MHC-II⁺ CD103⁺ CD24⁺), CD11b⁺ (CD45⁺ CD11c⁺ MHC-II⁺ CD11b⁺ CD24⁺), plasmacytoid DC (CD45⁺ CD11c⁺ MHC-II⁺ CD317⁺), mesenteric LNs (migratory fraction): CD103+ DC (CD45⁺ CD11c⁺ MHC-II⁺⁺ CD103⁺ CD24⁺), CD11b⁺ CD103⁺ (CD45⁺ CD11c⁺ MHC-II⁺⁺ CD103⁺ CD11b⁺ CD24⁺), CD11b⁺ (CD45⁺ CD11c⁺ MHC-II⁺⁺ CD11b⁺ CD24⁺), plasmacytoid DC (CD45⁺ CD11c⁺ MHC-II⁺ CD317⁺), mesenteric LNs (resident fraction): CD8+ DC (CD45⁺ CD11c⁺ MHC-II⁺ CD24⁺ CD11b⁻), CD11b⁺(CD45⁺ CD11c⁺ MHC-II⁺ CD11b⁺)

**Preparation of capsular polysaccharide-enriched fractions.** Fractions containing capsular polysaccharides were prepared as previously described (31). Briefly, bacteria were extracted twice by a hot 75% phenol/water mixture for 1h at 80°C. After centrifugation at 1000 g for 20 min, water phases were pooled and extracted with an equivalent volume of ether. Water phases were then extensively dialyzed against water and lyophilized. Extracted compounds were subsequently submitted to digestion by DNAase, RNAase, α-chymotrypsin, *Streptomyces griseus* proteases and trypsin. The digested solutions were dialysed against water and dried, resulting in fractions enriched in capsular polysaccharides. *Quantification of LPS content.* The presence of LPS in capsular polysaccharide-enriched fractions was investigated using the HEK-Blue^{™} TLR4 cell line (Invivogen , Toulouse), a derivative of HEK293 cells that stably expresses the human TLR4, MD2 and CD14 genes along with a NF-κB-inducible reporter system (secreted alkaline phosphatase). Cells were used according to the manufacturer's instructions. The different capsular polysaccharide-enriched fractions were added at concentrations ranging from 1 ng to 10 µg/ml in 96-wells plates and cells were then distributed at 5 × 10⁴ per well in 200 µl DMEM culture medium. Alkaline phosphatase activity in the culture supernatant was measured after 18h. LPS content was determined by TLR4 signaling activation by comparison with a standard curve obtained using ultrapure LPS from *E. coli* K12 (Invivogen, Toulouse). The LPS content in the PSA B. fragilis fraction was estimated at 1.4 pg/ µg of PSA. The LPS residual content in the PSA B.fragilis fraction is not responsible for its activity, as confirmed by the lack of any effect seen on addition of LPS in the PSA B. distasonis fraction compared with PSA B. distasonis alone.

**Assessing CD4⁺ T cell memory responses.** *Murine systems.* Bone marrow-derived dendritic cells (BM-DCs) were generated from femurs and tibiae of C57BL/6 mice, cultured for 7 days in Iscove's medium (Sigma-Aldrich) with J558 supernatant (final GM-CSF concentration of 40 ng/ml), 10% FCS, 100 IU/ml penicillin/streptomycin, 2 mM L-glutamine, 50 µM 2-mercaptoethanol (Sigma-Aldrich) and split every 3-4 days. At day 7, BM-DCs were infected with the isolated bacterial strains at multiplicity of infection (MOI) of 10 or 50, for 1h at 37°C in complete Iscove's medium without antibiotics, onto specific low binding 6 wells plates (Sigma). Cells were then washed with PBS and incubated in complete Iscove's medium supplemented with gentamicin (50µg/ml) to kill extracellular bacteria. For *Burkholderia cepacia,* the antibiotic meropenem (2µg/mL, Astra Zeneca) was required. After 24h, BM-DCs were cultured together with CD4⁺ T cells purified from the spleens of mice that had received 7 days treatment of anti-CTLA4 mAb (i.e 3 anti-CTLA4 mAb injections, as detailed above) in complete RPMI medium. CD4⁺ T cells were isolated by depletion of non CD4⁺ T cells using a cocktail of biotin-conjugated antibodies against CD8a, CD11b, CD11c, CD19, CD45R (B220), CD49b (DX5), CD105, Anti-MHC-class II, Ter-119 and TCRγ/δ as primary labeling reagent. The cells were then magnetically labeled with Anti-Biotin MicroBeads (Miltenyi Biotec, France). The magnetically labeled non-target cells were depleted by retaining them on an Automacs Separator, while the unlabeled target cells passed through the column. Co-cultures were set up at a ratio of 1 DC to 2 CD4⁺ T cells. We confirmed by flow cytometry that over 95% of magnetic-sorted cells were CD4⁺ T cells. Co-culture supernatants were collected at 24h, and assayed for IL-10 and IFN-γ using commercial ELISA. *Recall of human CD4*⁺ *T cell responses directed against commensals.* Frozen PBMC before and/or after ipilimumab therapy were thawed, washed and resuspended in the recommended separation medium (RoboSep Buffer; STEMCELL Technologies) for magnetic bead separation. Monocytes were enriched from 2 × 10⁶ PBMC (EasySep^{™} Human Monocyte Enrichment Kit, STEMCELL Technologies) and resuspended in RPMI-1640 (GIBCO Invitrogen), 10% human AB⁺ serum (Jacques Boy) supplemented with 1% 2 mmol/L glutamine (GIBCO Invitrogen), GM-CSF (1000UI/mL, Miltenyi), without any antibiotics. Monocytes were seeded in 96-well round bottom plates at 5 × 10³ cells/well either alone, in the presence of one of the five selected bacterial stains (Table 2) at a multiplicity of infection (MOI) of 100, or with LPS as positive control (1µg/mL, Sigma) plus sCD40L (1µg/mL, Miltenyi) and incubated for 1 hour at 37°C, 5% CO₂. During incubation, the remaining autologous PBMC fractions were enriched for memory CD4⁺ T cells (EasySep^{™} Human Memory CD4 Enrichment Kit, STEMCELL Technologies). The enriched CD4⁺CD45RO⁺ T cells were washed, counted and resuspended at 5 × 10⁴/well in RPMI-1640, 10% human AB⁺ serum, 1% 2 mmol/L glutamine, 20UI/mL IL-2 (Proleukine), 1% penicillin/streptavidin (PEST; GIBCO Invitrogen) and 50µg/ml of gentamycin (GIBCO Invitrogen). For *Burkholderia cepacia,* addition of meropenen antibiotic (2µg/mL, Astra Zeneca) was also required. CD4⁺CD45RO⁺ T cells were also incubated alone, or with CD3/CD28 beads (1µL/mL, Deanabeads T-Activator, InVitrogen) as positive control. Monocyte-bacteria/ CD4⁺CD45RO⁺ T cell co-cultures were incubated for 48 hours at 37°C, 5% CO₂. Monocytes and memory CD4⁺CD45RO⁺ T cell enrichment were confirmed as being >98% purity by flow cytometry. Supernatants were harvested, cleared by centrifugation (1200rpm, 5 min) and stored at -20°C for determination of IFN-γ, IL-10, as measured by commercial ELISA or Luminex MagPix technology (Biorad).

**Adoptive cell transfer.** CD4⁺ T cells were isolated from co-culture with DCs that had been pulsed with bacteria at a MOI of 10, as described above. One million CD4⁺ T cells were adoptively transferred i.v into GF mice, one day after the first anti-CTLA4 mAb injection. Mice were injected with anti-CTLA4 mAb and tumor sizes were monitored as detailed above. After 24h of coculture with bacteria as described above, BM-DCs were removed from low binding plates, washed, and counted. Alternatively to bacteria infection, BM-DC were pulsed with peptides (KD)₂₀ synthesized by Smartox Biotechnology (Gillonay, France). One million DC were adoptively transferred i.v into antibiotics -treated mice one day after the first anti-CTLA4 mAb injection. Mice were administered with anti-CTLA4 mAb and tumor sizes were monitored as detailed above.

**Statistical analysis.** Data were analyzed with Microsoft Excel (Microsoft Co., Redmont, WA, USA), Prism 5 (GraphPad, San Diego, CA, USA). Data are presented as means ± SEM and *p*-values were computed by paired or unpaired *t*-tests where applicable. Tumor growth was subjected to a linear mixed effect modeling applied to log pre-processed tumor volumes. The *p*-values were calculated by testing jointly whether both tumor growth slopes and intercepts (on a log scale) are where dissimilar between treatment groups of interests. All reported tests are two-tailed and were considered significant at *p*-values < 0.05. Statistical analyses pertaining to pyrosequencing of 16S rRNA of feces gene amplicons are detailed in Figure legends.

### Results

Ipilimumab is a fully human monoclonal antibody (mAb) directed against CTLA4, a major negative regulator of T cell activation (7). CTLA4, which is present in intracytoplasmic vesicles of resting T cells, is upregulated in activated T cells where it translocates to the plasma membrane to receive signals that ultimately maintain self-tolerance and prevent autoimmunity (2). Ipilimumab was the first FDA- and EMA-approved therapeutic (since 2011) after it had been shown to improve the overall survival of patients with metastatic melanoma (MM) in randomized Phase III trials (3, 4). After more than 7 years of follow-up, ipilimumab achieves durable disease control in approximately 20% of patients with MM (5). However, blockade of CTLA4 by ipilimumab often results in immune-related adverse events at sites that are exposed to commensal microorganisms, mostly the gut (6, 7). Patients treated with ipilimumab develop antibodies to components of the enteric flora (8), suggesting that such immune reactions may contribute to colitis. Despite these potentially live-threatening colitogenic effects, clinicians have combined CTLA blockade with that of another immune checkpoint, relying on the interraction beween PD-1 and PD-L1, further increasing objective responses as well as the side effects (9). Therefore, addressing the role of gut microbiota in the immunomodulatory effects of CTLA4 blockade is crucial for the future development of immune checkpoint blockers as antineoplastic agents.

We compared the relative therapeutic efficacy of the anti-CTLA4 Ab 9D9 against established MCA205 sarcomas in C57Bl/6 mice housed in normal laboratory conditions (which are specific pathogen-free, SPF) *versus* germ-free (GF) conditions. Five consecutive systemic administrations of the 9D9 Ab controlled tumor progression in SPF but not in GF mice (Fig. 1a, b). We next analyzed the effects of several antibiotics regimen on the antitumor effects of anti-CTLA4 Ab against MCA205 sarcomas in SPF conditions. A combination of broad-spectrum antibiotics (ampicillin + colistin + streptomycin, ACS), which sterilized the feces (Fig. 1c, left and right panels), as well as two antibiotics that mostly kill Gram⁻ bacteria, imipenem or colistin, (Fig. 1c, right panel), compromised the antitumor effects of 9D9 Ab. In addition, in GF or ACS-treated mice, the 9D9 Ab-induced activation of splenic effector CD4⁺ T cells, monitored by expression of Ki67 and ICOS, was dramatically suppressed (Fig. 1d), coinciding with reduced intratumoral accumulation of CD3⁺TILs and Th1 and Tc1 cells (Fig.1e, left middle and right panels). In contrast, antibiotherapy with ACS failed to compromise the depletion of intratumoral regulatory Foxp3⁺ CD4⁺ T cells (Tregs) induced by 3 intraperitoneal injections of 9D9 Ab, yet did affect the activation status of Tregs (Fig. 5a). These results implying Gram⁻ bacteria in the anticancer effects of CTLA4 blockade were confirmed for additional tumor types including the colon carcinomas MC38 (which grow in C57Bl/6 mice) and CT26 (which grow in BALB/c mice) (Fig. 5b, c).

Changes in the colonic microbiota from mice responding to CTLA4 blockade were determined by pyrosequencing of 16S ribosomal RNA gene amplicons. The principle component analysis indicated that one single injection of anti-CTLA4 Ab sufficed to significantly affect the microbiome at the genus level (Fig. 2a). CTLA4 blockade induced a rapid underrepresentation of both *Bacteroidales* and *Proteobacteria Burkholderiales* with a relative increase of *Clostridiales* (Fig. 2b, Table 1). The 9D9 mAb-induced underrepresentation of *Bacteroidales* family members was corroborated by qPCR analyses (Fig. 6). Next, we searched for CTL4 blockade-induced memory T cell responses specific for the most immunomodulatory species among *Bacteroidales,* in particular from the genus *Bacteroides* (*10, 11*). CD4⁺ T cells harvested from spleens of 9D9 (versus isotype control) mAb-treated mice were restimulated with various strains of *Bacteroides* (Table 2) loaded onto syngeneic bone marrow-derived dendritic cells (BM-DC). Memory Th1 responses consisting in the production of interferon-γ were observed against *B. fragilis* (*Bf*) and *B. thetaiotaomicron* (*Bt*)*,* with little concomitant IL-10 production (Fig. 7), mainly in splenocytes from 9D9 mAb-treated mice (Fig. 2c). Of note, no memory response could be detected in recall responses to *B. distasonis* (a rather abundant bacterium of the gut intestine) or *B. uniformis* (Fig. 2c). These findings suggest that the 9D9 Ab elicited immune responses against some *Bacteroides species* that may, in turn, have skewed the intestinal microbiome repertoire.

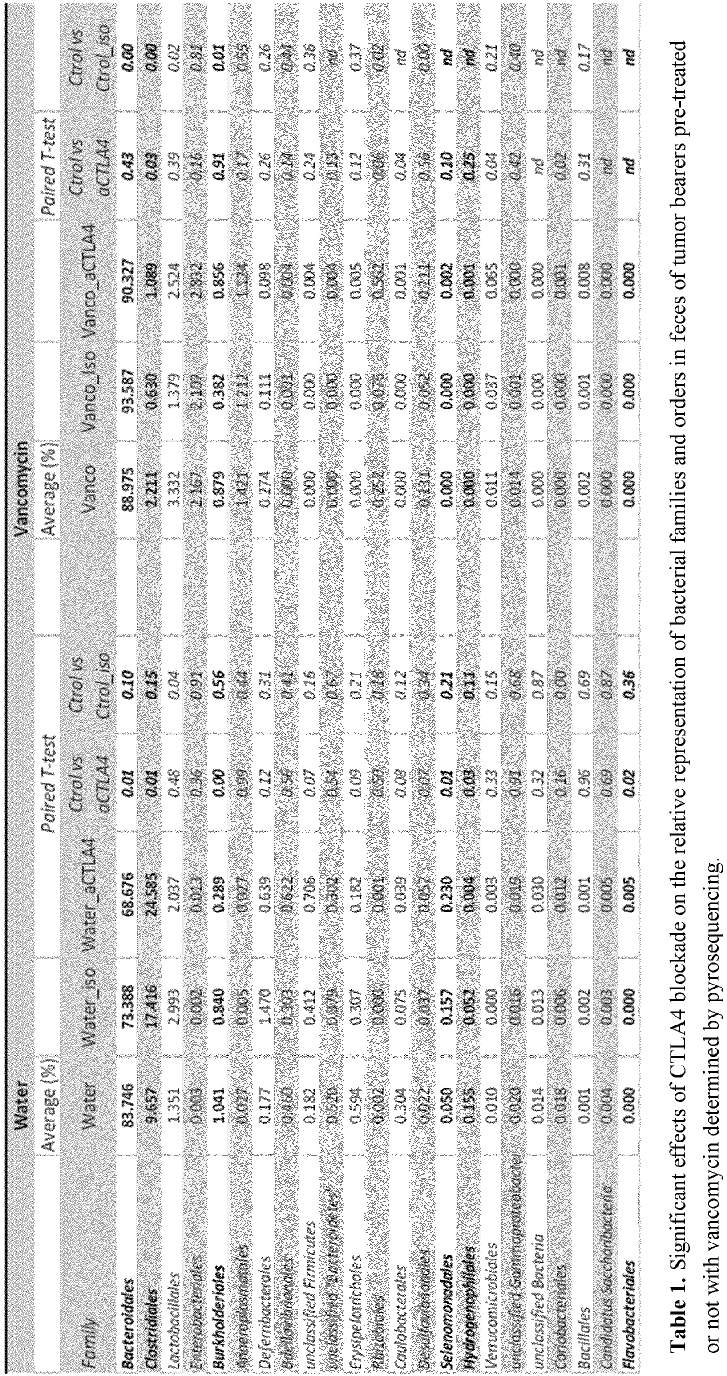

Next, we explored the clinical relevance of these experimental findings in patients with metastatic melanoma (MM) or advanced non-small cell lung cancer (NSCLC), by analyzing the composition of the gut microbiome and memory T cell responses against commensals before and after treatment with ipilimumab. A clustering algorithm that was based on genus composition and validated using the Calinski-Harabasz Index (12) revealed good performance in recovering two and three clusters before and after the first cycle of ipilimumab respectively (Fig. 2d-e, left panels, Monte-Carlo test, *p*= 0.046 and 0.07 respectively). Random Forest analysis was applied to identify the principal genera responsible for this significant clustering in ipilimumab-naïve patients. *Bacteroides, Enterorhabdus* and *Alloprevotella* were the main determinants of this clustering, with *Alloprevotella* driving cluster A and *Bacteroides species* driving cluster B (mostly *B. thetaiotaomicron, B. uniformis* and *B. massiliensis*) (Fig. 2d, middle and right panels). Post-ipilimumab, *Bacteroides, Dorea, Clostridium XIV* and *Alloprevotella* were the main determinants of the clustering with *Alloprevotella*/*Prevotella* (mostly *Alloprevotella rava)* driving cluster A and *Bacteroides* driving clusters B and C (*B. uniformis* for cluster B, *B. vulgatus* for cluster C, and *B. thetaiotaomicron* for both clusters B and C) (Fig. 2e, middle and right panels). During ipilimumab therapy, the proportions of MM patients falling into cluster C augmented to the expense of that belonging to cluster B (from 26 to 39% and from 58 to 39% respectively) (Fig. 8). We also analyzed circulating memory Th1 and Tr1 T cell responses against *Bacteroides* species and other commensals prior to and after at least 2 intravenous administrations of ipilimumab (3mg/kg/injection), given as a standalone therapy or combined with local radiotherapy. Compared with healthy volunteers (HV) (Fig. 2f, left panel), cancer patients exhibited a decreased commensal-specific Th1 immunity (Fig. 2f, middle panel) that, at least in some cases, could be restored by ipilimumab (Fig. 2f, right panel). Considering the mean values of IFNγ and IL-10 concentrations at baseline for the Melanoma patients, we determined the percentages of cases that became positive (and hence overcame the mean value) with the scope of identifying the repertoire of T cell specificities that was stimulated by CTLA4 blockade. Ipilimumab significantly increased IFNγ secretion and inhibited IL-10 production by T cells responding to *Bf, B. thetaiotaomicron and to some extent Burkholderia cepacia* (but not *E. coli* and *E. faecalis* ) (Fig. 2g, and Fig. 9a). Paired analyses performed on 5-12 patients of this cohort confirmed the ipilimumab-mediated decrease of *B.thetaiotaomicron-*specific Tr1 immune responses (Supplemental Fig. 9b, c).

Since patients and mice similarly responded to CTLA4 blockade by developing memory Th1 memory responses against *Bacteroides species,* we addressed the functional relevance of such T cell responses for the anticancer activity of the 9D9 Ab in mice. CTLA4 blockade by 9D9 Ab failed to reduce MCA205 cancers in GF mice, and this deficient response could be restored by the adoptive transfer of memory *Bf-*specific Th1 cells that had been restimulated with *Bf ex vivo* (Fig. 3a). In contrast, adoptive transfer of CD4⁺T cells restimulated in the presence of *B. distasonis* failed to improve the control of tumor growth (Fig. 3a). Knowing the importance of TLR2 and TLR4 in sensing *B.fragilis* (*10, 11*) and Gram⁻ bacteria respectively, we investigated the potential contribution of innate signaling through these two pathogen pattern receptors to the antitumor effects of CTLA4 blockade against MCA205 sarcomas and CT26 colon cancers. In *Tlr2*^{*-*/*-*} and *Tlr4*^{*-*/*-*} hosts, the 9D9 mAb partially lost its efficacy (Fig. 3b, Fig. 10 in BALB/c mice). However, oral administration of the prototypic TLR2 and TLR4 agonists, lipoteichoic acid and *E. coli* lipopolysaccharide respectively, failed to restore the 9D9 mAb-induced antitumor effects lost in ACS-treated mice (Fig. 3c), suggesting that TLR2 and TLR4 signaling is required but not sufficient to stimulate cancer relevant immune responses.

Driven by the consideration that innate signals may not be as essential as cognate immune responses against *Bacteroidaceae* family members, we investigated the dynamic changes of lamina propria dendritic cells (LP-DC) during CTLA4 blockade. Flow cytometric analyses of LP-DC subsets indicated a brisk loss of plasmacytoid DC in the colon accompanied by their local maturation (Fig. 3d, left panels) with a progressive accumulation of LP-CD11b⁺DC and a concomitant maturation in mesenteric lymph nodes of CD103⁺ DCs and CD103⁺CD11b⁺ DCs (Fig. 3d, right panels). In accord with the known capacity of *Bacteroides species* to occupy a mucosal niche in intestinal crypts (13, *14),* we were able to visualize the accumulation of *Bf* at the bottom of the colonic and ileal crypts at 48 hrs post-9D9 Ab by fluorescent in situ hybridization using a specific probe (Fig. 3e-f). Polysaccharide A (PSA) is (one of) the critical component(s) of *Bf* that explains its capacity to elicit IL-12-dependent IPNγ producing T cell responses and to stimulate lymphoid organogenesis in GF mice (*15*). Thus, *Bacteroides* zwitterionic polysaccharides, which contain alternating positive and negative charges in each repeating unit, can be taken up by CD11b⁺ DC and presented to T cells in the context of MHC class II molecules (*11*, *16, 17*)*.* Indeed, loading of CD11b⁺ DC with live *Bf* (but less so with *B. distasonis* or *B. uniformis*) induced a strong IL-12p70 release (Fig. 3g). The production of IL-12 (a heterodimer composed by p35 and p40 subunits) appears functionally relevant because the anti-CTLA4 Ab-mediated antitumor effects against MCA205 sarcoma in C57BL/6 mice (and against CT26 in BALB/c mice, not shown) were significantly reduced by a neutralizing anti-IL-12p40 Ab (Fig. 3h). Of note, IL-23 which shares p40 subunit with IL-12, was not significantly modulated by 9D9 Ab in the LP (not shown). Next, we loaded CD11b⁺ DC with purified polysaccharides from *Bf* capsids and injected these DC intravenously into ACS-treated mice. This maneuver rendered CTLA4 blockade more effective in ACS-treated mice (Fig. 3i), but only when DC were pulsed with *Bf* antigens, not when they were pulsed with *B.distasonis* capsids or the I-A^{b}-restricted KD peptidomimetic of zwitterionic motifs (18) (Fig. 3i). Only *Bf* capside polysaccharides promoted IL-12 release by DC (Fig. 3g, right panel). Of note, oral feeding of ACS-treated mice with *Bf* PSA (but not *B.distasonis* capsids) was sufficient to ameliorate the efficacy of CTLA4 blockade in half of the animals (Fig. 11), suggesting though that live *Bf* is more efficient that its inert antigens to stimulate an efficient anticancer immune response.

We considered the possibility that CTLA4 blockade might stimulate IL-12-dependent Th1 immunity against *Bacteroidaceae* by compromising gut homeostasis. 9D9 Ab administration failed to affect the subcellular distribution of CTLA4 molecules in LP TH17 and IL-10-producing regulatory T cells (Treg) that remained preferentially intracellular, at the same time as LP CD4⁺ T cells became ICOS⁺ in response to 9D9 Ab (Fig. 12). Importantly, mice deficient in IL-10- (but not NOD2-) manifested exacerbated histopathological signs of intestinal toxicity upon 9D9 administration, although they failed to control tumor growth in response to CTLA4 blockade (Fig. 13). In normal C57BL/6 mice, simultaneous blockade of CTLA4 and ICOS induced a strong increase in splenic TH1, Tc1 and pathogenic TH17 cells, and eventually caused death of 20% of the animals (Fig. 14). These findings suggest that monotherapy with CTLA4-blocking Ab does not affect the major pillars of gut tolerance (IL-10, NOD2, or ICOS) and that colitis is not required for the improvement of anticancer immunosurveillance elicited by CTLA4 blockade.

Altogether these results suggest that the microbiota-dependent immunostimulatory effects induced by CTLA4 blockade do not result from TLR2/TLR4-mediated innate signaling in the context of a compromised gut tolerance but rather from the mobilization of LP CD11b⁺ DC taking up a precise range of bacterial species and then mount IL-12-dependent cognate Th1 immune responses. To establish cause-effect relationships between the 9D9 Abs mediated- skewing of *Bacteroidales* and *Burkholderiales,* and the therapeutic efficacy of CTLA4 blockade, we applied an antibiotic regimen that skews the intestinal microflora towards Gram⁻ bacteria. Vancomycin induced the overrepresentation of the *Bacteroidales* (and *Enterobacteriales*) at the expense of *Clostridiales* and prevented the depletion of *Bacteroidales* and *Proteobacteria Burkholderiales* that is usually provoked by CTLA4 blockade (Fig. 2a-4a, left panel, Table 1). In contrast to pre-treatment with colistin or imipenem, two antibiotics which mainly killed Gram⁻ bacteria and compromised the antitumor effects of 9D9 Ab (Fig. 1c), vancomycin, which kills Gram⁺ bacteria, actually ameliorated the tumor growth reduction induced by anti-CTLA4 Ab (Fig. 4a, left panel). Next, we recolonized a priori sterile GF and ACS-treated mice with a panel of distinct bacterial strains. Oral feeding of GF mice with *Bf* completely restored the therapeutic response of tumor bearers to the 9D9 Ab (Fig. 4b, left panel). Moreover, *Bf* induced Th1 immune responses in the tumor draining lymph node (but not in the contralateral lymph node) (Fig. 4b, middle panel) and promoted the upregulation of CD80 in tumor infiltrating CD11c⁺I-A^{b+} DC (Fig. 4b, right panel). Similarly, oral gavage with *Bf, Bt, Burkholderia cepacia* (Table 2) or the combination of both completely compensated the lack of responsiveness of ACS-treated mice to anti-CTLA4 Ab, contrasting with all other isolates that failed to do so (Fig. 4c, left panel).

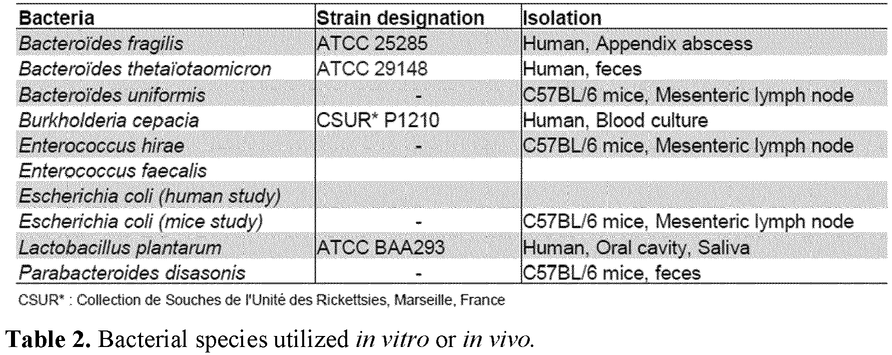

To evaluate whether the therapeutic efficacy of CTLA4 blockade might be uncoupled from its gut toxicity, we next addressed the impact of the gut microbiota on the incidence and severity of intestinal lesions. Neither weight loss nor intestinal bleeding were observed after 3 intraperitoneal injections of 9D9 Ab. Orally administered fluorescein isothiocyanate (FITC)-dextran failed to become detectable in the blood in spite of repeated 9D9 Ab injections, arguing against an increase in intestinal permeability (Fig. 15). 9D9 Ab did not significantly affect the mucus layer and the density of goblet cells, neither in distal ileum nor in the colon (Fig. 16a, b). However, colonic and ileal epithelial cells exhibited an increase proliferation index after one injection of 9D9 Ab in a Reg3b-dependent manner (Fig. 16c). Of note, 9D9 Ab reduced the transcription of the *Reg3b* gene in colon LP (not shown). Moreover, the antimicrobial peptide, lipocalin-2, which is often associated with neutrophil activation, markedly increased after repeated injections of 9D9 mAb in the caecum and stools in 50% of animals (Fig. 17) with a concomitant increase in RORyt -expressing TCRγδ and TCRαβ T cells as well as in Th1 cells (Fig. 18). The length of the villi and the mucosae height of the colon were significantly reduced following CTLA4 blockade (Fig. 19) in mice reared in SPF conditions, but less so in GF mice, suggesting that 'subclinical colitis' depends on the gut microflora (Fig. 19c). Depletion of neutrophils, which were increased in tumors post-CTLA4 blockade in a microbiota-dependent manner (Fig. 20 a-b), using the 1A8 Ab failed to affect gut toxicity or treatment efficacy. This applies also to the neutralization of granulocyte-derived metabolite nitric oxide by means of L-NMMA, which failed to affect the 9D9 mAb-induced tumoricidal activity (Fig. 20 c-e).

Vancomycin, which boosted the antitumor effects of 9D9 Ab (Fig. 1C) also worsened the histopathological score (Fig. 4A, right panels), suggesting that the efficacy and toxicity of CTLA4 blockade are mechanistically linked. However, efficacy and toxicity could be uncoupled when we evaluated the putative intestinal side effects of cancer therapy-relevant *Bacteroidaceae* in the context of a dysbiosis generated by ACS treatment and CTLA4 blockade. Intestinal reconstitution of ACS treated-animals with the combination of *Bf and B. cepacia* did not increase but rather reduced histopathological signs of colitis induced by CTLA4 blockade (Fig. 4d-e). We conclude that defined *Bacteroides* species can stimulate specific Th1 immune responses that are required for optimal therapeutic efficacy of CTLA4 blockade without that such species would contribute to the intestinal toxicity of the treatment. Hence, uncoupling toxicity and therapeutic efficacy can be achieved.

Among different environmental factors, the intestinal microbiota has emerged as a possible candidate responsible for the priming of aberrant systemic immunity in inflammatory bowel diseases and other autoimmune disorders (19). Hence, IgA coating defines a subset of bacteria that selectively stimulates intestinal immunity and mediate pathogenic processes in colitis ulcerosa and Crohn disease (20). TH17 cells specific for *segmented filamentous bacteria* are primed in the lamina propria, recirculate to secondary lymphoid organs and exacerbate systemic autoimmune diseases (21). As a result, much effort has been dedicated to the identification of bacteria that may mitigate autoimmune disease, with the ultimate scope to manipulate the gut microbiota for reducing adverse immune responses.

Here, we identified Gram⁻ bacteria, mostly *Bacteroides* species (such as *Bf* and *B. thetaiotaomicron*) as essential contributors to the warranted, live-preserving immune response induced by CTLA4 blockade. Such bacteria appear to mediate an essential positive contribution to the anticancer immune response required for restraining tumor growth, yet do not contribute to the intestinal side effects induced by CTLA4 blockade. What could be the factors dictating why such commensals might represent suitable "anticancer-probiotics"? First, the *Bf*-mediated cognate immunity in the context of CTLA4 blockade might stem from its geodistribution at the bottom of the colonic crypts. Second, its coordinated association with distinct *Proteobacteria* such as *Burkholderia cepacia* is remarkable. Indeed, this pathobiont, known to cause chronic lung infections in immunocompromised individuals, is recognized through the pattern recognition receptor Pyrin/casp-1 inflammasome that senses Bcc-induced RHOA deamidation for an optimal IL-1-dependent immune defence (22) synergizing with the TLR2/TLR4 signalin pathways required for *Bf.* Thirdly, a potential molecular mimicry between distinct commensal/pathobionts and tumor neoantigens is conceivable, eventually accounting for the efficacy of ICB (23) but deserves further experimental support.

Moreover, whether distinct anticancer therapies may profit from different probiotic adjuvants remains to be addressed. Surprisingly, the bacterial species that mediate adjuvant effects vary with the therapeutic agent that is used. In a previous study (24), we identified *E. hirae* as the bacterial species that increased the therapeutic effects of cyclophosphamide-based chemotherapy, contrasting with the present study that priviledges the use of *Bf* for optimizing the antineoplastic efficacy of CTLA4 blockade. Future studies must determine whether each of the multiple immune checkpoint blockers that is in preclinical or clinical development will synergize with distinct bacterial species or whether they obey to a common rule in which a fixed set of bacterial species favors anticancer immunosurveillance

It remains to be determined whether surmising on anti-cancer probiotics or synbiotics may be applied to humans. In particular, it remains an open conundrum whether probiotics, i.e. live bacteria, can be advantageously replaced by suitable bacterial products for triggering innate or acquired immune responses that favor the definitive elimination of tumor cells from the organism. Such anticancer pre- or pro- or syn-biotics may benefit patients presenting with a deviated gut microbiome, as suggested by the "enterotyping" of cancer patients that may predict autoimmune side effects, efficacy or resistance to ICB. This contention should be further substantiated by prospective studies.

### EXAMPLE 2: REFINING OF FECES CLUSTERING AND ASSESMENT OF ITS CLINICAL RELEVANCE

### Additional Materials and Methods

**Fecal microbiota transplant (FMT) experiments.** Germ-free adult female C57BL/6J mice were obtained from CDTA (Orleans, France) and Institut Pasteur (Paris, France). Fecal samples from GOLD cohort were frozen after being homogenised with brain-heart-infusion media (BHI) supplemented with 15% glycerol (0.1g/ml) and stored immediately at -80°C. FMT was performed by thawing the fecal material and 0.2 ml of the suspension was transferred by oral gavage into each germ-free recipient. In addition, another 0.1ml was applied on the fur of each animal. Mice were subsequently maintained in a gnotobiotic isolator with irradiated food and autoclaved water in our animal facility (Plateforme Evaluation Préclinique, Villejuif, France). Two weeks after microbiota transfer, MCA205-OVA tumor was injected subcutaneously and mice were treated with anti-CTLA4 mAb or isotype control as follows: mice were subcutaneously injected into the right flank with 1 × 10⁶ MCA205-OVA. When tumors reached a size of 20 to 40 mm² (day 0), mice were injected intraperitoneally (i.p) with 100µg of anti-CTLA-4 mAb (clone 9D9). Mice were injected 5 times at 3-day intervals with 9D9, and tumor size was routinely monitored by means of a caliper.

### Results

To further address the clinical relevance of our experimental findings, we analyzed the composition of the gut microbiome before and after treatment with ipilimumab in 25 MM patients. A clustering algorithm based on genus composition and validated using the Calinski-Harabasz Index revealed good performance in recovering three clusters before and under ipilimumab therapy (Fig. 21A, left panel, Monte-carlo test p value=0,000199). Random Forest analysis was applied to identify the principal genera responsible for this significant clustering in melanoma patients treated with ipilimumab. *Bacteroides, Prevotella,* and to a lesser extent *Flavonifractor* and unclassified *Ruminoccocaceae,* were the main determinants of this clustering (Fig. 21A, right panel and Table 3) with *Alloprevotella*/*Prevotella* driving cluster A and distinct *Bacteroides* spp. driving clusters B and C (Fig. 21B, right panel and table 3). Contrary to what was initially noted with a reduced number of patients (Example 1), we identified some cluster C patients before the beginning of the treatment with ipilimumab. We observed that during ipilimumab therapy, the proportions of MM patients falling into cluster C augmented to the expense of that belonging to cluster B (from 26 to 39% and from 58 to 39% respectively) (Fig. 21B, left panel, Fig. 22). This suggests a conversion of some patients' feces towards a more immunogenic enterotype. In a paired analysis of feces performed in 12 cases, the calculations indicated that out of the 7 stools of a B pattern, only 2 (29%) turned into the C favorable enterotype. It appears that ipilimumab could modify the pattern of enterotypes and after 1 or 2 injections of ipilimumab, so that about one third of individuals may fall into the favorable cluster C.

To support the clinical relevance of the ipilimumab-associated changes of the stool bacterial composition in the antitumor efficacy of this ICB, we performed fecal microbial transplantation (FMT) of feces harvested from three different MM patients of each enterotype two weeks before the first administration of ipilimumab into germ-free mice that were subsequently challenged with tumor cells. While this maneuver did not modulate the natural tumor growth, it markedly impacted the anticancer activity of the anti-CTLA4 Ab. Tumors growing in mice that had been transplanted with feces from cluster C patients markedly responded to CTLA4 blockade, contrasting with absent anticancer effects in mice transplanted with feces from cluster B patients (Fig. 21C). These FMT transfers into GF mice convey clearcut sensitivity or resistance to anti-CTLA4 Ab in cluster C and B, respectively.

Quantitative PCR analyses revealed that although bacteria from the *Bacteroidales* order equally colonized the recipient murine intestine, stools from cluster C (but not A or B) individuals specifically facilitated the colonization of the immunogenic bacteria *B. thetaiotaomicron* and *B. fragilis* (Fig. 21D). Moreover, after anti-CTLA4 therapy, only cluster C (not A or B) recipient mice manifested the outgrowth of *B. fragilis.* Importantly, the fecal abundance of *B. fragilis* (but not *B. distasonis* nor *B. uniformis*) negatively correlated with tumor size post-CTLA4 blockade in cluster C (but not in A or B) recipient mice (Fig. 21E). Altogether, these findings show that ipilimumab can modify the abundance of immunogenic *Bacteroides* species in the gut, which in turn impacts on its anticancer efficacy.

### EXAMPLE 3: A PREDICTIVE TOOL FOR THE CLINICAL RESPONSE TO CTLA4 BLOCKADE (ALONE OR COMBINED WITH IMMUNE CHECKPOINT BLOCKERS)

16S rRNA pyrosequencing of feces gene amplicons in patients followed by enterotyping of feces as cluster C and to a lesser extent cluster A can predict a good clinical outcome and benefit to ICB.

As described in Example 2 above, enterotyping can be performed by pyrosequencing of gene amplicons in feces. Three clusters (A, B, C) have been identified. The relative abundance of main *Bacteroides* spp significantly differed between cluster B and C. FMT of feces pre- versus post-ipi from several patients falling into each of the three clusters were transferred into GF animals. Tumor growth kinetics was followed (Fig.21C). Cluster C pre-or post-therapy and to a lesser extent cluster A (a rare enterotype in MM patients) stools from pre-therapy appeared to condition or predict the antitumor effects mediated by ipilimumab, suggesting that the immunogenic *Bacteroides* spp and some other family members of the *Bacteroidales* genus (i.e *Prevotella*) can all ignite antitumor immunity. In contrast, cluster B individuals completely resisted to therapy. The same experiment, performed with samples of cluster C feces from several patients, confirmed the positive effect of bacteria present in cluster C feces on the antitumor effects mediated by ipilimumab.

Clusters A and C are defined as described in Table 3 below:

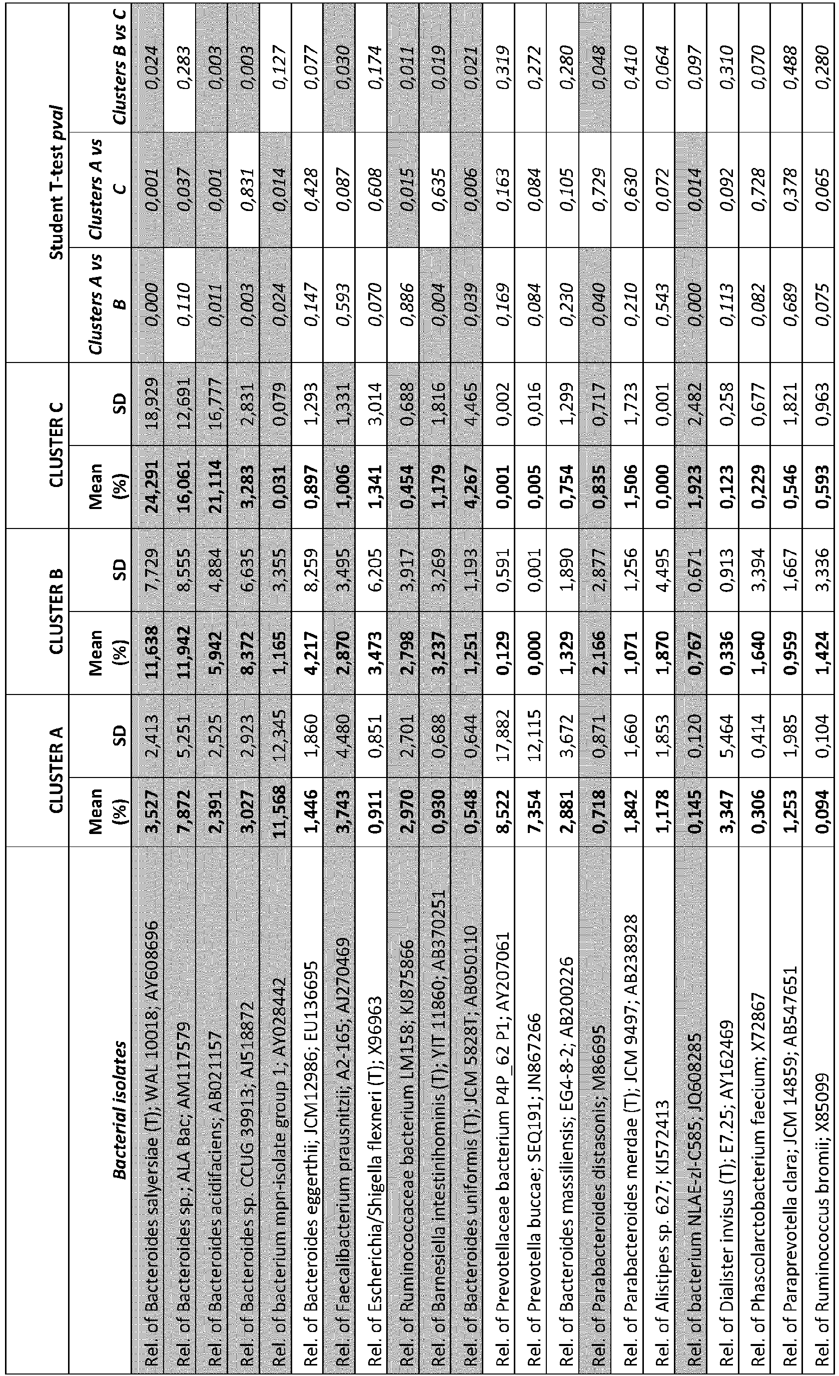

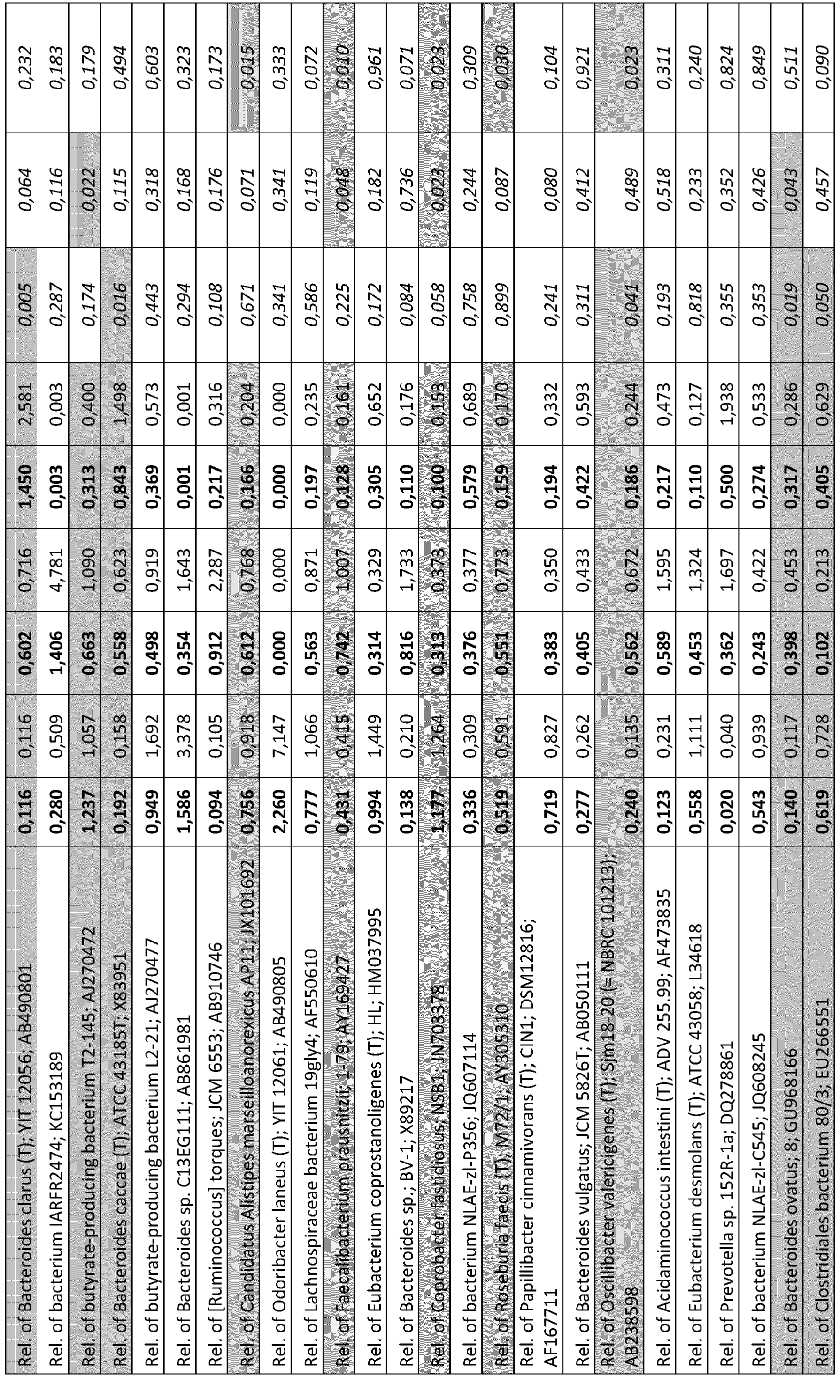

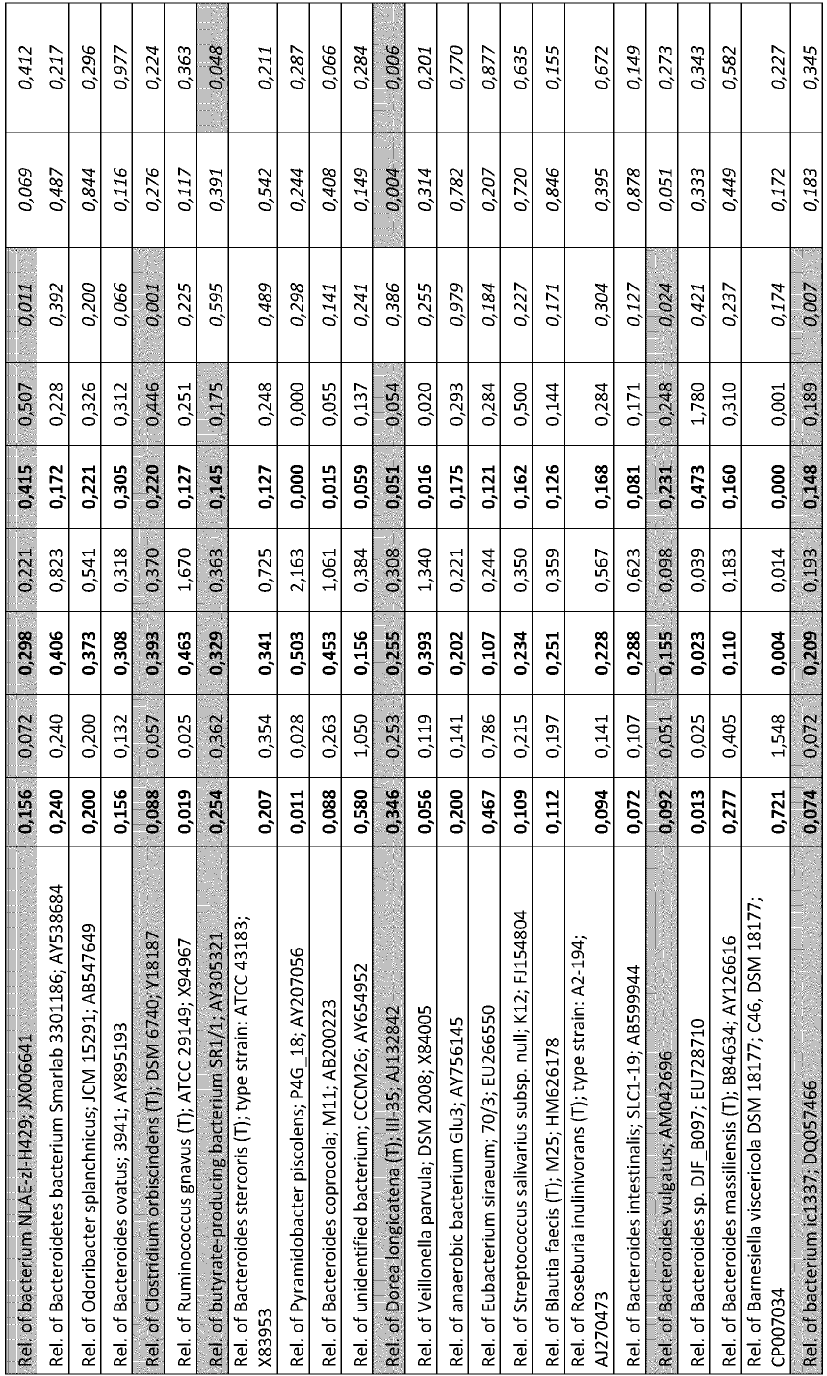

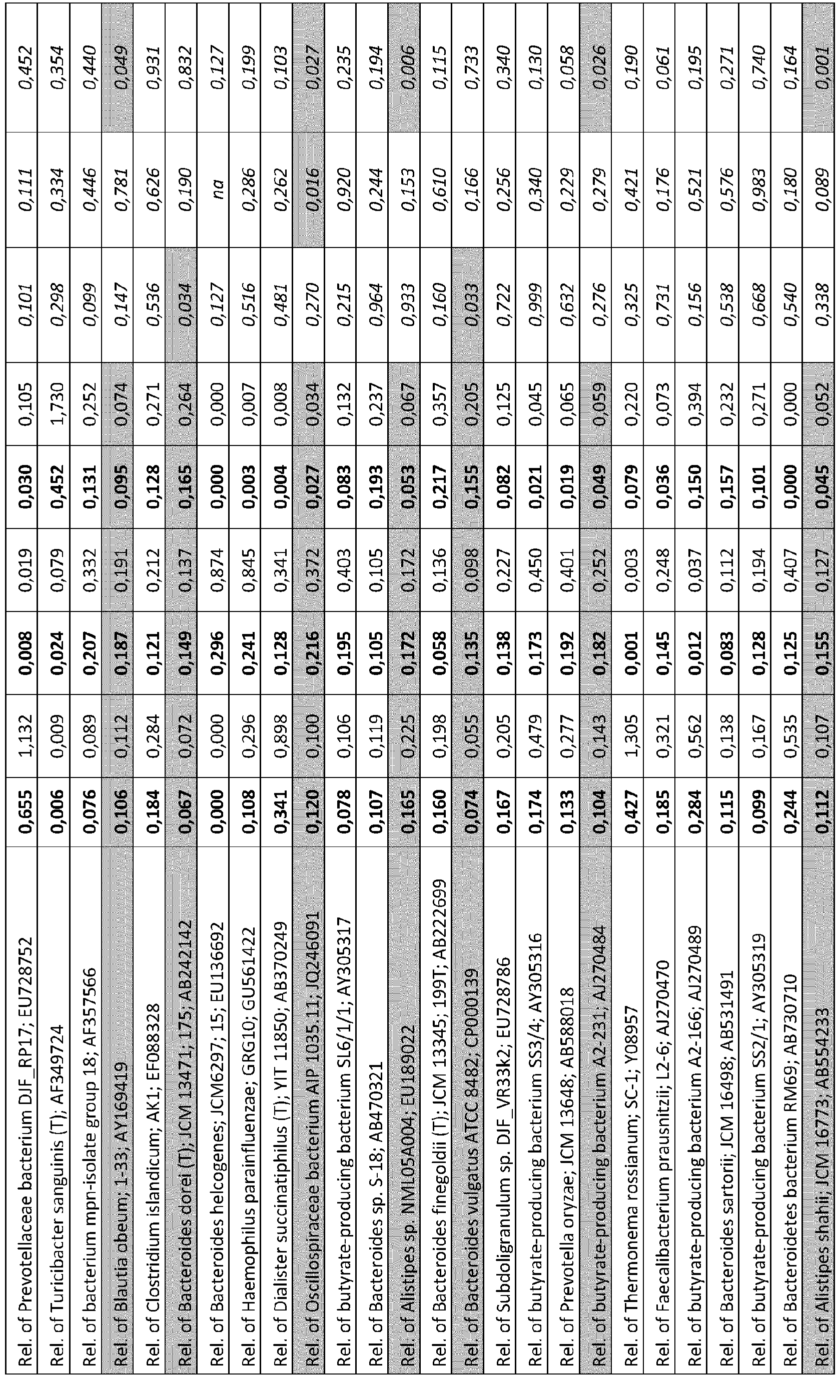

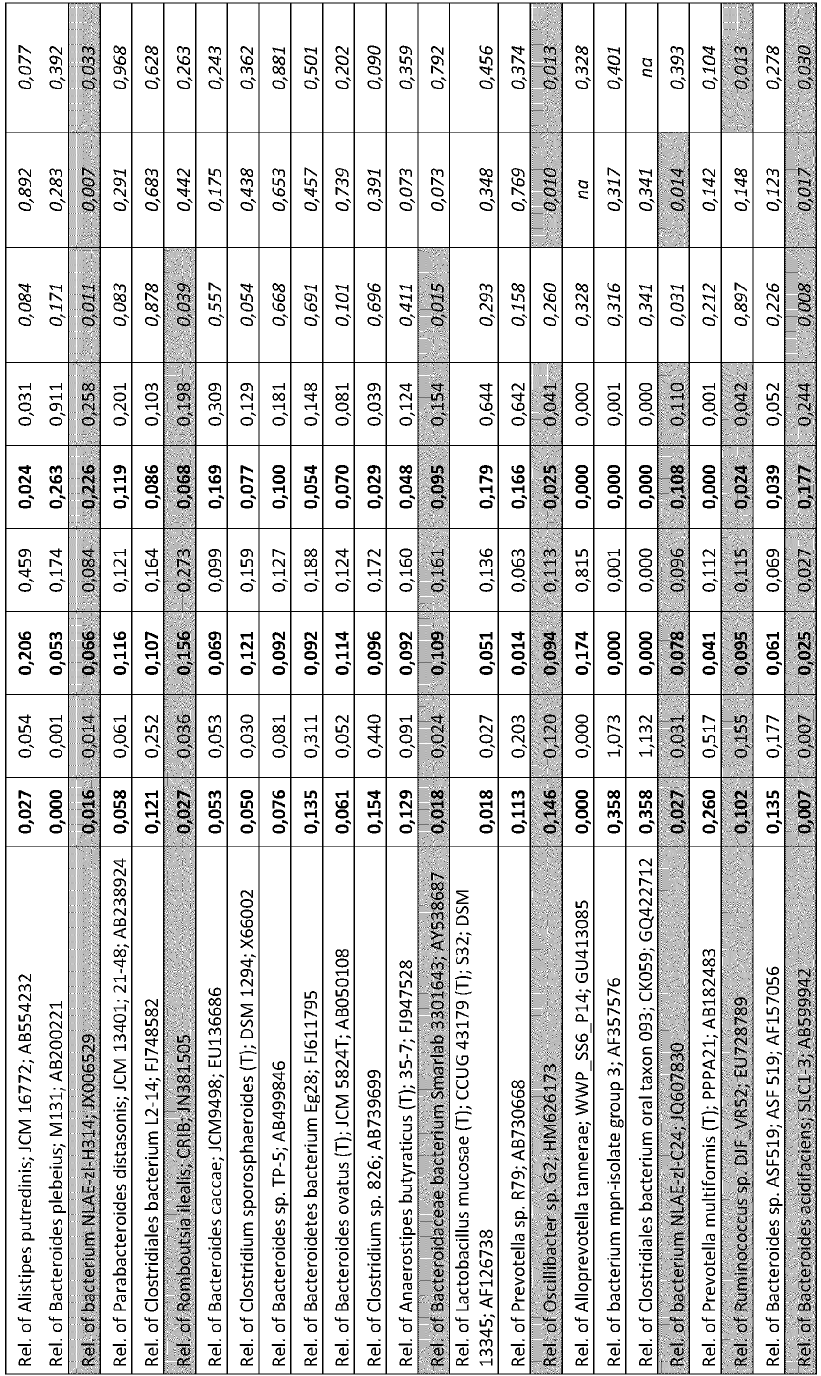

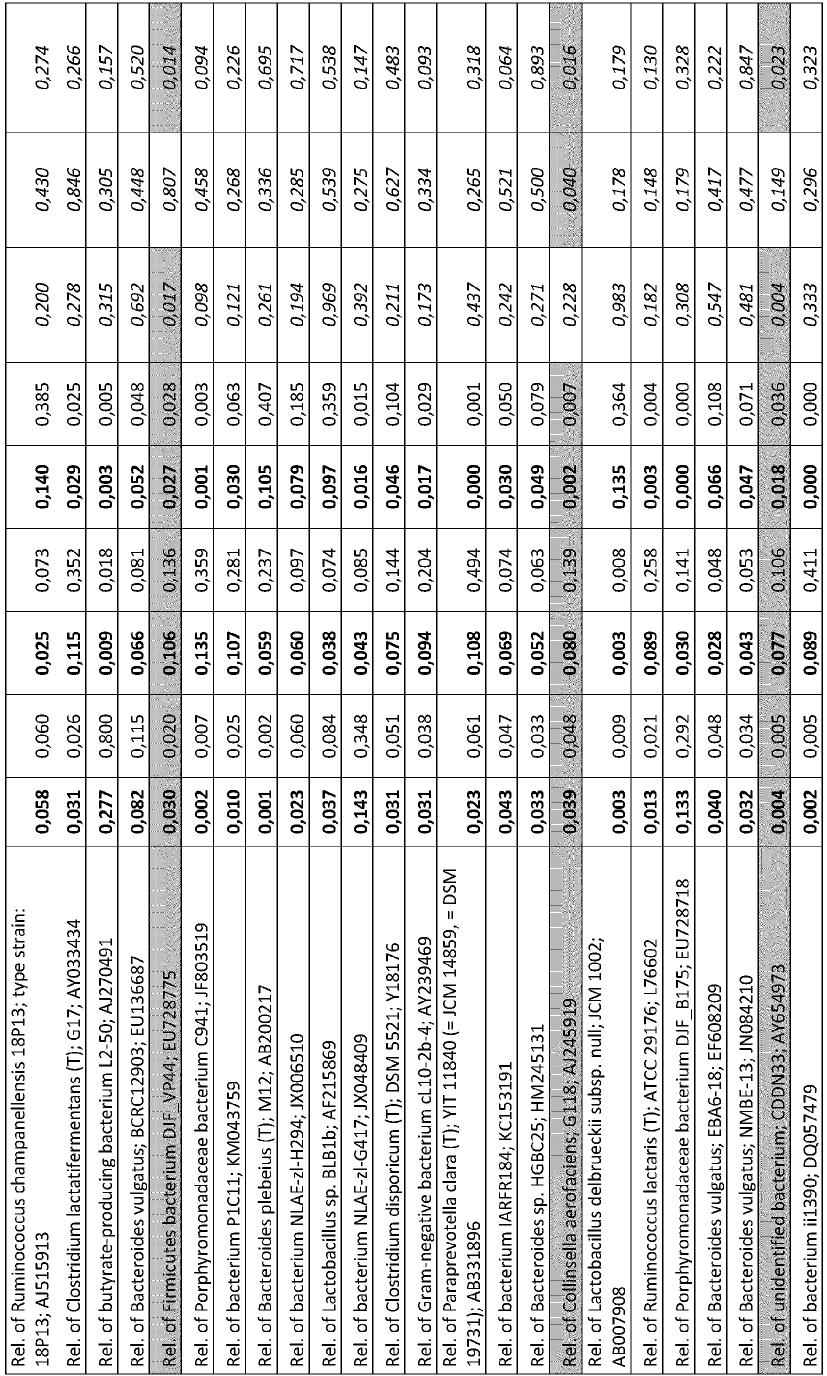

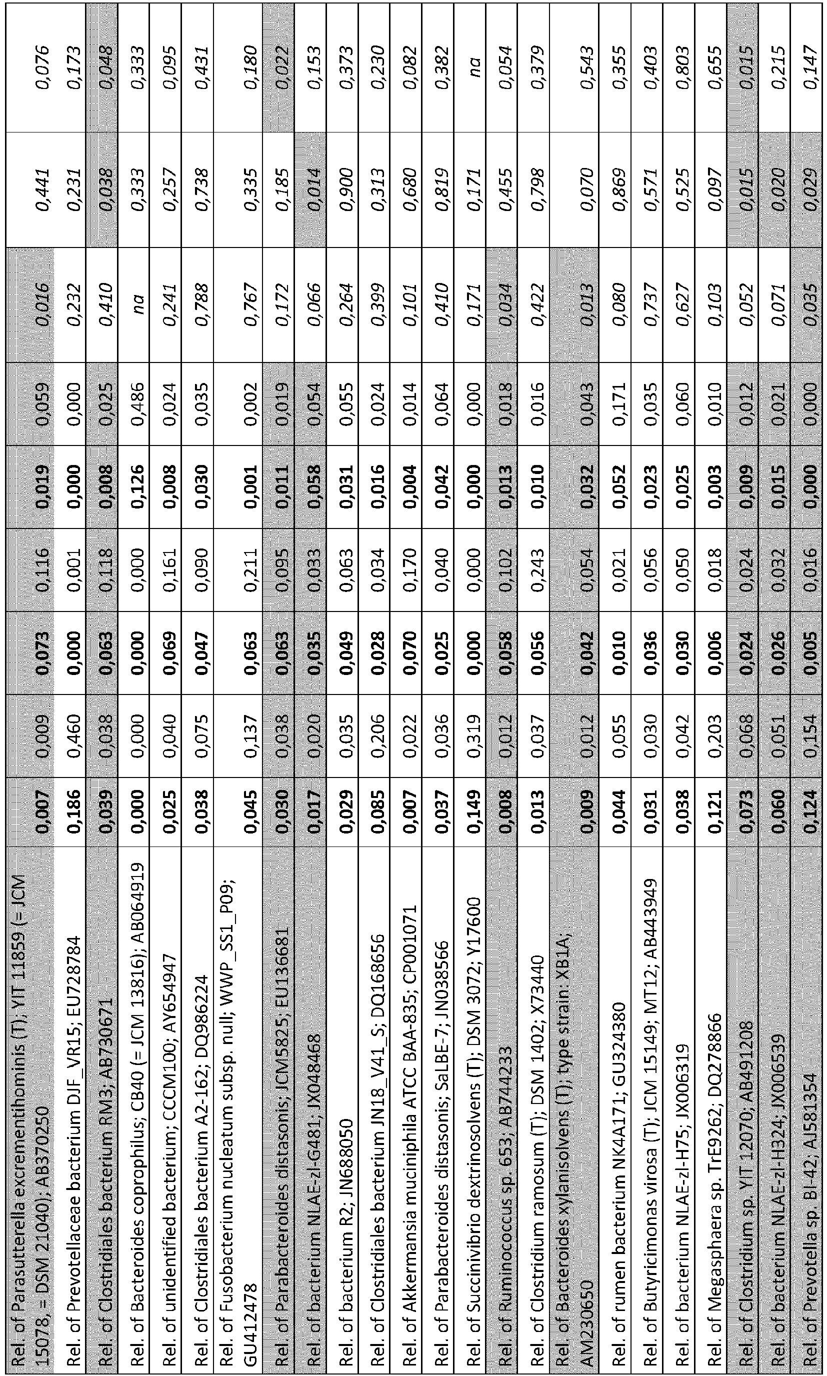

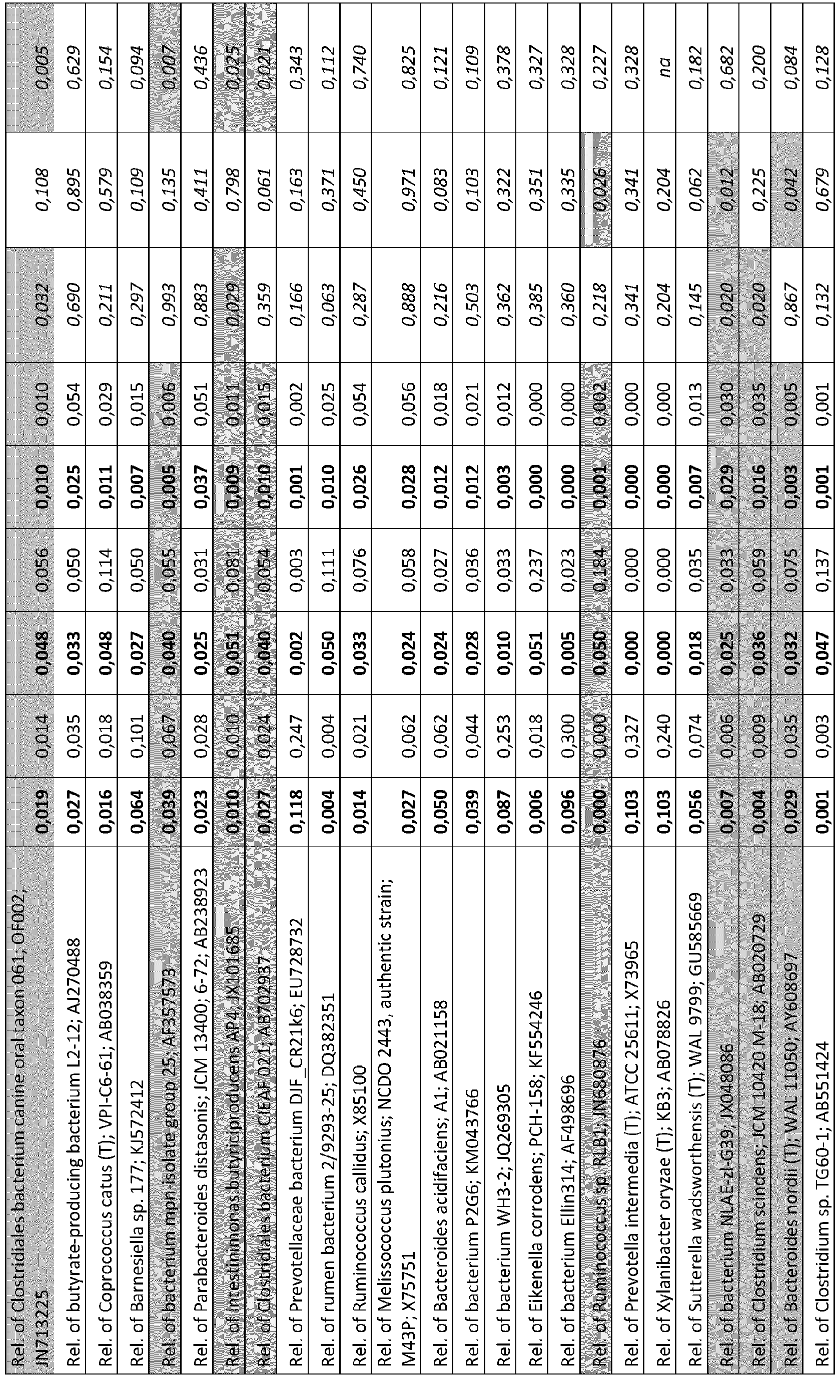

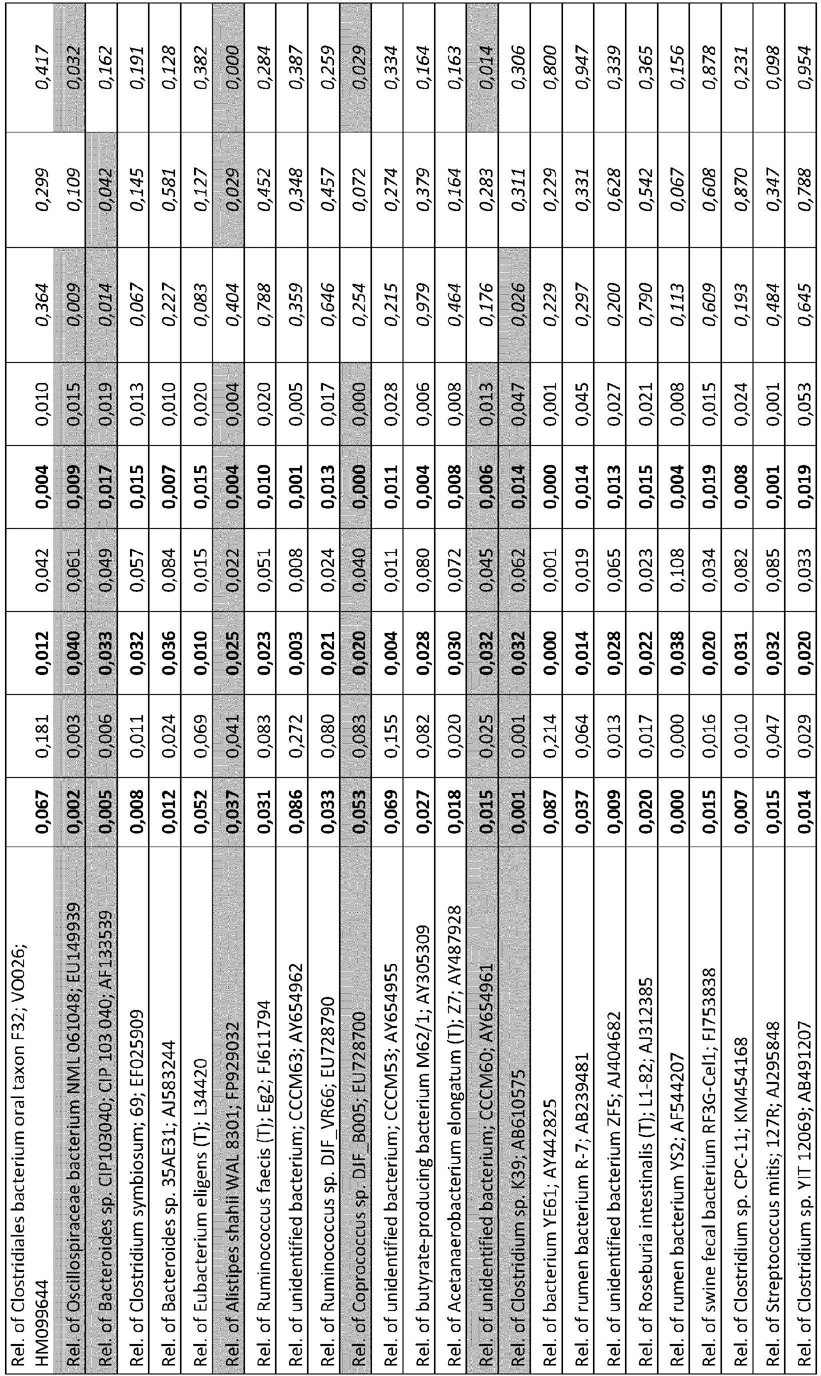

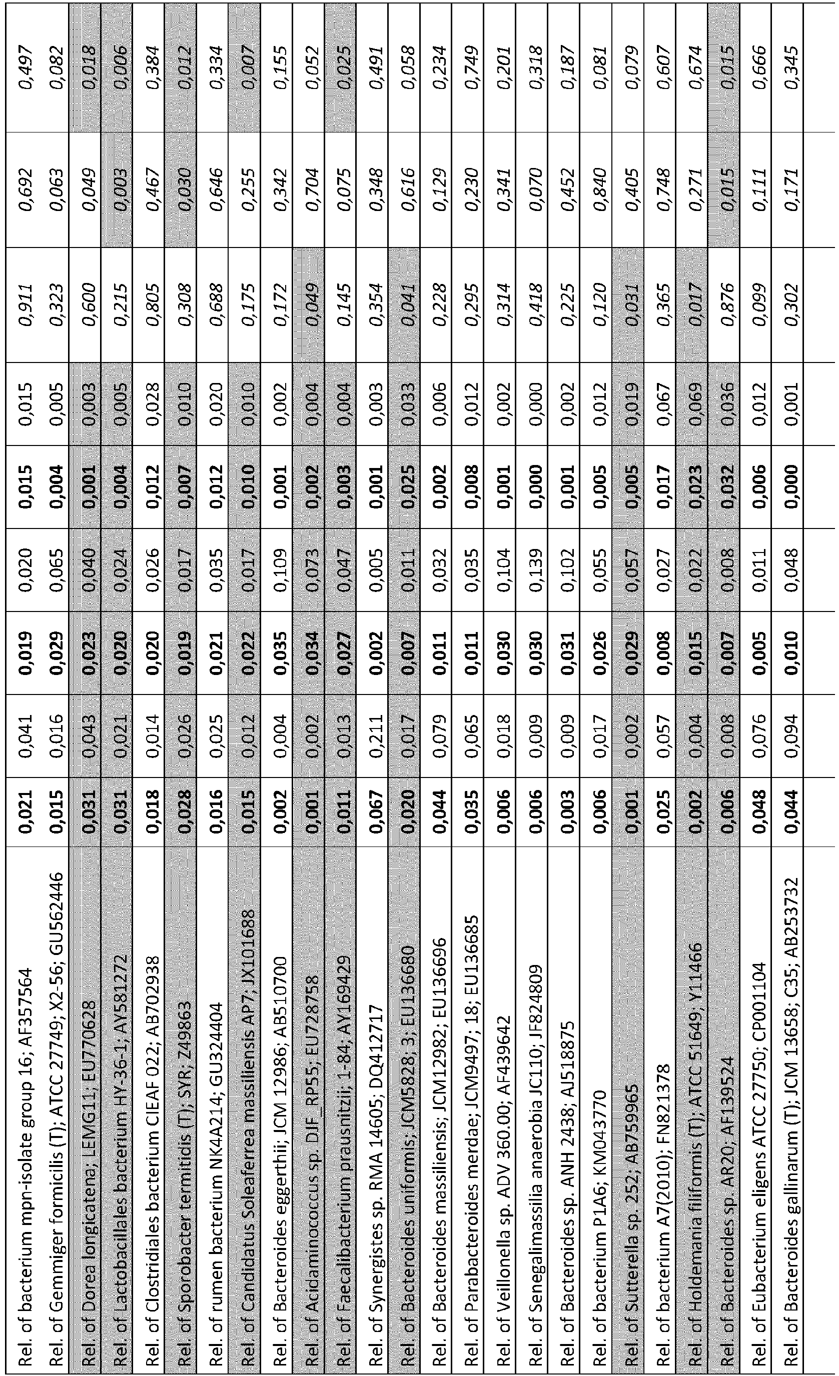

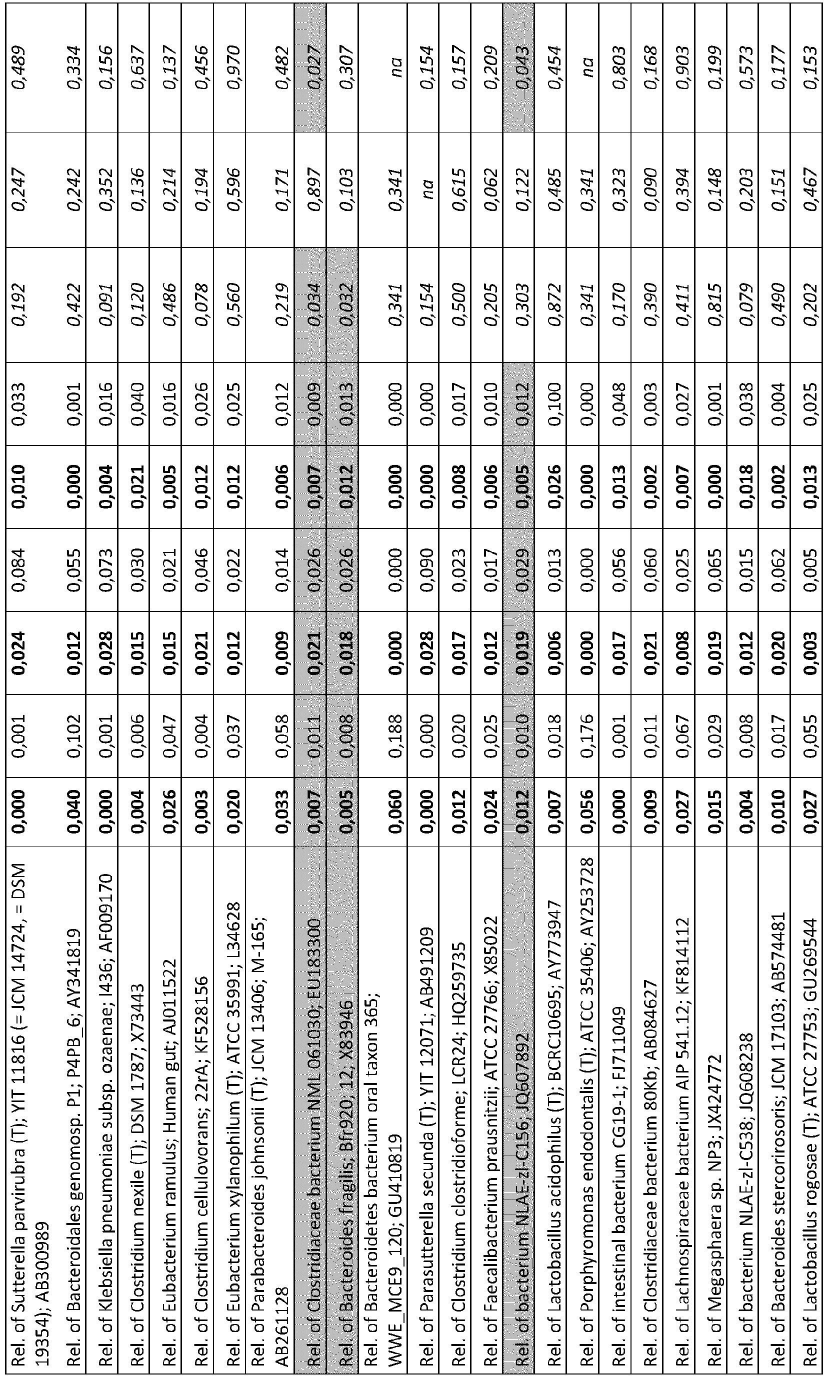

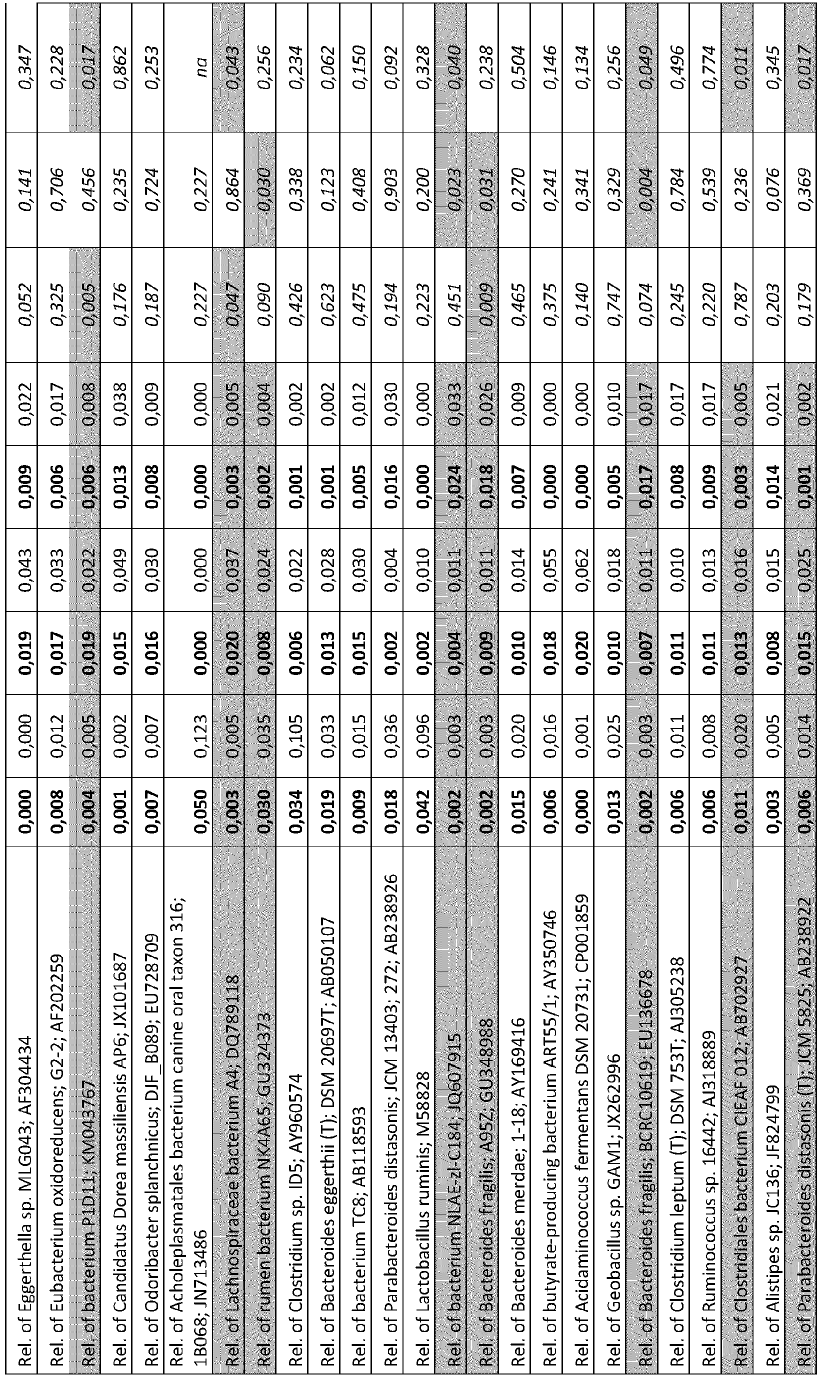

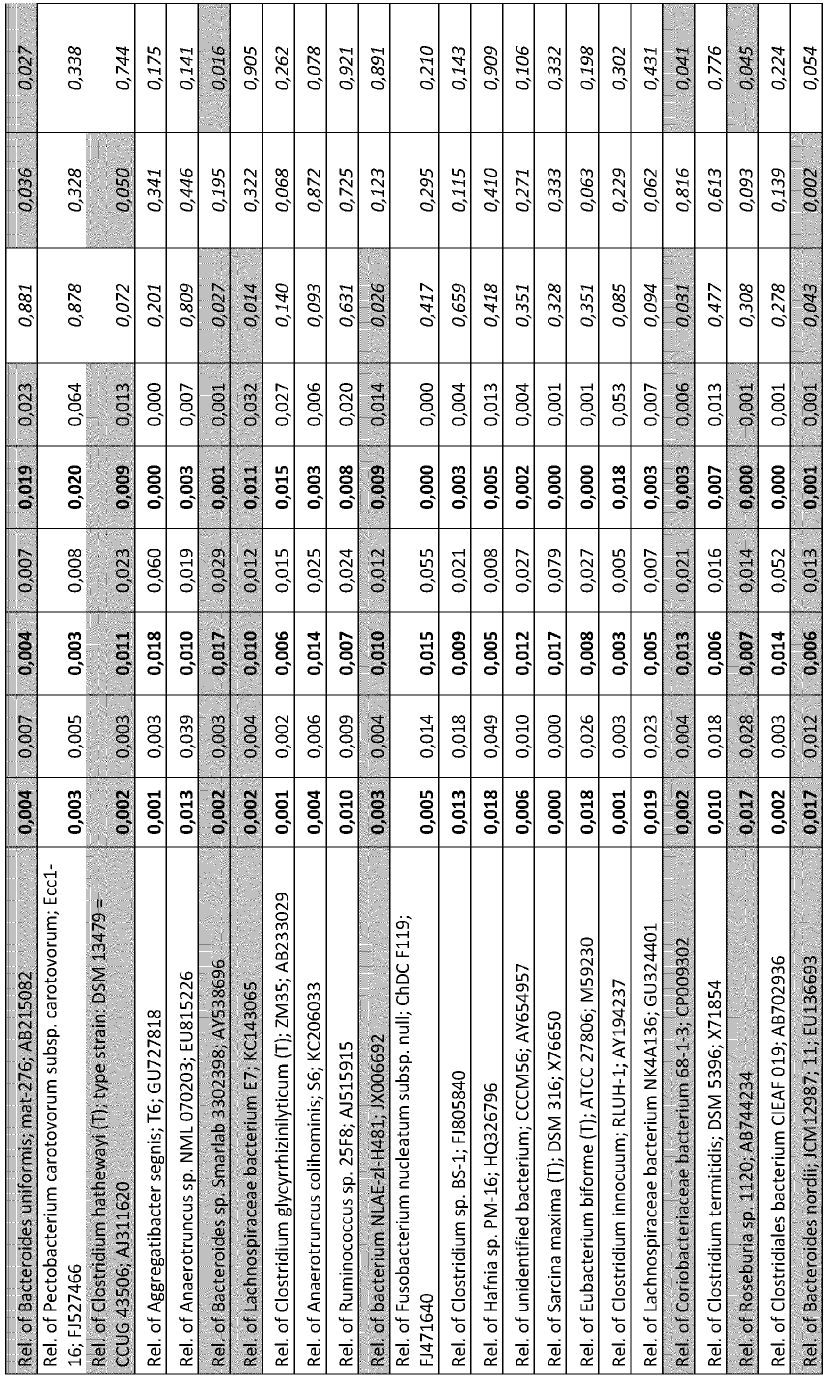

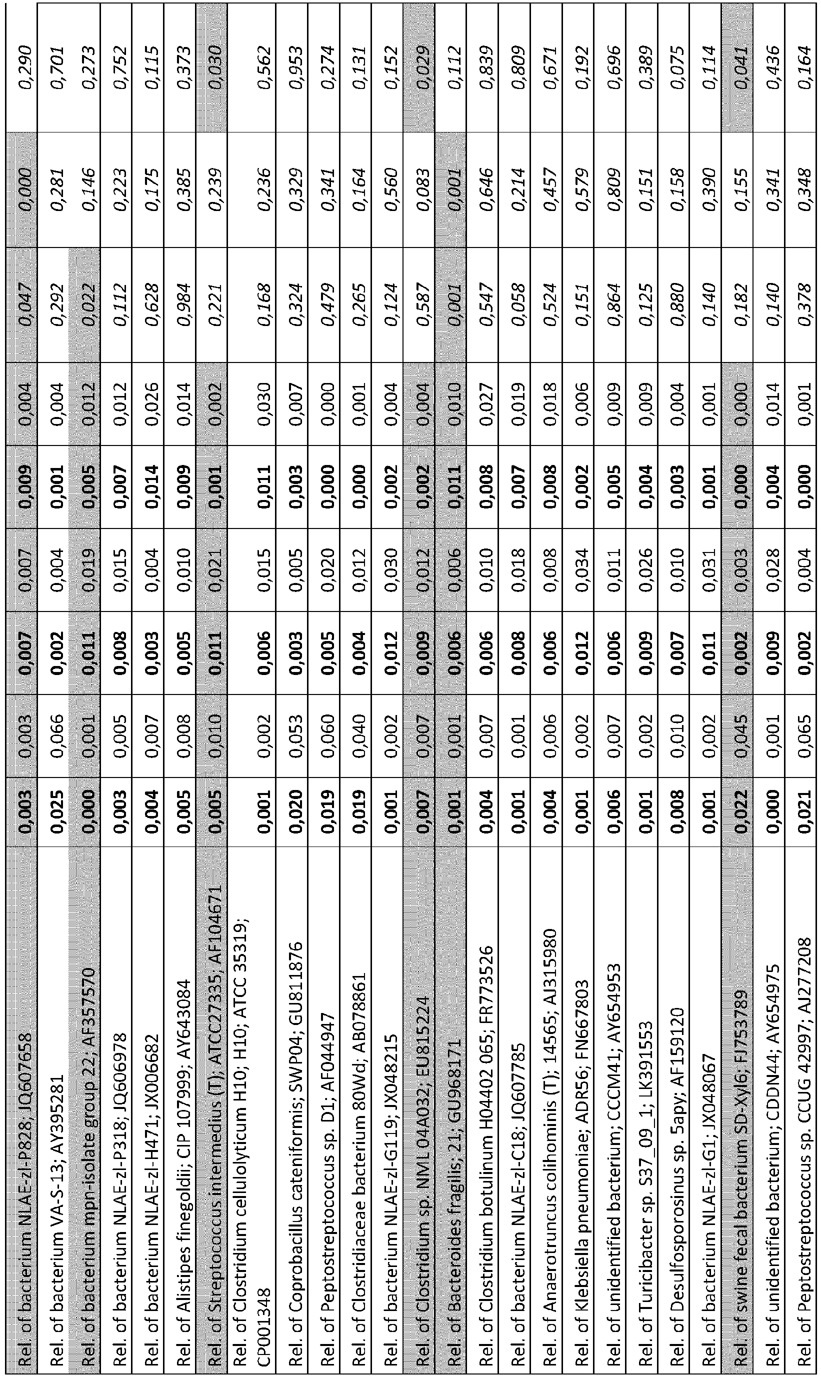

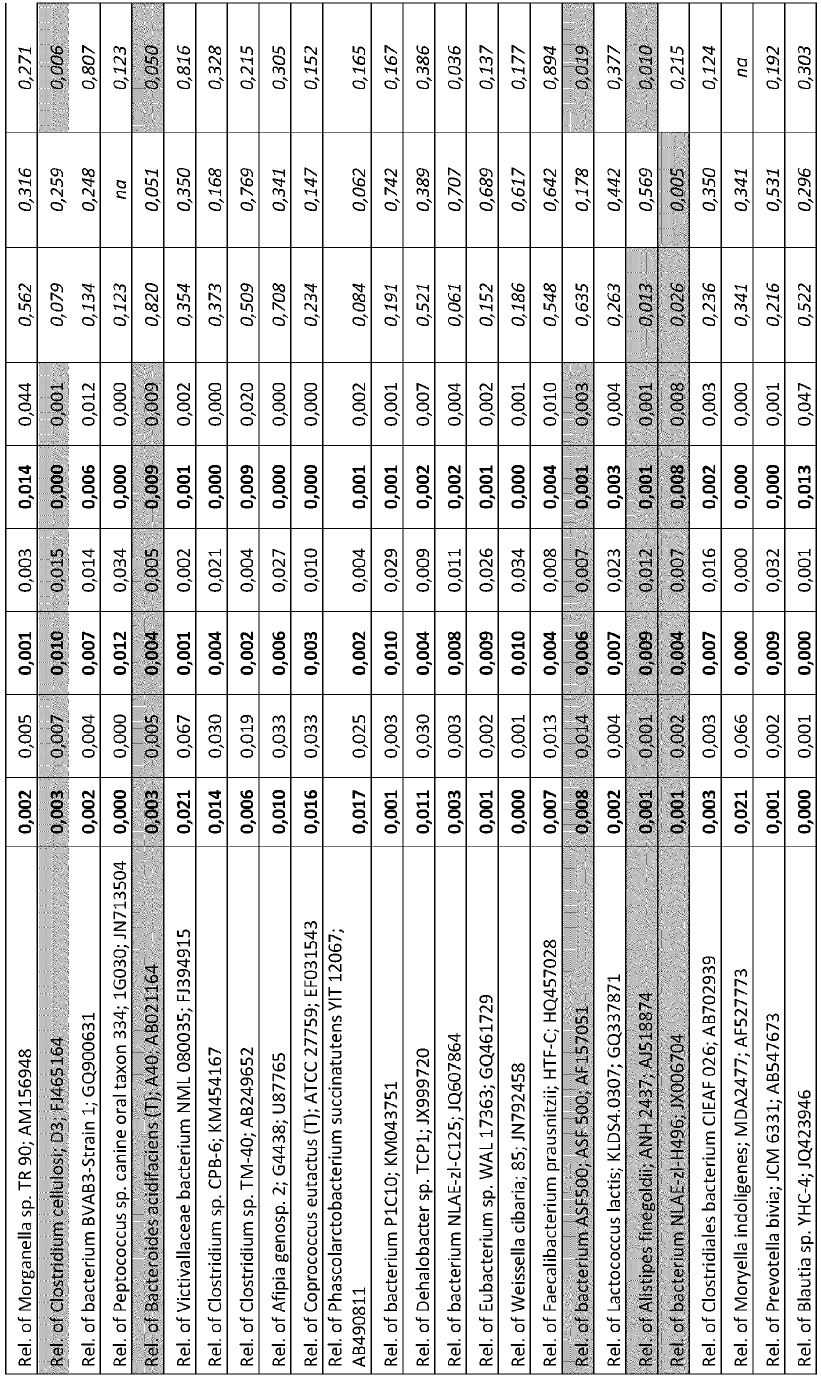

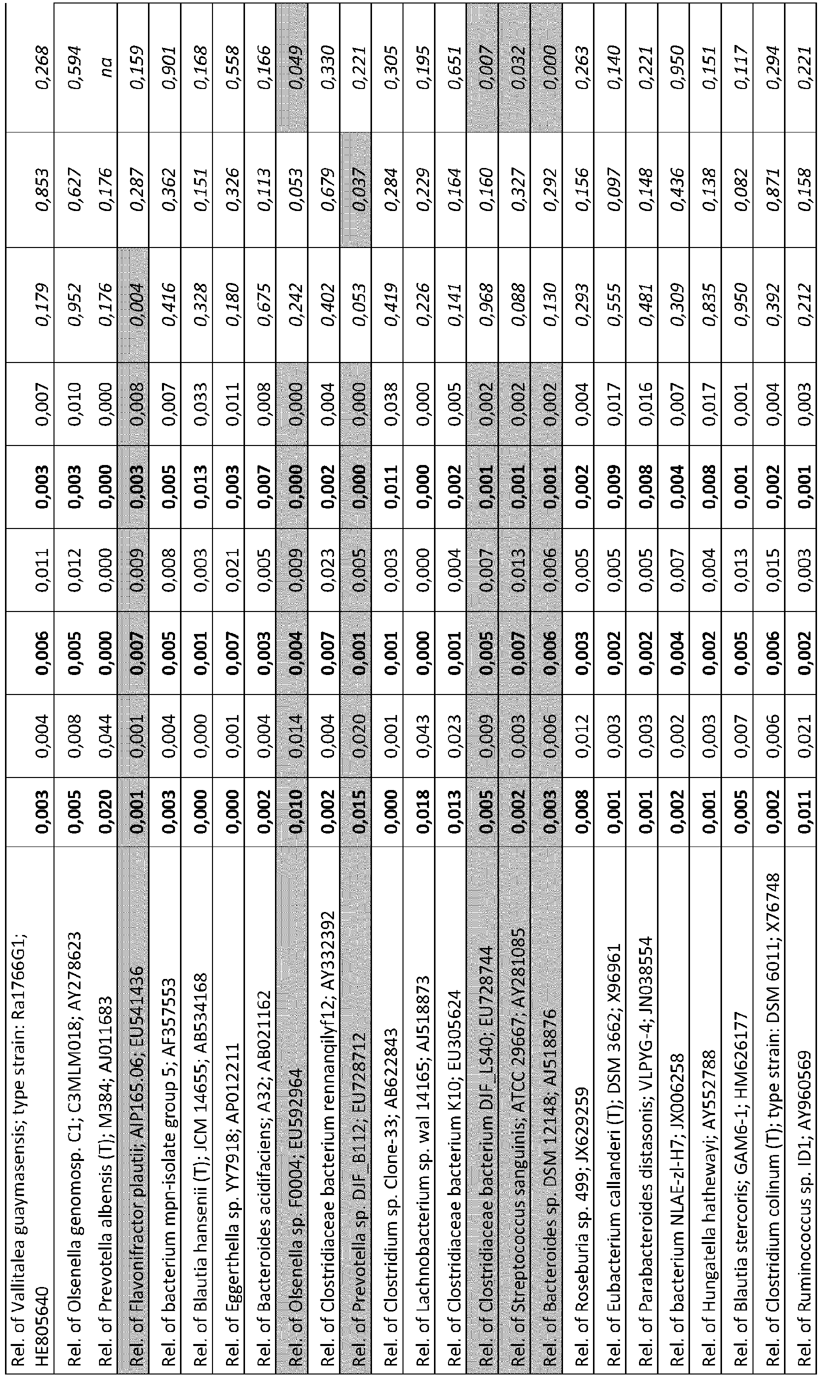

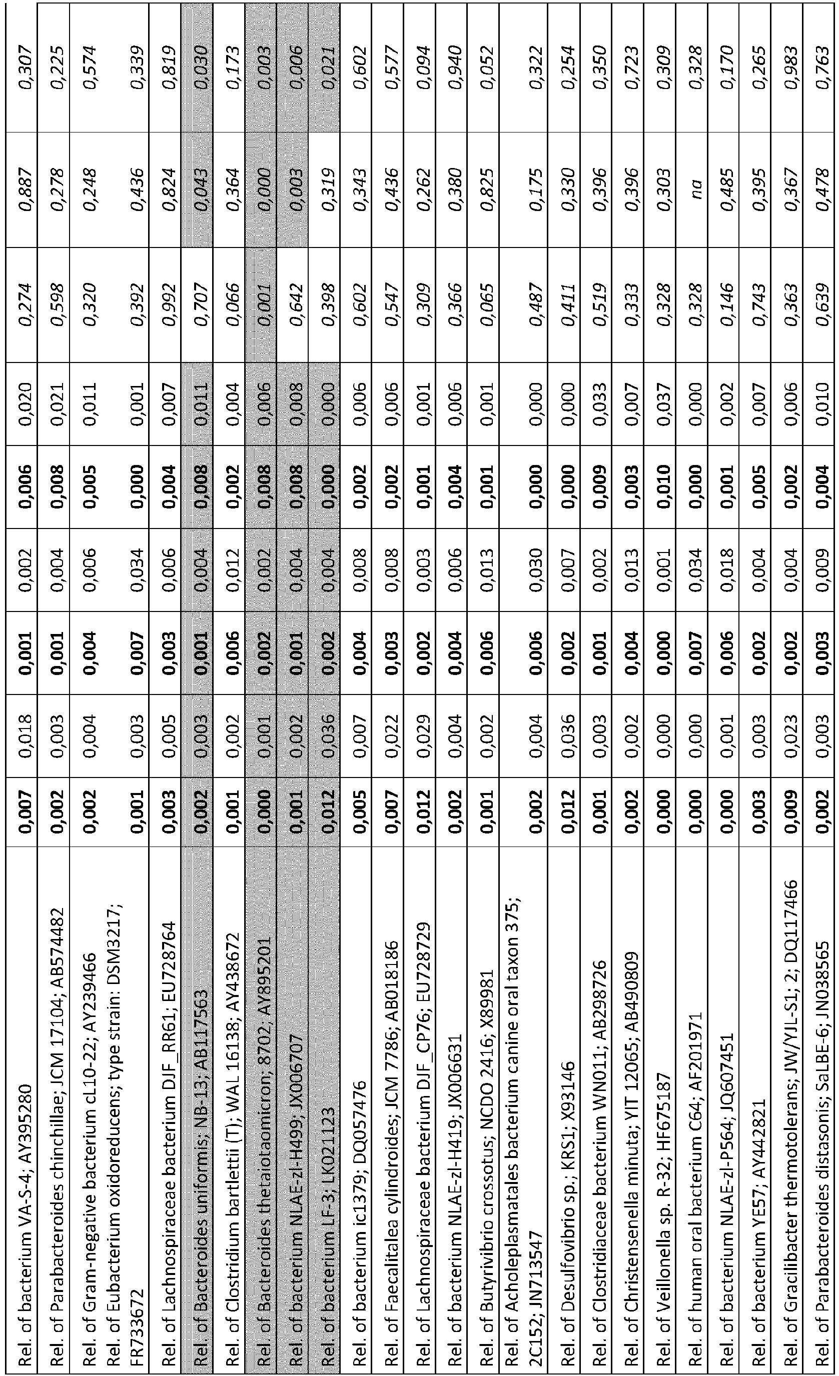

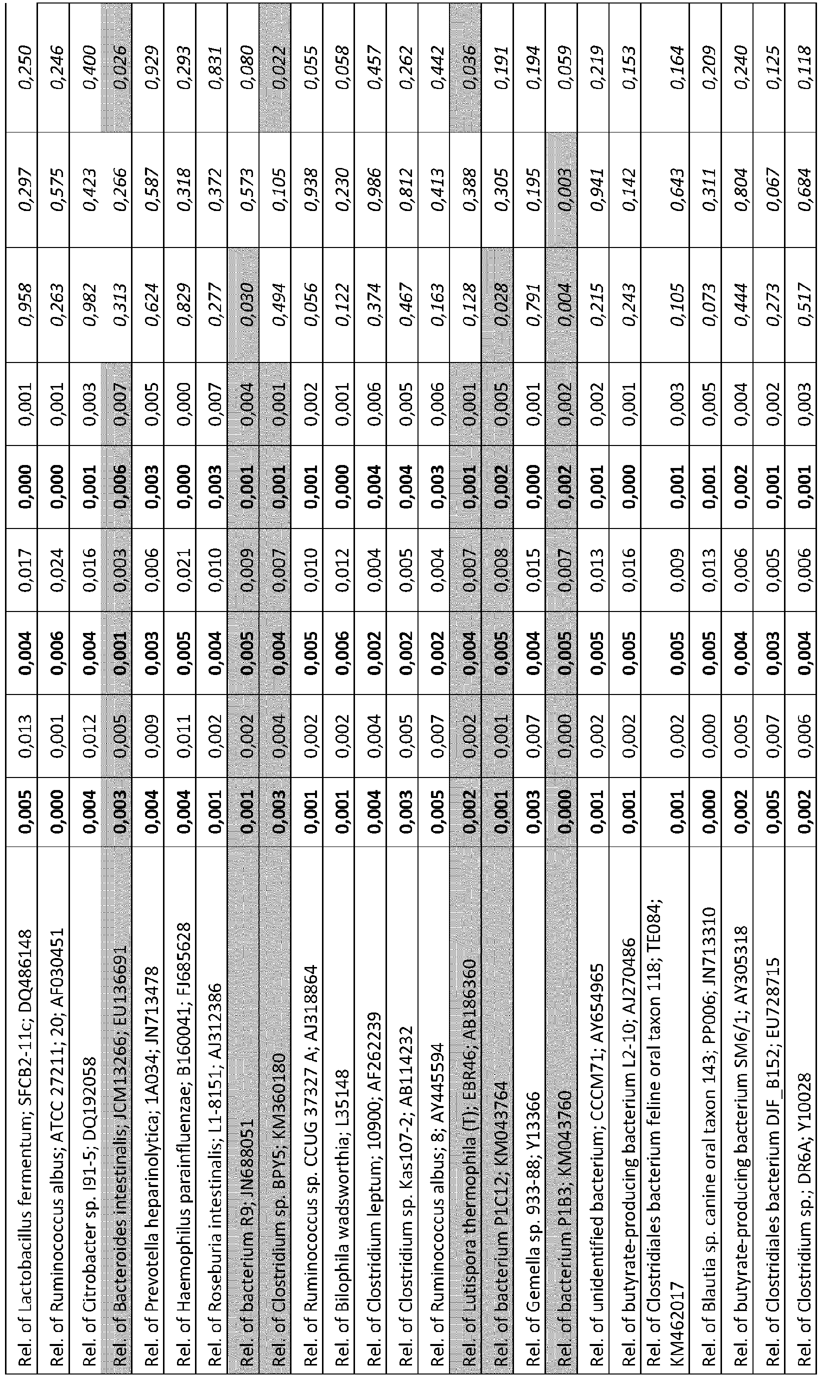

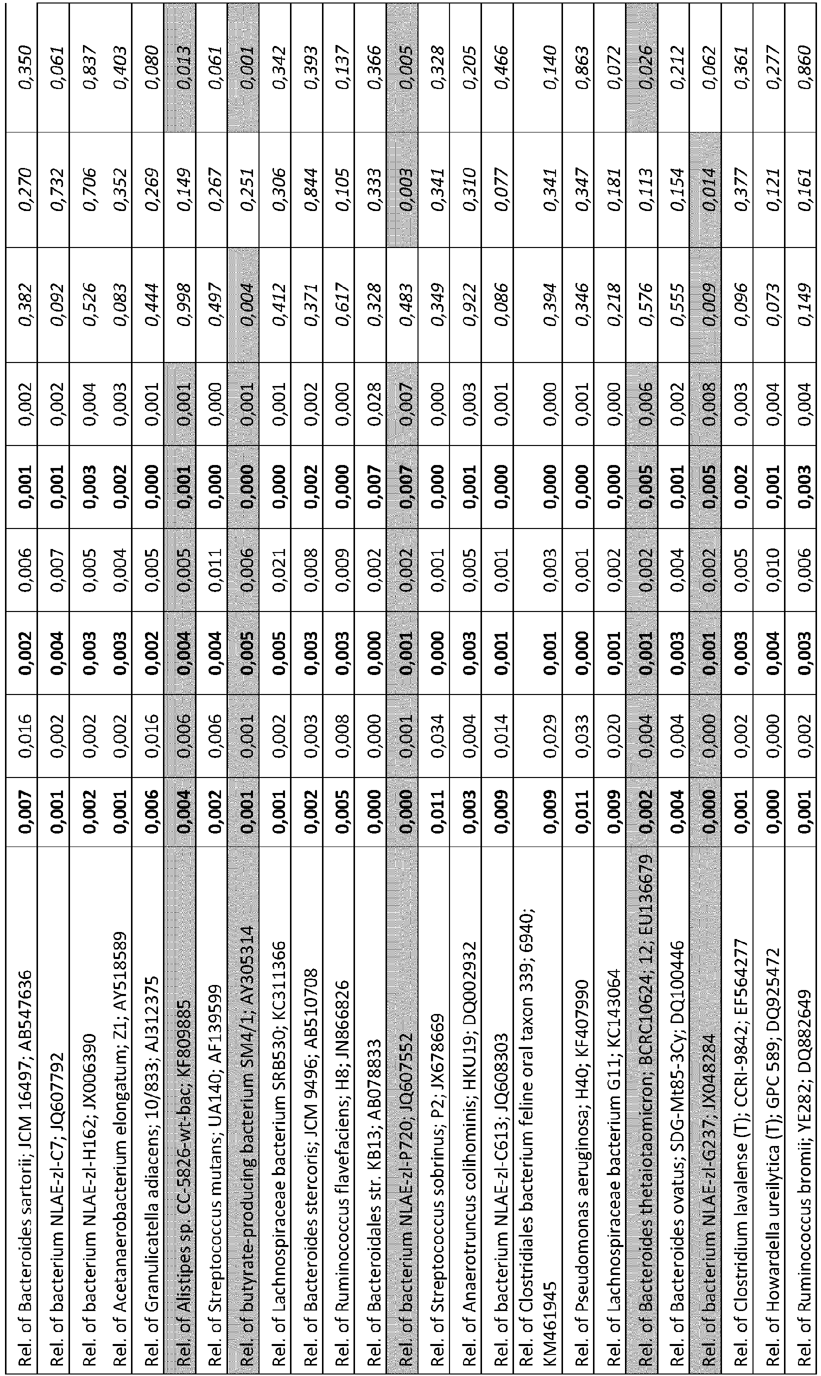

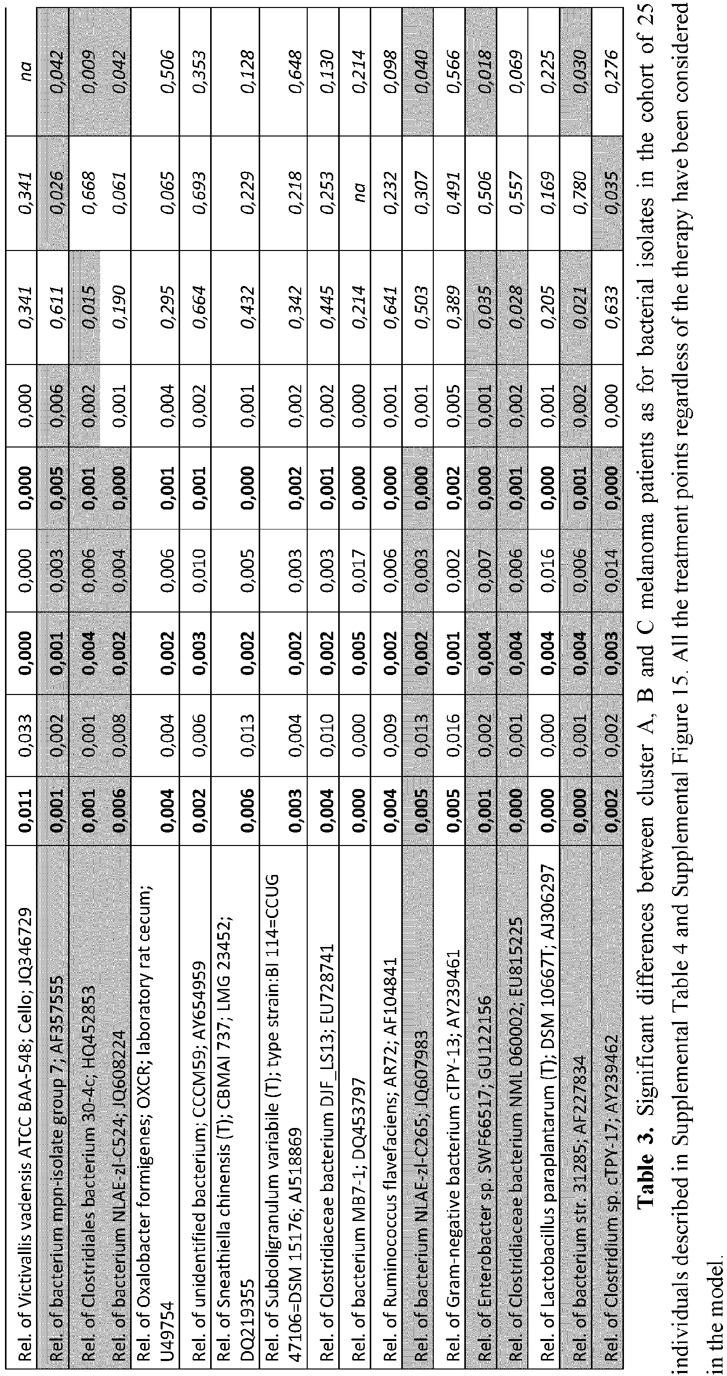

As shown in the above table, cluster C can be defined and distinguished from cluster B by enterotyping, as follows:
A composition obtained from 16S rRNA pyrosequencing of gene amplicons of stools, defined by:
(i) **over-representation** of the following species compared with cluster B:
   Rel. of *Bacteroides salyersiae,* Rel. of *Bacteroides acidifaciens*; AB021157 , Rel. of *Bacteroides acidifaciens* (T); A40; AB021164,
   Rel. of *Bacteroides* sp.; ALA Bac; AM117579,
   Rel. of *Bacteroides uniformis* (T); JCM 5828T; AB050110 and Rel. of *Bacteroides uniformis*; NB-13; AB117563
   Rel. of *Bacteroides fragilis*; 21; GU968171 and Rel. of *Bacteroides fragilis*; 21; GU968171
   Rel. of *Bacteroides thetaiotaomicron;* 8702; AY895201 and Rel. of *Bacteroides thetaiotaomicron;* BCRC10624; 12; EU136679
   Rel. of bacterium NLAE-zl-H499; JX006707
   Rel. of *Bacteroides intestinalis;* JCM13266; EU136691 ; and
(ii) **under-representation** of the following species (mostly butyrate- producing bacteria) compared with cluster B: *Bacteroides* sp. CCUG 39913; AJ518872
   Rel. of bacterium mpn-isolate group 1; AY028442
   Rel. of *Faecalibacterium prausnitzii;* A2-165; AJ270469 and Rel. of *Faecalibacterium prausnitzii;* 1-79; AY169427
   Rel. of *Ruminococcaceae* bacterium LM158; KJ875866 and Rel. of *Ruminococcus* sp.
   DJFVR52; EU728789
   Rel. of *Barnesiella intestinihominis* (T); YIT 11860; AB370251
   Rel. of *Parabacteroides distasonis;* M86695
   Rel. of *Candidatus Alistipes marseilloanorexicus* AP11; JX101692
   Rel. of *Coprobacter fastidiosus*; NSB1; JN703378
   Rel. of *Roseburia faecis* (T); M72/1; AY305310
   Rel. of *Oscillibacter valericigenes* (T); Sjm18-20 (= NBRC 101213); AB238598
   Rel. of *Dorea longicatena* (T); III-35; AJ132842
   Rel. of *Blautia obeum;* 1-33; AY169419
   Rel. of *Oscillospiraceae bacterium* AIP 1035.11; JQ246091 and Rel. of *Oscillibacter* sp. G2; HM626173 and Rel. of *Oscillospiraceae* bacterium NML 061048; EU149939
   Rel. of *Alistipes* sp. NML05A004; EU189022 and Rel. of *Alistipes shahii;* JCM 16773; AB554233 and Rel. of *Alistipes shahii* WAL 8301; FP929032
   Rel. of *Collinsella aerofaciens*; G118; AJ245919
   Rel. of *Parabacteroides distasonis;* JCM5825; EU136681
   Rel. of butyrate-producing bacterium SM4/1; AY305314
   Rel. of *Clostridium* sp. YIT 12070; AB491208 and Rel. of *Clostridiales* bacterium canine oral taxon 061; OF002; JN713225 and Rel. of *Clostridiales* bacterium CIEAF 021; AB702937
   Rel. of *Alistipes finegoldii;* ANH 2437; AJ518874

An alternative way of identifying cluster C is by FMT into a germ-free animal. Stools capable of allowing the niching/colonization of *B. thetaiotaomicron* and/or *B. fragilis* by 14 days upon transfer in germ free animals will be considered as belonging to cluster C.

A third way of defining cluster C is by FMT into a tumor bearing-germ free animal then treated with an anti-CTLA4. Stools capable of allowing the expansion or overrepresentation of *B. fragilis* by 14 days upon transfer into germ free tumor bearing-animals after a therapy with 9D9 anti-CTLA4 Ab.

### EXAMPLE 4: PREDICTING COLITIS AND/OR EFFICACY OF ANTI-CTLA4

### AB ALONE OR COMBINED WITH OTHER IMMUNE CHECKPOINT

### BLOCKERS BY FMT OF HUMAN FECES INTO GF TUMOR BEARERS

Experimental procedure: Fecal microbial transplantation (FMT) of human (patients) feces into germ free (GF) mice according to the method described in Example 2, followed by inoculation of a transplantable tumor model (sc MCA205) at least 15 days later, followed by therapy (systemic anti-CTLA4 Ab). During these experiments, we assessed:
i) the capacity of *B. fragilis* to colonize the recipient digestive tract (as assessed by qPCR) at day 14 post FMT;
ii) the expansion of *B. fragilis* in the recipient colon after 3 administrations of anti-CTLA4 Ab; and
iii) tumor stabilization or regression post-5 iv injections of 9D9 Ab (anti-CTLA4 Ab).
iv) subclinical colitis scores can be assessed in the ileum and colons by immunohistochemistry

We observed that i) and ii) both anti-correlate with tumor size as a result of efficient tumor control with the therapy.

### EXAMPLE 5: A METHOD FOR IMPROVING IMPROVING CLUSTER B PATIENTS TO AMELIORATE THEIR LIKELIHOOD TO RESPOND TO IPILIMUMAB-BASED IMMUNE CHECKPOINT BLOCKADE

Results obtained on model animals (examples 2 and 3) suggest that compensating cluster B-driven individuals with immunogenic *Bacteroides* spp., live or dead and possibly recombinant, or by FMT from cluster C-associated stools will improve their antitumor immune responses with immune checkpoint blockers.

For example, the response to an ICB of a patient, especially a melanoma patient, can be improved by oral administration of a composition of live or dead bacteria including one or several components listed below:
Rel. of *Bacteroides salyersiae,* Rel. of *Bacteroides acidifaciens*; AB021157 , Rel. of *Bacteroides acidifaciens* (T); A40; AB021164,
Rel. of *Bacteroides* sp.; ALA Bac; AM117579,
Rel. of *Bacteroides uniformis* (T); JCM 5828T; AB050110 and Rel. of *Bacteroides uniformis*; NB-13; AB117563
Rel. of *Bacteroides fragilis*; 21; GU968171 and Rel. of *Bacteroides fragilis;* 21; GU968171
Rel. of *Bacteroides thetaiotaomicron;* 8702; AY895201 and Rel. of *Bacteroides thetaiotaomicron;* BCRC10624; 12; EU136679
Rel. of bacterium NLAE-zl-H499; JX006707
Rel. of *Bacteroides intestinalis*; JCM13266; EU136691

Another way of improving the likelihood of a cluster B patient to benefit from a treatment with an ICB is to transplant fecal microbiota obtained from feces from (a) cluster C patient(s) or from normal individuals.

Vaccines based on *B. fragilis* capsides or adoptive T cell transfer of T cells educated to respond to one or several of the bacteria listed above can also be administered to a cluster B patient to improve his/her chances to respond to an anti-CTLA4 (or other ICB) treatment.

### EXAMPLE 6: A BLOOD TEST PREDICTING LONG TERM SURVIVAL OR CLINICAL OUTCOME IN LUNG CANCER PATIENTS TREATED WITH IPILIMUMAB AND RADIOTHERAPY

Memory Th1 CD4⁺ T cell responses directed against distinct components of the *Bacteroidales* order, *Porphyromonadaceae* family dominated by the *Barnesiella* genus *i.e., Barnesiella intestinihominis* were associated with long term protective antitumor immune responses and long term survival in NSCLC treated with irradiation and ipilimumab.

To show the clinical significance of these data combined with the findings from Iida (Iida et al., 2013) demonstrating the impact of the microbiota in platinum-based chemotherapy, we analyzed the predictive value of the preexisting memory CD4⁺CD45RO⁺ Th1 immune responses against Gram⁺ and Gram⁻ bacteria for progression-free survival (PFS) in advanced NSCLC patients treated from two different trials; one with a CTX-based vaccine and a second using Ipilimumab and radiotherapy in patients refractory to platinum-based chemotherapy. Indeed, our team carried out a Phase II clinical trial testing the clinical benefit of an immunization based on autologous IFNγ-DC-derived exosomes used with the cyclophosphamide adjuvant. These self-manufactured nanoparticles (Dex) were loaded with MHC class I and class II -restricted cancer antigens as maintenance immunotherapy in HLA-A2⁺ patients bearing inoperable non-small cell lung cancer (NSCLC) responding to or stabilized after induction chemotherapy (Besse et al OncoImmunology, in press). Additionally, collaborators from NYU enrolled NSCLC patients refractory to platinum-based chemotherapy in a phase 2 trial of combined Ipilimumab plus radiotherapy to assess radiation abscopal effect. Similarly to Dex/CTX trial, the PBMCs tested were collected after platinum-based chemotherapy. Strikingly, high levels of memory Th1 cells recognizing *B*. *intestinihominis* (and to a lesser extent *Burkholderia cepacia*) post-chemotherapy predicted longer progression-free survival in this combined cohort of 26 patients (15 from Dex/CTX and 11 from Ipi/Rdiotherapy), while Th1 recall responses towards other bacteria were not relevant (Figure 26). Importantly, when we separated the analysis and split the two cohorts, we still recovered a statistically significant results considering only the NY cohort treated with ipilimumab (Figure 27). Moreover, a high ratio between IFNγ and IL-10 release following stimulation with *B. intestinihominis* (and to a lesser extent *Burkholderia cepacia*) but not to *E. hirae* or others after chemotherapy was also associated with a longer survival (Figure 28).

Of note, the predictive impact of *Barnesiella intestinihominis* was discovered in mice receiving anti-CTLA4 Ab. The enterotyping of such mice treated with 9D9 Ab revealed that *Barnesiella intestinihominis* were revealed by the Forest plot (Figure 29).

### EXAMPLE 7: A BLOOD TEST PREDICTING LONG TERM SURVIVAL OR CLINICAL OUTCOME IN MELANOMA PATIENTS TREATED WITH IPILIMUMAB: TH1 MEMORY RESPONSES AGAINST B. FRAGILIS OR B. THETAIOTA OMICRON

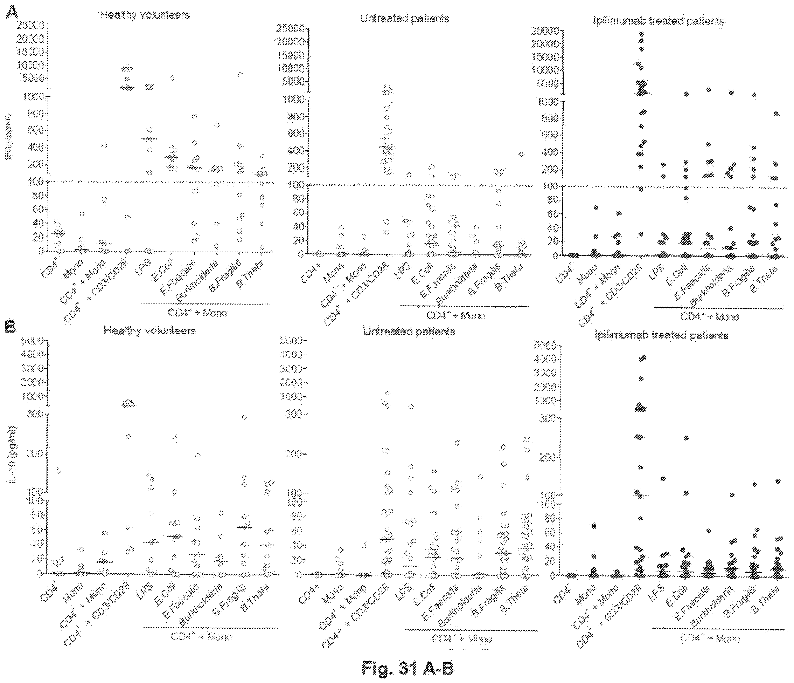

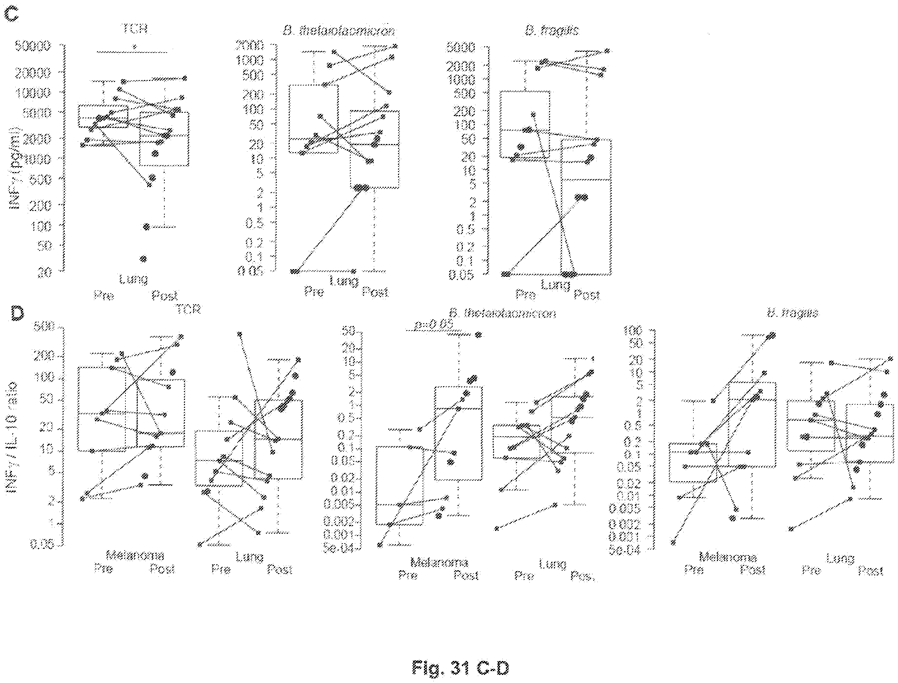

We investigated circulating memory Th1 and Tr1 T cell responses against some species of *Bacteroides* and other commensals prior to and after at least 2 intravenous administrations of ipilimumab, given as a standalone therapy or combined with local radiotherapy in advanced melanoma (MM) and non small cell lung cancers (NSCLC). Compared with healthy volunteers (HV) (Fig. 30A, Fig. 31), cancer patients exhibited a baseline decreased commensal-specific Th1 immunity (Fig. 31A) that, at least in some cases, could be restored by CTLA4 blockade (Fig. 31) with a concomitant drop in IL-10 release (Fig. 31B). Ipilimumab significantly increased IFNγ secretion by T cells responding to *B.fragilis* (*Bf*) and *B.thetaiotaomicron* (*Bt*) (but not irrelevant bacteria) in MM but not in NSCLC patients (Fig. 30A). However, memory T cells from NSCLC patients tend to respond to *Burkholderia cepacia* (*Bc*) by augmenting their IFNγ/IL-10 cytokine release ratio (Fig. 30B).

### REFERENCES

1. K. S. Peggs, S. A. Quezada, A. J. Korman, J. P. Allison, Principles and use of anti-CTLA4 antibody in human cancer immunotherapy. Curr Opin Immunol 18, 206 (Apr, 2006).
2. E. A. Tivol et al., Loss of CTLA-4 leads to massive lymphoproliferation and fatal multiorgan tissue destruction, revealing a critical negative regulatory role of CTLA-4. Immunity 3, 541 (Nov, 1995).
3. F. S. Hodi et al., Improved survival with ipilimumab in patients with metastatic melanoma. N Engl J Med 363, 711 (Aug 19, 2010).
4. C. Robert et al., Ipilimumab plus dacarbazine for previously untreated metastatic melanoma. N Engl J Med 364, 2517 (Jun 30, 2011).
5. D. B. Page, M. A. Postow, M. K. Callahan, J. D. Wolchok, Checkpoint modulation in melanoma: an update on ipilimumab and future directions. Current oncology reports 15, 500 (Oct, 2013).
6. K. E. Beck et al., Enterocolitis in patients with cancer after antibody blockade of cytotoxic T-lymphocyte-associated antigen 4. J Clin Oncol 24, 2283 (May 20, 2006).
7. J. Weber et al., A randomized, double-blind, placebo-controlled, phase II study comparing the tolerability and efficacy of ipilimumab administered with or without prophylactic budesonide in patients with unresectable stage III or IV melanoma. Clinical cancer research : an official journal of the American Association for Cancer Research 15, 5591 (Sep 1, 2009).
8. D. Berman et al., Blockade of cytotoxic T-lymphocyte antigen-4 by ipilimumab results in dysregulation of gastrointestinal immunity in patients with advanced melanoma. Cancer immunity 10, 11 (2010).
9. O. Hamid et al., Safety and tumor responses with lambrolizumab (anti-PD-1) in melanoma. N Engl J Med 369, 134 (Jul 11, 2013).
10. A. Khosravi et al., Gut microbiota promote hematopoiesis to control bacterial infection. Cell Host Microbe 15, 374 (Mar 12, 2014).
11. N. K. Surana, D. L. Kasper, The yin yang of bacterial polysaccharides: lessons learned from B. fragilis PSA. Immunol Rev 245, 13 (Jan, 2012).
12. M. Arumugam et al., Enterotypes of the human gut microbiome. Nature 473, 174 (May 12, 2011).
13. J. Y. Huang, S. M. Lee, S. K. Mazmanian, The human commensal Bacteroides fragilis binds intestinal mucin. Anaerobe 17, 137 (Aug, 2011).
14. S. M. Lee et al., Bacterial colonization factors control specificity and stability of the gut microbiota. Nature 501, 426 (Sep 19, 2013).
15. S. K. Mazmanian, C. H. Liu, A. O. Tzianabos, D. L. Kasper, An immunomodulatory molecule of symbiotic bacteria directs maturation of the host immune system. Cell 122, 107 (Jul 15, 2005).
16. F. Stingele et al., Zwitterionic polysaccharides stimulate T cells with no preferential V beta usage and promote anergy, resulting in protection against experimental abscess formation. J Immunol 172, 1483 (Feb 1, 2004).
17. A. O. Tzianabos, A. B. Onderdonk, B. Rosner, R. L. Cisneros, D. L. Kasper, Structural features of polysaccharides that induce intra-abdominal abscesses. Science 262, 416 (Oct 15, 1993).
18. A. O. Tzianabos et al., The capsular polysaccharide of Bacteroides fragilis comprises two ionically linked polysaccharides. J Biol Chem 267, 18230 (Sep 5, 1992).
19. J. U. Scher, S. B. Abramson, The microbiome and rheumatoid arthritis. Nature reviews. Rheumatology 7, 569 (Oct, 2011).
20. N. W. Palm et al., Immunoglobulin A coating identifies colitogenic bacteria in inflammatory bowel disease. Cell 158, 1000 (Aug 28, 2014).
21. Y. Yang et al., Focused specificity of intestinal TH17 cells towards commensal bacterial antigens. Nature 510, 152 (Jun 5, 2014).
22. H. Xu et al., Innate immune sensing of bacterial modifications of Rho GTPases by the Pyrin inflammasome. Nature 513, 237 (Sep 11, 2014).
23. A. Snyder et al., Genetic basis for clinical response to CTLA-4 blockade in melanoma. N Engl J Med 371, 2189 (Dec 4, 2014).
24. S. Viaud et al., The intestinal microbiota modulates the anticancer immune effects of cyclophosphamide. Science 342, 971 (Nov 22, 2013).
25. W. H. Kruit et al., Selection of immunostimulant AS 15 for active immunization with MAGE-A3 protein: results of a randomized phase II study of the European Organisation for Research and Treatment of Cancer Melanoma Group in Metastatic Melanoma. J Clin Oncol 31, 2413 (Jul 1, 2013).
26. B. Escudier et al., Vaccination of metastatic melanoma patients with autologous dendritic cell (DC) derived-exosomes: results of thefirst phase I clinical trial. Journal of translational medicine 3, 10 (Mar 2, 2005).
27. W. H. Kruit et al., Phase 1/2 study of subcutaneous and intradermal immunization with a recombinant MAGE-3 protein in patients with detectable metastatic melanoma. International journal of cancer. Journal international du cancer 117, 596 (Nov 20, 2005).
28. M. Venkatesh et al., Symbiotic bacterial metabolites regulate gastrointestinal barrier function via the xenobiotic sensor PXR and Toll-like receptor 4. Immunity 41, 296 (Aug 21, 2014).
29. C. A. Schneider, W. S. Rasband, K. W. Eliceiri, NIH Image to ImageJ: 25 years of image analysis. Nature methods 9, 671 (Jul, 2012).
30. A. Schlitzer et al., IRF4 transcription factor-dependent CD11b+ dendritic cells in human and mouse control mucosal IL-17 cytokine responses. Immunity 38, 970 (May 23, 2013).
31. A. Pantosti, A. O. Tzianabos, A. B. Onderdonk, D. L. Kasper, Immunochemical characterization of two surface polysaccharides of Bacteroides fragilis. Infect Immun 59, 2075 (Jun, 1991).

## Claims

1. A probiotic bacterial composition comprising bacteria selected from the group consisting of *Bacteroidesfragilis, Bacteroides thetaiotaomicron, Burkholderia cepacia* and mixtures thereof, for use for treating a cancer, in combination with an anti-CTLA4 antibody blocking CTLA4.

2. The probiotic bacterial composition according claim 1, for the use according to claim 1, wherein said bacterial composition further comprises bacteria selected from the group consisting of *Bacteroides salyersiae, Bacteroides acidifaciens, Bacteroides intestinalis, Bacteroides vulgatus, Burkholderia cenocepacia, Bacteroides uniformis, Bacteroides massiliensis* and *Barnesiella intestinihominis.*

3. The probiotic bacterial composition according claim 1 or claim 2, for the use according to claim 1, wherein said probiotic bacterial composition induces immunostimulation in a cancer patient.

4. The probiotic bacterial composition according to claim 1 or claim 2, for use in combination with an anti-CTLA4 antibody blocking CTLA4, for treating a cancer patient who has a dysbiosis with an under-representation of Gram-negative bacteria.

5. The probiotic bacterial composition according to claim 1 or claim 2, for the use according to any one of claims 1, 3 and 4, wherein said patient is treated by radiotherapy.

6. A fecal microbiota composition, for use as a medicament for treating a cancer, in combination with a treatment comprising immunotherapy with an anti-CTLA4 antibody blocking CTLA4, wherein said fecal microbiota composition is from one or several patient(s) falling into cluster C, and/or from healthy individual(s) and wherein said fecal microbiota composition potentiates the effects of said treatment comprising immunotherapy with an anti-CTLA4 antibody blocking CTLA4.

7. The fecal microbiota composition of claim 6, for the use of claim 6, wherein said fecal microbiota composition (i) allows the niching of *Bacteroides fragilis* and/or *Bacteroides thetaiotaomicron* upon transplant into a germ-free animal and/or (ii) allows the expansion of *B. fragilis* upon transplant into a germ-free tumor-bearing animal after treatment of said animal with an anti-CTLA4 antibody blocking CTLA4.

8. The fecal microbiota composition of claim 6 or claim 7, for the use of claim 6 or claim 7, for potentiating the anticancer effects of a treatment comprising immunotherapy with an anti-CTLA4 antibody blocking CTLA4 in a cancer patient whose feces belongs to cluster B.

9. A combination of an anti-CTLA4 antibody blocking CTLA4 and of an antibiotic killing Gram positive bacteria and not killing *Bacteroidales,* for use in the treatment of cancer.

10. The combination of claim 9, for the use according to claim 9, wherein the antibiotic is vancomycin or penicilline G.

11. The combination of any of claims 9 to 10, for the use according to claim 9, wherein said patient also receives radiotherapy.

12. An antibiotic selected from the group consisting of vancomycin and other antibiotics killing Gram positive bacteria and not killing *Bacteroidales,* for use for treating a cancer, in combination with an anti-CTLA4 antibody blocking CTLA4, wherein said antibiotic potentiates the anticancer effects of the anti-CTLA4 antibody blocking CTLA4 administered to a patient by modulating the gut microbiota of said patient.

13. The antibiotic of claim 12, for the use according to claim 12, for potentiating the anticancer effects of a treatment comprising radiotherapy and immunotherapy with an anti-CTLA4 antibody blocking CTLA4.

14. The bacterial composition of any of claims 1 to 5, for the use of any of claims 1 to 5, the fecal microbiota composition of any of claims 6 to 8, for the use of any of claims 6 to 8, the combination of any of claims 9 to 11, for the use according to any of claims 9 to 11, or the antibiotic of claim 12 or claim 13, for the use of claim 12 or claim 13, wherein said anti-CTLA4 antibody blocking CTLA4 is an anti-CTLA4 monoclonal antibody.

## Patentansprüche

1. Probiotische bakterielle Zusammensetzung, die Bakterien umfasst, die aus der Gruppe bestehend aus *Bacteroides fragilis, Bacteroides thetaiotaomicron, Burkholderia cepacia* und Mischungen davon ausgewählt sind, zur Verwendung zur Behandlung von Krebs in Kombination mit einem Anti-CTLA4-Antikörper, der CTLA4 blockiert.

2. Probiotische bakterielle Zusammensetzung nach Anspruch 1 zur Verwendung nach Anspruch 1, wobei die bakterielle Zusammensetzung ferner Bakterien umfasst, die aus der Gruppe bestehend aus *Bacteroides salyersiae, Bacteroides acidifaciens, Bacteroides intestinalis, Bacteroides vulgatus, Burkholderia cenocepacia, Bacteroides uniformis, Bacteroides massiliensis* und *Barnesiella intestinihominis* ausgewählt sind.

3. Probiotische bakterielle Zusammensetzung nach Anspruch 1 oder Anspruch 2 zur Verwendung nach Anspruch 1, wobei die probiotische bakterielle Zusammensetzung bei einem Krebspatienten eine Immunstimulation induziert.

4. Probiotische bakterielle Zusammensetzung nach Anspruch 1 oder Anspruch 2 zur Verwendung in Kombination mit einem Anti-CTLA4-Antikörper, der CTLA4 blockiert, zur Behandlung eines Krebspatienten, der eine Dysbiose mit einer Unterrepräsentation von gramnegativen Bakterien aufweist.

5. Probiotische bakterielle Zusammensetzung nach Anspruch 1 oder Anspruch 2 zur Verwendung nach einem der Ansprüche 1, 3 und 4, wobei der Patient durch eine Strahlentherapie behandelt wird.

6. Fäkale Mikrobiota-Zusammensetzung zur Verwendung als Medikament zur Behandlung von Krebs in Kombination mit einer Behandlung, die eine Immuntherapie mit einem Anti-CTLA4-Antikörper umfasst, der CTLA4 blockiert, wobei die fäkale Mikrobiota-Zusammensetzung von einem oder mehreren Patienten, die ins Cluster C fallen, und/oder von gesunden Personen stammen, und wobei die fäkale Mikrobiota-Zusammensetzung die Wirkung der Behandlung, die eine Immuntherapie mit einem Anti-CTLA4-Antikörper umfasst, der CTLA4 blockiert, verstärkt.

7. Fäkale Mikrobiota-Zusammensetzung nach Anspruch 6 zur Verwendung nach Anspruch 6, wobei die fäkale Mikrobiota-Zusammensetzung (i) die Nischenbildung von *Bacteroides fragilis* und/oder *Bacteroides thetaiotaomicron* bei der Transplantation in ein keimfreies Tier ermöglicht und/oder (ii) die Ausbreitung von *B. fragilis* bei der Transplantation in ein keimfreies, tumortragendes Tier nach der Behandlung dieses Tieres mit einem Anti-CTLA4-Antikörper, der CTLA4 blockiert, ermöglicht.

8. Fäkale Mikrobiota-Zusammensetzung nach Anspruch 6 oder Anspruch 7 zur Verwendung nach Anspruch 6 oder Anspruch 7 zur Verstärkung der krebshemmenden Wirkung einer Behandlung, die eine Immuntherapie mit einem Anti-CTLA4-Antikörper umfasst, der CTLA4 blockiert, bei einem Krebspatienten, dessen Fäkalien zu Cluster B gehören.

9. Kombination aus einem Anti-CTLA4-Antikörper, der CTLA4 blockiert, und einem Antibiotikum, das grampositive Bakterien abtötet und *Bacteroidales* nicht abtötet, zur Verwendung bei der Behandlung von Krebs.

10. Kombination nach Anspruch 9 zur Verwendung nach Anspruch 9, wobei das Antibiotikum Vancomycin oder Penicillin G ist.

11. Kombination nach einem der Ansprüche 9 bis 10 zur Verwendung nach Anspruch 9, wobei der Patient außerdem eine Strahlentherapie erhält.

12. Antibiotikum, das aus der Gruppe bestehend aus Vancomycin und anderen Antibiotika, die grampositive Bakterien abtöten und *Bacteroidales* nicht abtöten, ausgewählt ist, zur Verwendung zur Behandlung von Krebs in Kombination mit einem Anti-CTLA4-Antikörper, der CTLA4 blockiert, wobei das Antibiotikum die krebshemmende Wirkung des Anti-CTLA4-Antikörpers, der CTLA4 blockiert, verstärkt und einem Patienten durch Modulieren der Darmmikrobiota des Patienten verabreicht wird.

13. Antibiotikum nach Anspruch 12, zur Verwendung nach Anspruch 12, zur Verstärkung der krebshemmenden Wirkung einer Behandlung, die eine Strahlentherapie und eine Immuntherapie mit einem Anti-CTLA4-Antikörper, der CTLA4 blockiert, umfasst.

14. Bakterielle Zusammensetzung nach einem der Ansprüche 1 bis 5 zur Verwendung nach einem der Ansprüche 1 bis 5, fäkale Mikrobiota-Zusammensetzung nach einem der Ansprüche 6 bis 8 zur Verwendung nach einem der Ansprüche 6 bis 8, Kombination nach einem der Ansprüche 9 bis 11 zur Verwendung nach einem der Ansprüche 9 bis 11 oder Antibiotikum nach Anspruch 12 oder Anspruch 13 zur Verwendung nach Anspruch 12 oder Anspruch 13, wobei der Anti-CTLA4-Antikörper, der CTLA4 blockiert, ein monoklonaler Anit-CTLA4-Antikörper ist.

## Revendications

1. Composition bactérienne probiotique comprenant des bactéries sélectionnées parmi le groupe constitué de *Bacteroides fragilis, Bacteroides thetaiotaomicron, Burkholderia cepacia* et des mélanges de celles-ci, pour une utilisation pour traiter un cancer, en combinaison avec un anticorps anti-CTLA4 bloquant le CTLA4.

2. Composition bactérienne probiotique selon la revendication 1, pour l'utilisation selon la revendication 1, dans laquelle ladite composition bactérienne comprend en outre des bactéries sélectionnées parmi le groupe constitué de *Bacteroides salyersiae, Bacteroides acidifaciens, Bacteroides intestinalis, Bacteroides vulgatus, Burkholderia cenocepacia, Bacteroides uniformis, Bacteroides massiliensis* et *Barnesiella intestinihominis.*

3. Composition bactérienne probiotique selon la revendication 1 ou la revendication 2, pour l'utilisation selon la revendication 1, dans laquelle ladite composition bactérienne probiotique induit une immunostimulation chez un patient atteint d'un cancer.

4. Composition bactérienne probiotique selon la revendication 1 ou la revendication 2, pour l'utilisation en combinaison avec un anticorps anti-CTLA4 bloquant le CTLA4, pour le traitement d'un patient atteint d'un cancer qui présente une dysbiose avec une sous-représentation de bactéries Gram négatif.

5. Composition bactérienne probiotique selon la revendication 1 ou la revendication 2, pour l'utilisation selon l'une quelconque des revendications 1, 3 et 4, dans laquelle ledit patient est traité par radiothérapie.

6. Composition de microbiote fécal, pour une utilisation comme médicament pour le traitement d'un cancer, en combinaison avec un traitement comprenant de l'immunothérapie avec un anticorps anti-CTLA4 bloquant le CTLA4, dans laquelle ladite composition de microbiote fécal provient d'un patient ou de plusieurs patients appartenant au groupe C, et/ou d'un individu sain ou d'individus sains et dans laquelle ladite composition de microbiote fécal potentialise les effets dudit traitement comprenant l'immunothérapie avec un anticorps anti-CTLA4 bloquant le CTLA4.

7. Composition de microbiote fécal selon la revendication 6, pour l'utilisation selon la revendication 6, dans laquelle ladite composition de microbiote fécal (i) permet l'implantation de *Bacteroides fragilis* et/ou de *Bacteroides thetaiotaomicron* lors de la transplantation chez un animal stérile et/ou (ii) permet l'expansion de *B. fragilis* lors de la transplantation chez un animal stérile porteur d'une tumeur après le traitement dudit animal avec un anticorps anti-CTLA4 bloquant le CTLA4.

8. Composition de microbiote fécal selon la revendication 6 ou la revendication 7, pour l'utilisation selon la revendication 6 ou la revendication 7, pour potentialiser les effets anticancéreux d'un traitement comprenant de l'immunothérapie avec un anticorps anti-CTLA4 bloquant le CTLA4 chez un patient atteint d'un cancer dont les fèces appartiennent au groupe B.

9. Combinaison d'un anticorps anti-CTLA4 bloquant le CTLA4 et d'un antibiotique tuant les bactéries Gram positif et ne tuant pas les *Bacteroidales,* pour une utilisation dans le traitement d'un cancer.

10. Combinaison selon la revendication 9, pour l'utilisation selon la revendication 9, dans laquelle l'antibiotique est de la vancomycine ou de la pénicilline G.

11. Combinaison selon l'une quelconque des revendications 9 à 10, pour l'utilisation selon la revendication 9, dans laquelle ledit patient reçoit également de la radiothérapie.

12. Antibiotique sélectionné parmi le groupe constitué de vancomycine et d'autres antibiotiques tuant les bactéries Gram positif et ne tuant pas les *Bacteroidales,* pour une utilisation dans le traitement d'un cancer, en combinaison avec un anticorps anti-CTLA4 bloquant le CTLA4, dans lequel ledit antibiotique potentialise les effets anticancéreux de l'anticorps anti-CTLA4 bloquant le CTLA4 administré à un patient en modulant le microbiote intestinal dudit patient.

13. Antibiotique selon la revendication 12, pour l'utilisation selon la revendication 12, pour potentialiser les effets anticancéreux d'un traitement comprenant de la radiothérapie et de l'immunothérapie avec un anticorps anti-CTLA4 bloquant le CTLA4.

14. Composition bactérienne selon l'une quelconque des revendications 1 à 5, pour l'utilisation d'une quelconque des revendications 1 à 5, composition de microbiote fécal selon l'une quelconque des revendications 6 à 8, pour l'utilisation d'une quelconque des revendications 6 à 8, combinaison d'une quelconque des revendications 9 à 11, pour l'utilisation selon l'une quelconque des revendications 9 à 11, ou antibiotique selon la revendication 12 ou la revendication 13, pour l'utilisation de la revendication 12 ou la revendication 13, dans laquelle/lequel ledit anticorps anti-CTLA4 bloquant le CTLA4 est un anticorps monoclonal anti-CTLA4.
